# EUROPEAN PATENT SPECIFICATION

(11) **EP 4 041 285 B1**
(45) Date of publication and mention of the grant of the patent: **18.09.2024**
(21) Application number: 20875427.5
(22) Date of filing: 09.10.2020
(51) Int. Cl.: A61K 31/19, A61K 38/43, A61P 1/14, A61K 31/59, A61K 31/405, A61K 31/593, A61K 31/198, A61P 1/00, A61K 9/28, A61K 38/16

(54) **GASTROINTESTINAL HEALTH COMPOSITION**
GASTROINTESTINALE GESUNDHEITSZUSAMMENSETZUNG
COMPOSITION DE SANTÉ GASTRO-INTESTINALE

(30) Priority: 11.10.2019 AU 2019903822
(43) Date of publication of application: 17.08.2022
(73) Proprietor: Anatara Lifesciences Limited, Brisbane, QLD 4120 (AU)
(72) Inventor: BROWN, Tracey, Flemington, Victoria 3031 (AU)
(74) Representative: Haseltine Lake Kempner LLP
(86) International application number: PCT/IB2020/059483
(87) International publication number: WO 2021/070121

(56) References cited:
- WO-A1-2016/005793
- US-A1- 2011 064 712
- US-A1- 2011 064 712
- US-A1- 2014 161 784
- US-A1- 2016 235 822
- HEAD KATHLEEN A ET AL: "Inflammatory bowel disease Part 1: ulcerative colitis--pathophysiology and conventional and alternative treatment options", ALTERNATIVE MEDICINE REVIEW, THORNE RESEARCH INC., SANDPOINT, US, vol. 8, no. 3, 1 August 2003 (2003-08-01), pages 247 - 283, XP002349422, ISSN: 1089-5159
- ANDRZEJ ZA&LSTROK;&EOGON;SKI ET AL: "Butyric acid in irritable bowel syndrome", GASTROENTEROLOGY REVIEW, vol. 6, 1 January 2013 (2013-01-01), pages 350 - 353, XP055426645, ISSN: 1895-5770, DOI: 10.5114/pg.2013.39917
- JULIEN BERTRAND ET AL: "Glutamine Restores Tight Junction Protein Claudin-1 Expression in Colonic Mucosa of Patients With Diarrhea-Predominant Irritable Bowel Syndrome", JOURNAL OF PARENTERAL AND ENTERAL NUTRITION, vol. 40, no. 8, 13 May 2015 (2015-05-13), pages 1170 - 1176, XP055816771
- HALE LAURA P. ET AL: "Dietary supplementation with fresh pineapple juice decreases inflammation and colonic neoplasia in IL-10-deficient mice with colitis :", HHS PUBLIC ACCESS AUTHOR MANUSCRIPT, vol. 16, no. 12, 1 December 2010 (2010-12-01), US, pages 2012 - 2021, XP055816766, ISSN: 1078-0998, DOI: 10.1002/ibd.21320
- WILLIAMS CLAIRE E ET AL: "Vitamin D status in irritable bowel syndrome and the impact of supplementation on symptoms: what do we know and what do we need to know?", EUROPEAN JOURNAL OF CLINICAL NUTRITION, NATURE PUBLISHING GROUP, GB, vol. 72, no. 10, 25 January 2018 (2018-01-25), pages 1358 - 1363, XP036609042, ISSN: 0954-3007, [retrieved on 20180125], DOI: 10.1038/S41430-017-0064-Z
- HALE LAURA P., CHICHLOWSKI MACIEJ, TRINH CHAU T., GREER PAULA K.: "Dietary Supplementation with Fresh Pineapple Juice Decreases Inflammation and Colonic Neoplasia in IL -10-deficient Mice with Colitis", INFLAMMATORY BOWEL DISEASES, vol. 16, no. 12, 2010, pages 2012 - 2021, XP055816766
- ANDRZEJ ZALESKI; BANASZKIEWICZ ALEKSANDRA; WALKOWIAK JAROSLAW: "Butyric acid in irritable bowel syndrome", GASTROENTEROLOGY REVIEW,, vol. 8, no. 6, 2013, pages 350 - 353, XP055535391, DOI: 10.5114/pg.2013.39917
- JULIEN BERTRAND, IBTISSEM GHOUZALI , CHARLÈNE GUÉRIN , CHRISTINE BÔLE-FEYSOT , MÉLODIE GOUTEUX , PIERRE DÉCHELOTTE , PHILIPPE DUC: "Glutamine Restores Tight Junction Protein Claudin-1 Expression in Colonic Mucosa of Patients With Diarrhea-Predominant Irritable Bowel Syndrome", JOURNAL OF PARENTERAL AND ENTERAL NUTRITION, vol. 40, no. 8, 2016, pages 1170 - 1176, XP055816771
- WILLIAMS, C. ET AL.: "Vitamin D status in irritable bowel syndrome and the impact of supplementation on symptoms: what do we know and what do we need to know?", EUROPEAN JOURNAL OF CLINICAL NUTRITION, vol. 72, 2018, pages 1358 - 1363, XP036609042, DOI: 10.1038/s41430-017-0064-z

## Description

### FIELD OF THE INVENTION

This invention relates generally to a composition for improving gastrointestinal health, to nutritional and/or dietary supplements formulated for such use and to the use of such compositions and/or supplements for gastrointestinal reprogramming. This invention provides a composition comprising the following components:
(a) bromelain,
(b) one or more short chain fatty acids (SCFAs) or esters thereof,
(c) one or more amino acids selected from the group consisting of L-threonine, L-tryptophan, L-arginine, L-glutamine and L-methionine and combinations thereof, and
(d) Vitamin D or alfacalcidiol.

### BACKGROUND TO THE INVENTION

### Gastrointestinal disease

Gastrointestinal disease and disorders such as irritable bowel syndrome (IBS) and inflammatory bowel disease (IBD) are caused by the interaction of a number of different drivers including microbial, immunologic, genetic and environmental factors (Bernstein CN, 2010). Dysbiosis of the gastrointestinal bacteria exerts the downstream effects of mucosal inflammation, immune disruption and mucosal damage (Bernstein CN, 2010). The gastrointestinal disorders of IBD and IBS share common disease characteristics such as an altered microbiome, impaired intestinal function and mucosal damage. The differentiating factor between these two diseases is the level of inflammation observed; IBS has low grade inflammation in contrast to IBD which is characterised by chronic inflammation. The current treatment approach to IBD is to supress the inflammation with the goal of inducing and maintaining remission. In contrast, gastroenterologists treating IBS primarily treat the symptoms of abdominal pain, bloating and alternating constipation and diarrhoea. The therapeutic approaches currently employed to treat both IBD and IBS have high treatment failure rates.

Consequently, there is an unmet medical need in the art for products that can successfully treat and/or relieve or eliminate these conditions and their symptoms from IBD and IBS patients without suffering from the currently observed high treatment failure rates.

Accordingly, it is an object of the invention to provide a gastrointestinal dietary supplement that can go at least some way towards addressing this unmet need and/or that will at least provide the public with a useful choice.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.
Head, K. A. et al, 1 Aug 2003, Altern Med Rev vol 8. No. 3, pp247-283 describes ulcerative colitis pathophysiology and conventional and alternative treatment options.
Zaleski A. et al, 1 Jan 2013, Prz Gastroenterol: vol 8 (6), pp350-353 describes butyric acid in irritable bowel syndrome.
Bertrand, J. et al, 13 May 2015, J. Parenteral and Enteral Nutrition, vol. 40, no. 8, pp1170-1176 describes the glutamine restores tight junction protein Claudin-1 expression in colonic mucosa of patients with diarrhea-predominant irritable bowel syndrome.
Hale, L. P. et al, 1 Dec 2010, Inflamm. Bowl, Dis, vol 16, no. 12, pp212-2021 describes dietary supplementation with fresh pineapple juice decreases inflammation and colonic neoplasia in IL-10-deficienct mice with colitis.
Williams, C. E.,25 Jan 2018, European Journal of Clinical Nutrition, vol 72, no. 10, pp 1358-1363 describes Vitamin D status in irritable bowel syndrome and the impact of supplementation on symptoms.

### SUMMARY OF THE INVENTION

The present invention is defined in the appended claims.

The present invention provides a composition comprising the following components:
(a) bromelain,
(b) one or more short chain fatty acids (SCFAs) or esters thereof,
(c) one or more amino acids selected from the group consisting of L-threonine, L-tryptophan, L-arginine, L-glutamine and L-methionine and combinations thereof, and
(d) Vitamin D or alfacalcidiol.

In one aspect the invention relates to a composition comprising the following components:
(a) bromelain,
(b) one or more SCFAs or esters thereof,
(c) L-threonine, and
(d) Vitamin D or a metabolite thereof which is alfacalcidiol.

In another aspect the invention relates to an oral dosage form comprising about 1 to about 10 g of a composition of the invention.

In another aspect the invention relates to an oral dosage form consisting essentially of a composition of the invention wherein at least two components of the composition are comprised in a delayed release formulation.

The reference to method of treatment in the summary and detailed description of the invention in this description are to be interpreted as references to the compositions of the present invention for use in a method for treatment of the human (or animal) body by therapy.

In another aspect the invention relates a method of preventing, treating or managing IBD or IBS comprising administering to a subject in need thereof, a therapeutically effective amount of a composition or oral dosage form of the invention.

In another aspect the invention relates to a method of at least partially alleviating at least one symptom of IBD or IBS comprising administering to a subject in need thereof, a therapeutically effective amount of a composition or oral dosage form of the invention.

In another aspect the invention relates to a method of improving at least one parameter associated with IBD or IBS comprising administering to a subject in need thereof, a therapeutically effective amount of a composition or oral dosage form of the invention.

In another aspect the invention relates to a method of reducing bacterial attachment to the cells of the gastrointestinal tract of a subject in need thereof, comprising contacting the bacteria with an effective amount of the composition or oral dosage form of the invention.

In another aspect the invention relates to a method of reducing bacterial invasion into the cells of the gastrointestinal tract of a subject in need thereof, comprising contacting the bacteria with an effective amount of the composition or oral dosage form of the invention.

In another aspect the invention relates to a method of increasing the gastrointestinal mucosal layer in a subject in need thereof, comprising comprising administering to the subject a therapeutically effective amount of the composition or oral dosage form of the invention.

In another aspect the invention relates to a method of improving gastrointestinal barrier integrity in a subject in need thereof comprising administering to the subject, a therapeutically effective amount of a composition of the invention

In another aspect the invention relates to a method of restoring homeostasis of the microbiome of a subject in need thereof, comprising administering to the subject, a therapeutically effective amount of a composition of the invention.

In another aspect the invention relates to a method of treating gastrointestinal inflammation in a subject in need thereof, comprising administering to the subject, a therapeutically effective amount of a composition of the invention.

Other aspects of the invention may become apparent from the following description which is given by way of example only and with reference to the accompanying drawings.

### BRIEF DESCRIPTION OF THE FIGURES

The invention will now be described by way of example only and with reference to the drawings in which:

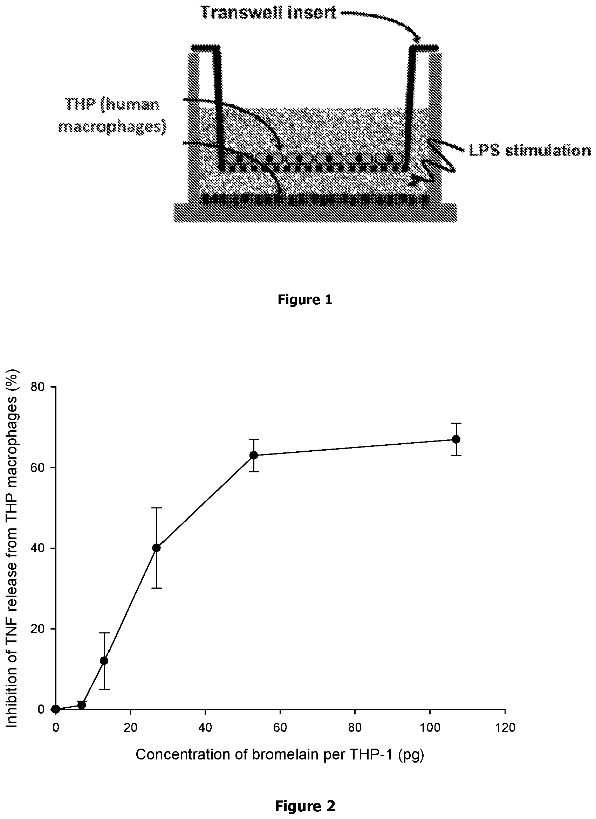

**Figure 1****- Depiction of transwell plate assay.** The transwell plate assay was established by seeding Caco-2-BBe1 cells at a 5×10⁵ cells/ well onto 24-well transwell insert plates (4.67 cm², 0.4 µm pore size). The cell culture medium was changed every 3 day until the cells were fully differentiated [Trans Epithelial Resistance (TER) value ≥1100/cm²]. To test the translocation of bacteria or the effect of a treatment on cytokine release, bacteria or treatments were placed in the apical compartment.
Figure 2 - **The effect of GaRP and bromelain on the release of TNFa from THP-1 macrophages.** THP-1 cells were plated at 1×10⁶ cells/cm² into 24 well plates. The macrophages were differentiated by treatment with 200nm PMA for 48h. After 48h the macrophages were washed and grown in PMA and antibiotic-free media for 24h before commencing the experiment. The macrophages were stimulated with 50ng/ml of LPS immediately prior to the addition of the GaRP ± 0-80µg/ml bromelain (0-107 pg/macrophage). Unstimulated wells were used as comparative controls to confirm the effectiveness of the LPS stimulation. After 8h the media was removed and TNFα was quantitated using an ELISA. Each data point is the mean ± SD of 5-10 independent experiments.

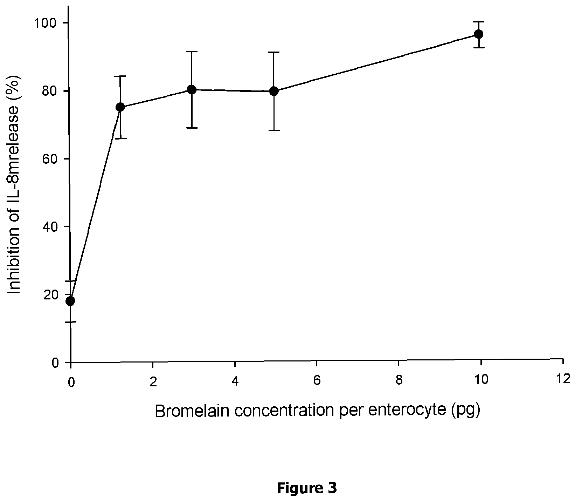

**Figure 3** **- The effect of GaRP and bromelain on the release of IL-8 from polarised Caco-2 monolayers co-incubated with LPS-stimulated THP-1 macrophages.** The transwell plate assay was established by seeding Caco-2-BBe1 cells at a 5×10⁵ cells/ well onto 24-well transwell insert plates (4.67 cm², 0.4 µm pore size). The cell culture medium was changed every 3 days until the cells were fully differentiated (TER value ≥1100/cm²). In a separate 24 well plate, THP-1 monocytes cells were seeded at 1×10⁶ cells/well and differentiated with 200nM PMA for 48h followed by culture in normal media for 24h prior to the experiment. After replacing all media with RPMI1640, all transwell inserts containing the Caco-2 differentiated monolayers were loaded into the wells containing differentiated THP-1 cells. In experiments to evaluate the anti-inflammatory effect of different bromelain formulations, 0.25 ml of test samples was applied to the apical side for 24 h, at the same time 50ng/ml of LPS was added to the basolateral side in this model. After the 24h incubation culture supernatants from the apical side were collected for IL-8 measurement using an IL-8 ELISA. Each data point is the mean ± SD of 5 independent experiments.

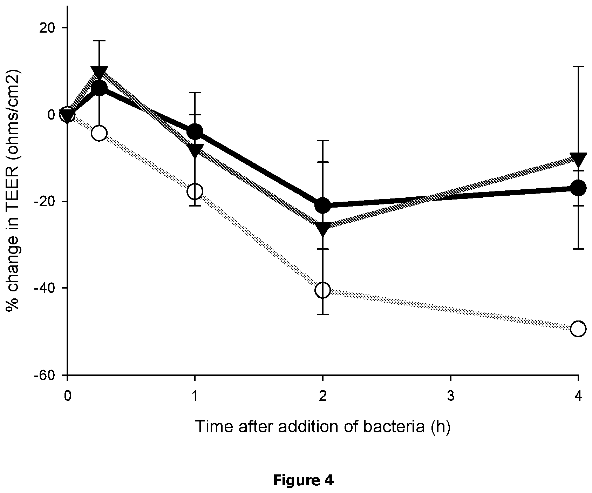

**Figure 4** - **GaRP (minus bromelain) prevents the bacterially-induced loss in TER.**
   A M-cell model was established by plating 5×10⁵ Caco-2 cells onto the apical face of 12 mm transwell cell culture inserts which had been precoated with Matrigel basement membrane matrix. Cells were maintained for 14-18 days, until the TER was > 600 Ω per transwell (equivalent to 300 Ω cm² tissue monolayer). On the day of co-culture, Raji B cells were harvested, re-suspended in complete DMEM and 5×10⁵ cells added to the basal transwell compartment. Co-cultures were maintained for 4-6 days, until M-cells were generated. The M-cell model was infected with AIEC HM605 by adding the bacteria to the apical well of the transwell insert at an MOI of 30 for a period of 4h. After 4h the cells were washed and incubated with 100µg/ml of gentamicin to kill any residual bacteria. At that point, GaRP minus bromelain was placed on the cells and the TER of all monolayers was measured at 2-hour intervals up to 12 hours and bacteria in the basal well were enumerated every 1 hours up to 4 hours.
   When the AIEC HM605 which were suspended in normal media (○-○) or in GaRP (minus bromelain, ▼-▼) and applied to the M-cell model, the GaRP formulation provided a protective effect on the integrity of the cell layer. Bacteria applied to the cell monolayers in GaRP produced a similar effect on cell layer integrity as the DMEM/no bacteria control (●-●). Each data point is the mean ± SD of 4 independent determinations.

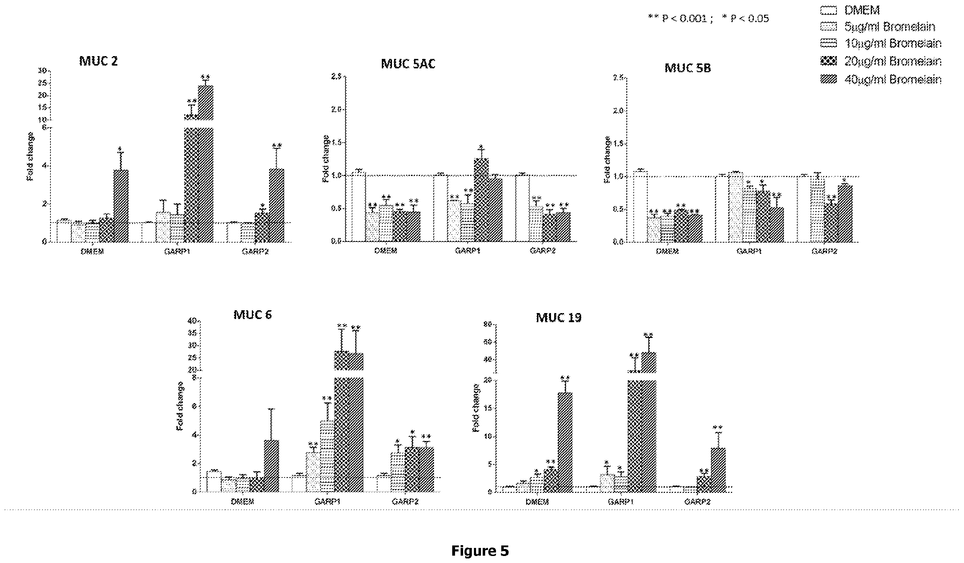

**Figure 5** - **Titration of the effect of bromelain in GaRP on the expression of gel-forming mucin genes.**
   A 75:25 Caco-2/HT-29 co-culture was established by seeding the combined cells (6.4 × 10⁴ cells/ml) into 25 cm² in a total of 5 ml per flask. At 15 days post seeding, the coculture monolayer was treated with 5 ml of test solutions for 24 h. After the 24h exposure to bromelain, threonine, sodium butyrate, vitamin D3 and the two different GaRP formulations, the expression of MUC genes was investigated using qPCR. Data points are the mean ± SEM of 12 (DMEM) or 6 independent determinations.

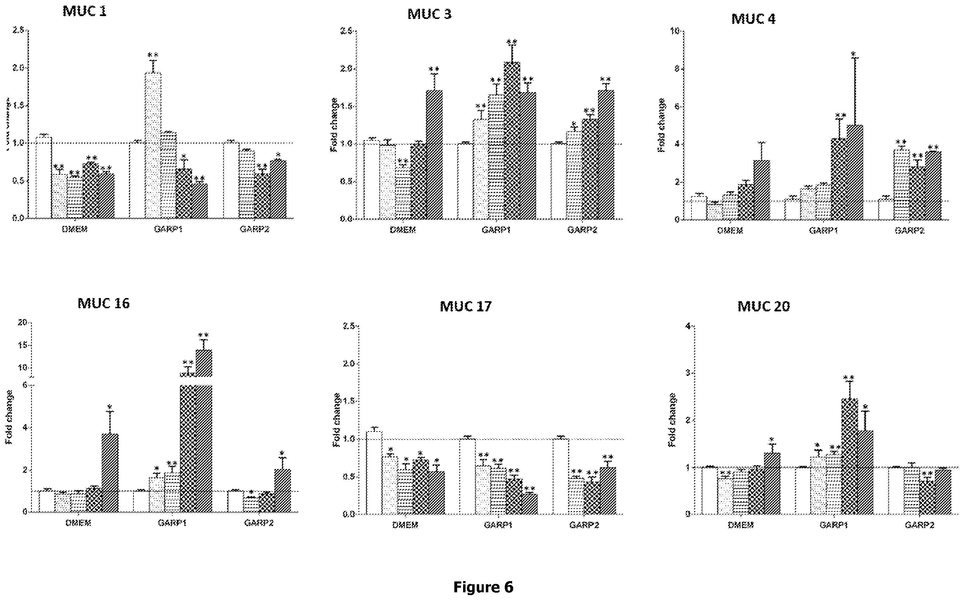

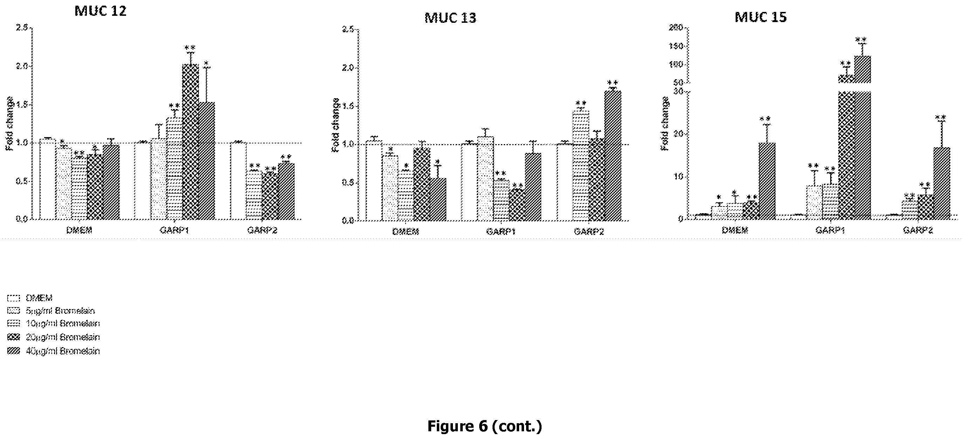

**Figure 6** - **Titration of the effect of bromelain in GaRP on the expression of membrane-associated mucins.**
   A 75:25 Caco-2/HT-29 co-culture was established by seeding the combined cells (6.4 × 10⁴ cells/ml) into 25 cm² in a total of 5 ml per flask. At 15 days post seeding, the coculture monolayer was treated with 5 ml of test solutions for 24 h. After the 24h exposure to bromelain, threonine, sodium butyrate, vitamin D3 and the two different GaRP formulations, the expression of MUC genes was investigated using qPCR. Data points are the mean ± SEM of 12 (DMEM) or 6 independent determinations.

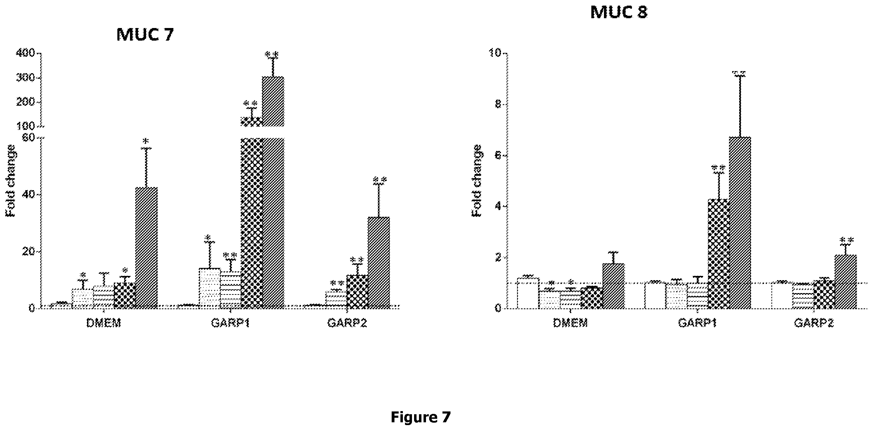

**Figure 7** - **Titration of the effect of bromelain in GaRP on the expression of secreted mucins.**
   A 75:25 Caco-2/HT-29 co-culture was established by seeding the combined cells (6.4 × 10⁴ cells/ml) into 25 cm² in a total of 5 ml per flask. At 15 days post seeding, the coculture monolayer was treated with 5 ml of test solutions for 24 h. After the 24h exposure to bromelain, threonine, sodium butyrate, vitamin D3 and the two different GaRP formulations, the expression of MUC genes was investigated using qPCR. Data points are the mean ± SEM of 12 (DMEM) or 6 independent determinations.
**Figure 8** - **Effect of vitamin D3 and L-threonine on the expression of mucin genes**
   A 75:25 Caco-2/HT-29 co-culture was established by seeding the combined cells (6.4 × 10⁴ cells/ml) into 25 cm² in a total of 5 ml per flask. At 15 days post seeding, the coculture monolayer was treated with 5 ml of test solutions for 24 h. After the 24h exposure to threonine vitamin D3 the expression of MUC genes was investigated using qPCR. Data points represent mean ± SEM (where n=6)

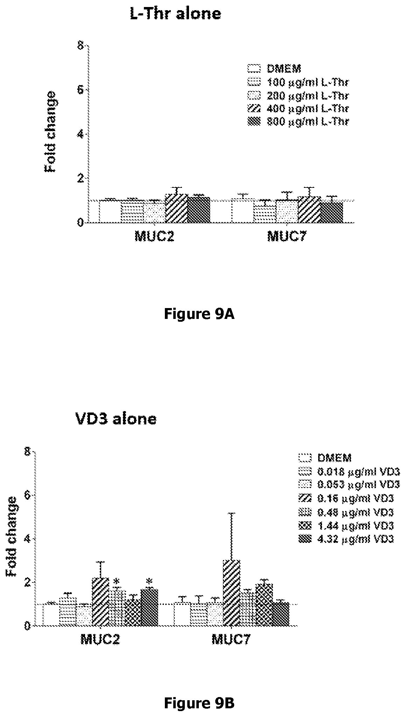

**Figure 9** - **9A****: Effect of L-threonine on the expression of MUC 2 and MUC7**
   A 75:25 Caco-2/HT-29 co-culture was established by seeding the combined cells (6.4 × 10⁴ cells/ml) into 25 cm² in a total of 5 ml per flask. At 15 days post seeding, the coculture monolayer was treated with 5 ml of test solutions for 24 h. After the 24h exposure to L-threonine the expression of MUC genes was investigated using qPCR. Data points are the mean ± SEM of 12 (DMEM) or 6 independent determinations.
   **9B:** Effect of vitamin D3 on the expression of MUC 2 and MUC7. Data points represent mean ± SEM (where n=6).
**Figure 10** - **10A****: Effect of different vitamin D3 and threonine concentrations on the expression of MUC 2; 10B:** Effect of different vitamin D3 and threonine concentrations on the expression of MUC 7.
   Data points represent mean ± SEM (n=6) where all data points were significantly different from the no treatment control (p<0.05, student t-test).
**Figure 11****- Evaluating GaRP in a 2,4,6-Trinitrobenzenesulfonic acid (TNBS) mouse model.** 2,4,6-Trinitrobenzenesulfonic acid solution (TNBS) colitis was induced in 5-week-old C56/BL/6 mice by rectal installation of TNBS (5% w/v) in 50% ethanol. The treatment schedule and experimental process is outlined in Figure 11.
**Figure 12** - **Comparing the change in body weight at experimental endpoint.**
   Colitis was induced in C56/BL/6 mice, 4 days after disease induction daily treatments commenced. Mice were orally administered bromelain (75mg/kg) ± 15mg/kg menthol and/or rectally administered GaRP. Body weights were taken daily where experimental endpoint changes in body mass were determined by comparing the endpoint weight to the baseline Day 4 weight (just prior to commencement of treatment). Each point represents the mean ± SD of 5-9 animals.

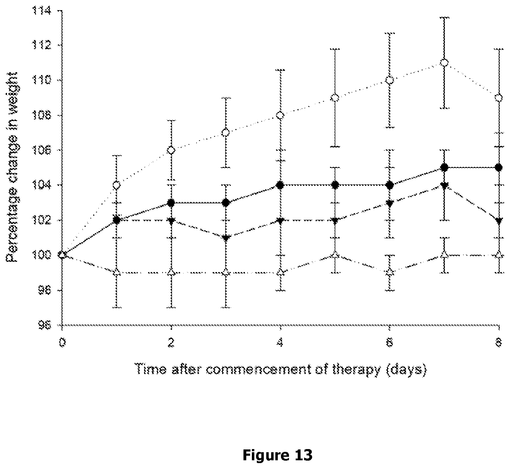

**Figure 13** - **Comparing the effect of formulations containing 75mg/kg bromelain & 15mg/kg menthol with vehicle controls.** Colitis was induced in C56/BL/6 mice and 4 days after disease induction daily treatments commenced. Treatments were oral vehicle ( ●-● ), 75mg/kg bromelain in water (○--○), 75mg/kg bromelain + 15mg/kg menthol in oral vehicle (▼--▼) or 75mg/kg bromelain + 15mg/kg menthol in oral vehicle and GaRP rectally administered (△---△). Each point represents the mean ± SD of 8-9 animals.

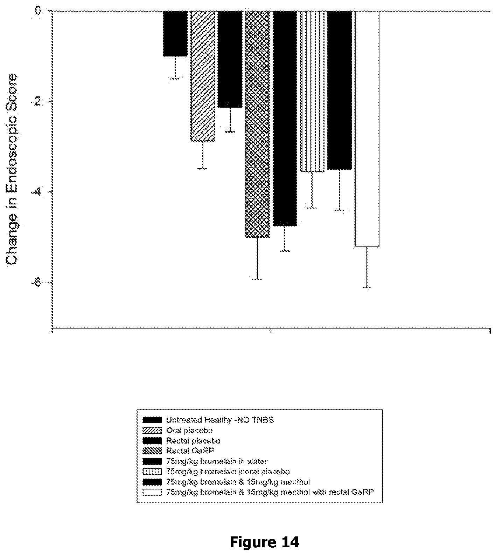

**Figure 14** - **Comparing the effect of the GaRP formulation and vehicle controls on the reduction in endoscopic score.** Colitis was induced in C56/BL/6 mice and 4 days after disease induction daily treatments commenced. Each point represents the mean ± SD of 5-9 animals. * Indicate statistical significance when compared to the oral or rectal vehicle control.

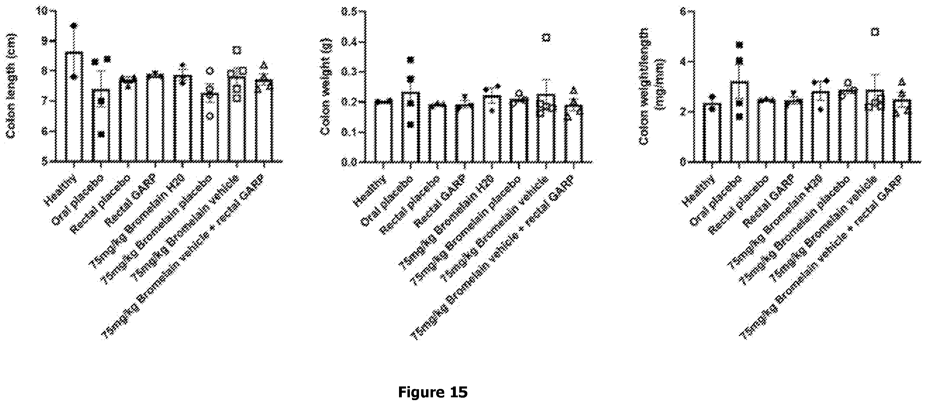

**Figure 15** - **Disease activity in experimental colitis. (A) Colon length, (B) colon weight, (C) colon weight/length ratio.** Colitis was induced in C56/BL/6 mice and 4 days after disease induction daily treatments commenced. At experimental endpoint the animals were euthanised and the colon removed, weighed and measured. Data presented as means ± SEM (n=5-9 animals).

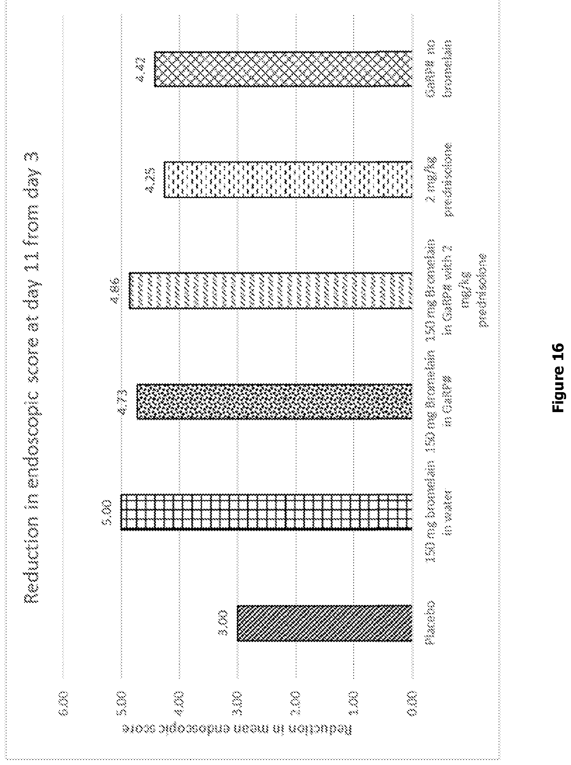

**Figure 16** - **Endoscopic scores.** Colitis was induced in C56/BL/6 mice and 3 days after disease induction daily treatments commenced. Each bar represents the mean improvement in endoscopic score at day 11 for the treatment groups. Values of the number of animals, means, standard deviations and statistical significance relative to placebo are shown in parentheses and the means are shown on top of each bar. Treatments shown are combined oral & rectal oral vehicle (n=34, x̅ 3.00, σ 2.00), 150mg/kg bromelain in water (n=8, x̅=5.00, σ=1.41, p=0.0036), 150mg/kg bromelain + 15mg/kg menthol in oral vehicle and GaRP rectally administered (n=11, x̅=4.73, σ=2.42, p=0.0294), 150mg/kg bromelain + 15mg/kg menthol in oral vehicle and 2 mg/kg prednisolone and GaRP rectally administered (n=7, x̅=4.86, σ=1.36, p=0.0080), 2 mg/kg prednisolone rectally administered (n=12, x̅=4.25, σ=2.38, p=0.0445),and GaRP rectally administered (n=19, x̅=4.42, σ=2.32, p=0.0167).

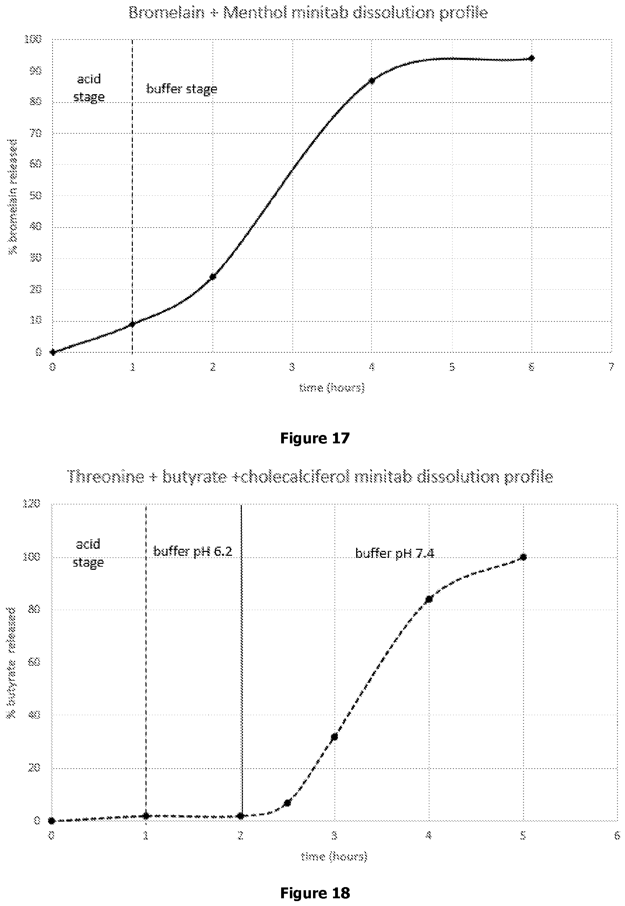

**Figure 17** - **Dissolution profile of an enterically coated type A tablet containing bromelain and menthol.** Bromelain/menthol minitablets prepared according to the methods of example 13 were subjected to USP dissolution testing conditions and demonstrated acceptable stability in the acid stage followed by sustained release starting at a pH equivalent to post gastric clearance and continuing over 3 hours.
**Figure 18** - **Dissolution profile of an enterically coated type B tablet containing sodium butyrate, threonine and vitamin D3.** Minitablets prepared according to the methods of example 13 were subjected to modified USP dissolution testing conditions in which the composition was exposed to acid (1N HCl) for 1 hour, buffer at pH 6.2 (1 hour) and buffer at pH 7.4 (5 hours). The figure shows acceptable stability in the acid and pH 6.2 stages followed by sustained release starting at pH 7.4 equivalent to the pH seen in the lower small intestines and large intestines.

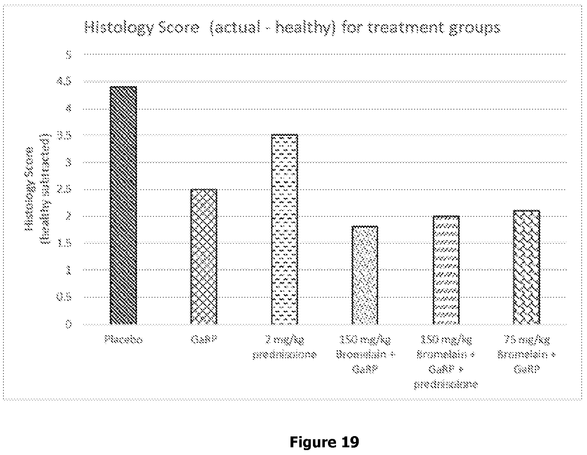

**Figure 19** - **Histology scores.** Colitis was induced and treated C56/BL/6 mice from examples 11 and 12 were euthanised post treatment and the colons sectioned for histopathological analysis. Each bar represents the mean histopathology score for the treatment groups. Values of the number of animals, means, standard deviations and statistical significance relative to placebo are shown in parentheses. Treatments shown are placebo (n=16, x̅ 4.4, σ 2.2), GaRP (n=10, x̅ 2.5, σ 2.4, p=0.034), prednisolone (n=10, x̅ 3.5, σ 2.5, p=0.196), 150mg/kg bromelain + GaRP (n=10, x̅=1.8, σ=2.6, p=0.012), 150mg/kg bromelain + GaRP + prednisolone (n=7, x̅=2.0, σ=1.5, p=0.005), 75mg/kg bromelain + GaRP (n=8, x̅=2.1, σ=2.8, p=0.045).

### DETAILED DESCRIPTION OF THE INVENTION

### Definitions

The following definitions are presented to better define the present invention and as a guide for those of ordinary skill in the art in the practice of the present invention.

Unless otherwise specified, all technical and scientific terms used herein are to be understood as having the same meanings as is understood by one of ordinary skill in the relevant art to which this disclosure pertains.

It is also believed that practice of the present invention can be performed using standard cell biology, microbiological, molecular biology, pharmacology and biochemistry protocols and procedures as known in the art, and as described, for example in numerous commonly available reference materials relevant in the art to which this disclosure pertains.

The term "food" means any substance, whether processed, semi-processed or raw, which is intended for consumption by animals including humans and includes, but is not limited to, solid and semi-solid foods, drinks, beverages and chewing gum. A food composition of the invention comprises a composition as described herein in combination with food.

The term "dietary supplement" refers to a product intended to supplement the diet that includes one or more of the following ingredients: enzymes, vitamins, minerals, herbs, metabolites, extracts and the like. A dietary supplement is not typically intended to be used as the sole item of a meal but can be consumed independently of any food. A dietary supplement of the invention comprises a composition as described herein in combination with one or more dietary supplements.

The term "medical food" refers to a food which is formulated to be consumed or administered enterally (e.g., via a feeding or nasogastric tube) under the supervision of a medical practitioner. Medical foods are intended for the dietary management of conditions which have distinctive nutritional requirements. Examples of medical foods include, but are not limited to, sole source nutrition products, oral rehydration solutions and products intended for use in dietary management of metabolic disorders. A medical food as contemplated herein comprises a composition as described herein that is formulated as a medical food.

One or more consumable excipients may be present in a nutraceutical formulation, food composition, dietary supplement or medical food as described herein. Such consumable excipients may include carriers, diluents, binders, adhesives, lubricants, plasticizers, disintegrants, colourants, bulking agents, flavouring agents, sweeteners, buffering agents, absorbents and the like as would be apparent to the skilled worker.

Examples of binders that may be used in compositions and oral dosages as described herein include cellulose derivatives such as ethylcellulose, hydroxypropyl cellulose, or hydroxypropyl methylcellulose, povidone, crospovidone, pharmaceutical glaze, gums, milk derivatives such as whey and casein, starches and starch derivatives. Sweetening agents may include dipeptide-based sweeteners, water-soluble natural sweeteners and artificial sweeteners. Flavouring agents may include flavour oils, both synthetic and natural, including peppermint oil, spearmint oil, cinnamon oil, oil of wintergreen; natural and artificial fruit flavours such as citrus oils including lemon, lime, grapefruit and orange, and fruit essences including cherry, pineapple, strawberry, raspberry, apple and the like. Bulking substances may include glucose, sucrose, lactose, starch, gelatine and silicon dioxide. All of the above binders, sweetening agents, flavouring agents and bulking substances are examples, and non-limiting.

The terminology "different type of pill, tablet, minitab, capsule, granule, particle or microparticle" and grammatical variations thereof as used herein means a given pill, tablet, minitab, capsule, granule, particle or microparticle as described herein as a "type" (including Type A, Type B and Type C as described herein) is formulated to comprise or consist essentially of different components, the components included in each type then being delivered to, and being active in different parts of the intestine. In one embodiment the different parts of the intestine are the duodenum and the colon.

As used herein, the terms "treat", "treating" and "treatment" refer to therapeutic measures which reduce, alleviate, ameliorate, manage, prevent, restrain, stop or reverse the symptoms caused by or associated with IBD and/or IBS, including the symptoms associated with or related to IBD and/or IBS. The subject may show observable or measurable (statistically significant) decrease in one or more of the symptoms associated with or related IBD and/or IBS as known to those skilled in the art, as indicating improvement.

The term "effective amount" as used herein means an amount effective to protect against, delay, reduce, stabilize, improve or treat at least one symptom of IBD and/or IBS as known in the art, and/or as described herein. In some embodiments, an effective amount is an amount sufficient to achieve a statistically different result as compared to an untreated control or placebo-treated control.

A "therapeutically effective amount" of a composition as described herein is an amount that is sufficient to achieve at least a lessening of at least one symptom associated with IBD and/or IBS that is being or is to be treated or that is sufficient to achieve a change in that symptom that has a therapeutically beneficial effect. Examples of changes in symptoms that are contemplated herein include a reduction in gastrointestinal inflammation, an increase in the thickness of the gastrointestinal mucosal layer and/or an improvement in gastrointestinal barrier integrity, but not limited thereto.

As used herein, the terms "manage", "managing" and "management" in the context of the administration of therapy to a subject refer to the beneficial effects that a subject derives from the therapy, while not resulting in a cure of the condition. For example, management of the condition includes preventing a worsening of the condition.

As used herein, the terms "prevent", "preventing" and "prevention" in the context of the administration of therapy to a subject refer to the prevention or inhibition of the recurrence, onset or development of the condition resulting from administration of the therapy.

The term "about" when used in connection with a referenced numeric indication means the referenced numeric indication plus or minus up to 10% of that referenced numeric indication. For example, "about 100" means from 90 to 110 and "about six" means from 5.4 to 6.6.

The term "comprising" as used in this specification means "consisting at least in part of". When interpreting statements in this specification that include that term, the features, prefaced by that term in each statement, all need to be present but other features can also be present. Related terms such as "comprise" and "comprised" are to be interpreted in the same manner.

The term "consisting essentially of" as used herein means the specified materials or steps and those that do not materially affect the basic and novel characteristic(s) of the claimed invention.

The term "consisting of" as used herein means the specified materials or steps of the claimed invention, excluding any element, step, or ingredient not specified in the claim.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

### Description

Gastrointestinal disease and disorders such as irritable bowel syndrome (IBS) and inflammatory bowel disease (IBD) are caused by a number of different factors in an individual, including microbial, immunologic, genetic and environmental factors.

These three factors contribute to the initiation and chronic persistence of IBD, IBS and associated symptoms such as altered bowel habits (ie, diarrhoea, constipation or a mixture thereof), abdominal pain and bloating:
- loss of gastrointestinal microbial homeostasis
- mucosal damage
- inflammation.

There are currently very few products, including dietary supplements, available on the market that can successfully re-establish the homeostasis of the gastrointestinal microbiome, reduce inflammation and promote mucosal healing. Re-establishing microbiome homeostasis is considered beneficial for reducing the disease effects associated with diarrhoea, inflammation, obesity, diabetes, cardiovascular disease (Vamanu, 2018).

However, the inventor has now discovered a composition of agents that work together to act on all three of these causative factors, providing effective management of chronic bowel conditions such as IBD and IBS.

Disclosed herein is Gastrointestinal Reprogramming Product (GaRP). GaRP is an all-natural dietary product specifically developed by the inventor to support and maintain gastrointestinal health. GaRP as described herein can be formulated as, or included in, a dietary supplement, medical food or medicinal product for addressing the primary underlying factors associated with gastrointestinal disorders. Without wishing to be bound by theory the inventor believes that GaRP acts by supporting an individual's gastrointestinal microbiota and restoring normal microbial gut function. In addition, the formulation components reduce inflammation which is critical for restoring the normal functioning of the gastrointestinal tract.

Accordingly, in one aspect, the invention relates to a composition comprising the following components:
(a) bromelain,
(b) one or more SCFAs or esters thereof,
(c) one or more amino acids selected from the group consisting of L-threonine, L-tryptophan, L-arginine, L-glutamine and L-methionine and combinations thereof, and
(d) Vitamin D or a metabolite thereof which is alfacalcidiol.

In one embodiment the composition consists essentially of the following components:
(a) bromelain,
(b) one or more SCFAs or esters thereof,
(c) one or more amino acids selected from the group consisting of L-threonine, L-tryptophan, L-arginine, L-glutamine and L-methionine and combinations thereof, and
(d) Vitamin D or a metabolite thereof which is alfacalcidiol.

In one embodiment (c) comprises or consists essentially of one or more amino acids selected from L-tryptophan, L-arginine, L-glutamine and L-methionine and combinations thereof.

In one embodiment (c) comprises or consists essentially of L-tryptophan, L-arginine, L-glutamine and L-methionine. In one embodiment (c) comprises or consists essentially of L-arginine, L-glutamine and L-methionine. In one embodiment (c) comprises or consists essentially of L-tryptophan, L-glutamine and L-methionine. In one embodiment (c) comprises or consists essentially of L-tryptophan, L-arginine, and L-methionine. In one embodiment (c) comprises or consists essentially of L-tryptophan, L-arginine and L-glutamine.

In one embodiment (c) comprises or consists essentially of L-tryptophan and L-arginine. In one embodiment (c) comprises or consists essentially of L-tryptophan and L-glutamine. In one embodiment (c) comprises or consists essentially of L-tryptophan and L-methionine. In one embodiment (c) comprises or consists essentially of L-arginine and L-glutamine. In one embodiment (c) comprises or consists essentially of L-arginine and L-methionine. In one embodiment (c) comprises or consists essentially of L-glutamine and L-methionine.

In one embodiment (c) comprises or consists essentially of L-tryptophan, L-arginine, L-glutamine or L-methionine.

In another aspect, the invention relates to a composition comprising the following components:
(a) bromelain,
(b) one or more SCFAs or esters thereof,
(c) L-threonine, and
(d) Vitamin D or a metabolite thereof which is alfacalcidiol.

In one embodiment the composition consists essentially of the following components:
(a) bromelain,
(b) one or more SCFAs or esters thereof,
(c) L-threonine, and
(d) Vitamin D or a metabolite thereof which is alfacalcidiol.

In one embodiment the L-threonine in (c) is substituted for one or more different amino acids selected from the group consisting of L-tryptophan, L-arginine, L-glutamine and L-methionine.

In one embodiment the composition comprises at least one additional amino acid component: L-tryptophan, L-arginine, L-glutamine or L-methionine or any combination thereof.

In one embodiment the composition consists essentially of the components: bromelain, a SCFA, L-threonine and Vitamin D or a metabolite thereof which is alfacalcidiol.

In one embodiment the one or more SCFA or ester thereof is butyric acid, propionic acid or acetic acid, or a physiologically acceptable salt thereof. Preferably, the SCFA is sodium butyrate, calcium butyrate, sodium propionate or sodium acetate, preferably sodium butyrate.

In one embodiment the composition consists essentially of the components: bromelain, butyrate, preferably sodium butyrate, L- threonine and Vitamin D or a metabolite thereof which is alfacalcidiol.

In one embodiment the composition comprises as a component: menthol.

In one embodiment the composition is a dietary supplement, nutraceutical, or food. In one embodiment the food is a medical food.

In one embodiment the composition comprises one or more additional component that is a consumable excipient.

### Components

The inventor has surprisingly determined that the various components of the composition of the invention act synergistically to restore normal gastrointestinal functioning, reducing the symptoms of IBD and IBS, in particular diarrhea.

Bromelain is the collective name for a proteolytic extract obtained from the stems of pineapple (*Ananus comosus*). The term "bromelain" is also used to refer to the two main proteolytic enzymes present in pineapple - stem bromelain protease (CAS 37189-34-7/EC 3.4.22.32), and fruit bromelain protease (EC 3.4.22.33). While the names reflect their primary location in the plant, both enzymes are present in extracts of stem bromelain. As well as stem bromelain protease and fruit bromelain protease, bromelain may contain the proteases ananain, cosmosain and canazain, as well as small amounts of other compounds.

Bromelain extract is prepared by blending the peel, stem, leaves and other non-edible parts of the pineapple and filtering the mixture to obtain a liquid containing the soluble bromelain enzymes, which may then be purified, fractionated, concentrated and/or dried. Bromelain can be fractionated using ion exchange chromatography. Fractions associated with major absorbance peaks can be subjected to SDS-PAGE to isolate and identify individual enzymes.

As used herein, the term "bromelain" refers to an extract of proteolytic enzymes obtained from the stems of pineapple, including the crude extract, purified extract or any fraction or combination of fractions thereof.

While bromelain has not been approved by the US Food and Drug Administration for the treatment of any gastrointestinal disorder, it is a well-known dietary supplement, suggested to be useful as a digestive aid, and general anti-inflammatory. It has also been suggested to be useful in supporting the body's response to stress, aiding the digestion of proteins, reducing swelling of the nose and sinuses, removing dead and damaged tissue after burns, relaxing muscles, preventing pulmonary edema, stimulating muscle contractions, and shortening labour; as well as treating joint pain, sinus infections, allergies, asthma, cancer, varicose veins, haemorrhoids, urinary tract infections, bronchitis, gout, fever and Carpel tunnel syndrome.

The composition of the invention combines bromelain with specific agents that in combination, reduce the three causative factors of inflammatory gastrointestinal diseases IBD and IBS.

In one embodiment the composition is a unit dosage form that comprises about 50 mg to about 2500 mg, preferably about 250 to about 2250 mg, about 500 to about 2000 mg, about 750 to about 1750 mg, about 1000 to about 1600 mg, about 1300 to about 1550 mg, about 1400 to about 1500 mg, about 1420 to about 1460 mg, preferably about 1440 mg bromelain. In one embodiment the composition comprises about 1440 mg, preferably about 1030 mg, preferably about 720 mg bromelain.

In one embodiment the composition is a unit dosage form that comprises about 460 mg to about 500 mg bromelain, preferably about 480mg bromelain.

In one embodiment the composition is a unit dosage form that comprises 460 mg to 500 mg bromelain, preferably 480 mg bromelain.

In one embodiment the composition is a unit dosage form that comprises 50 mg to 2500 mg, preferably 250 to 2250 mg, 500 to 2000 mg, 750 to 1750 mg, 1000 to 1600 mg, 1300 to 1550 mg, 1400 to 1500 mg, 1420 to 1460 mg, preferably 1440 mg, 1030 mg, preferably 720 mg bromelain. In one embodiment the composition comprises 1440 mg, preferably 1030 mg, preferably 720 mg bromelain.

Short chain fatty acids (SCFAs) are the end products of microbial fermentation of macronutrients such as plant polysaccharides that cannot be digested by humans who lack the necessary glycoside hydrolases and polysaccharide lyases. The microbiome supplies the missing enzymes, producing SCFA which may promote intestinal epithelial barrier integrity. SCFAs such as butyrate may also help restore gut microbial homeostasis.

An increased concentration of SCFAs promotes re-growth of beneficial bacteria that reduce inflammation via the inhibition of NF-κB and the reduction of pro-inflammatory cytokines.

The terms "short chain fatty acid" and "SCFA" refer to weak acids containing from 2 to 7 carbon atoms but also include the anion and salt form of the particular base acid. Referring to the anion, SCFAs include acetate, butyrate, propionate, valerate, caproate, isobutyrate, 2-methyl-isobutylrate and isovalerate. SCFA salts for use in the composition of the invention include the alkali metals (e.g., Na and K) and alkaline earth metals, (e.g., Mg and Ca).

The term "SCFA esters thereof" refers to esters of SCFAs. SCFA esters for use in the composition of the invention include methyl, ethyl and the like.

The SCFAs and selected amino acids present in the composition of the invention promote regeneration of the intestinal mucosa which renews the integrity of the gastrointestinal tract and gut microbiome.

In one embodiment the composition is an unit dosage form that comprises about 45 to 1800 mg SCFA, preferably about 75 to about 1650 mg, about 250 to about 1500, about 500 to about 1400, about 750 to about 1500, about 1000 to about 1400, about 1100 to about 1300 mg, preferably about 1200 mg SCFA. In one embodiment the composition comprises about 1200 mg, preferably about 600 mg, preferably about 300 mg SCFA .

In one embodiment the composition is a unit dosage form that comprises 45 to 1800 mg SCFA, preferably 75 to 1650 mg, 250 to 1500, 500 to 1400, 750 to 1500, 1000 to 1400, 1100 to 1300 mg, preferably 1200 mg SCFA. In one embodiment the composition comprises 1200 mg, preferably 600 mg, preferably 300 mg SCFA.

In one embodiment the composition is a unit dosage form that comprises about 45 to 1800 mg sodium butyrate, sodium propionate or sodium acetate, preferably about 75 to about 1650 mg, about 250 to about 1500, about 500 to about 1400, about 750 to about 1500, about 1000 to about 1400, about 1100 to about 1300 mg, preferably about 1200 mg sodium butyrate. In one embodiment the composition comprises about 1200 mg, preferably about 600 mg, preferably about 300 mg sodium butyrate, sodium propionate or sodium acetate, preferably sodium butyrate.

In one embodiment the composition is a unit dosage form that comprises 45 to 1800 mg sodium butyrate, sodium propionate or sodium acetate, preferably 75 to 1650 mg, 250 to 1500, 500 to 1400, 750 to 1500, 1000 to 1400, 1100 to 1300 mg, preferably 1200 mg sodium butyrate. In one embodiment the composition comprises 1200 mg, preferably 600 mg, preferably 300 mg sodium butyrate sodium propionate or sodium acetate, preferably sodium butyrate.

In one embodiment the composition is a unit dosage form that comprises about 600 mg sodium butyrate. In one embodiment the composition is a unit dosage form that comprises 600 mg sodium butyrate.

L-Threonine is included as a component of a composition as described herein. Threonine is an essential amino acid used in the biosynthesis of proteins, produces collagen and may benefit immune health.

Additional amino acids may also be included in a composition as described herein. In some embodiments, L-threonine may be substituted in the composition by another amino acid selected from the group consisting of L-tryptophan, L-glutamine, L-arginine and L-methionine. Without wishing to be bound by theory the inventor believes that each amino acid selected from L-threonine, L-tryptophan, L-glutamine, L-arginine and L-methionine, as well as any combination thereof in the composition may reduce inflammation and/or promote mucosal healing. Preferably the amino acid is L-threonine. In conjunction with bromelain, these amino acids may also act to reduce pro-inflammatory cytokines which promotes reversion of the microbiome and reduces inflammation.

In one embodiment the composition is a unit dosage form that comprises about 67.5 to about 2700 mg, preferably about 112.5 to about 2250 mg, about 250 to about 2000 mg, about 500 to about 1900 mg, about 900 to about 1800 mg, preferably about 1800 mg L-threonine, L-tryptophan, L-glutamine, L-arginine or L-methionine or any combination thereof. In one embodiment the composition comprises about 1800 mg, preferably about 900 mg, preferably about 450 mg L-threonine, L-tryptophan, L-glutamine, L-arginine or L-methionine or any combination thereof.

In one embodiment the composition is a unit dosage form that comprises about 67.5 to about 2700 mg, preferably about 112.5 to about 2250 mg, about 250 to about 2000 mg, about 500 to about 1900 mg, about 900 to about 1800 mg, preferably about 1800 mg L-threonine. In one embodiment the composition comprises about 1800 mg, preferably about 900 mg, preferably about 450 mg L-threonine.

In one embodiment the composition is a unit dosage form that comprises 67.5 to 2700 mg, preferably 112.5 to 2250 mg, 250 to 2000 mg, 500 to 1900 mg, 900 to 1800 mg, preferably 1800 mg L-threonine, L-tryptophan, L-glutamine, L-arginine or L-methionine or any combination thereof. In one embodiment the composition comprises 1800 mg, preferably 900 mg, preferably 450 mg L-threonine, L-tryptophan, L-glutamine, L-arginine or L-methionine or any combination thereof.

In one embodiment the composition is a unit dosage form that comprises 67.5 to 2700 mg, preferably 112.5 to 2250 mg, 250 to 2000 mg, 500 to 1900 mg, 900 to 1800 mg, preferably 1800 mg L-threonine. In one embodiment the composition comprises 1800 mg, preferably 900 mg, preferably 450 mg L-threonine.

Vitamin D is included as a component in the composition described herein. Vitamin D has anti-inflammatory effects and can also help move the microbiome back into homeostasis as well as tighten gap junctions between the I cells of the gastrointestinal tract which reduces gut permeability.

The term "Vitamin D" refers to a group of fat-soluble secosteroids including vitamin D2 (ergocalciferol) and vitamin D3 -1,25dihydroxyvitamin D3 (cholecalciferol); vitamin D without a subscript refers to either D2 or D3 or both. Both forms of vitamin D are known collectively as calciferol. The active metabolite of vitamin D known as alfacalcidiol which does not require the second hydroxylation step in the kidney.

In one embodiment the composition is an unit dosage form that comprises about 4 to about 150 ug, about 6.5 to about 140 ug, about 13 to about 130 ug, about 25 to about 120 ug, about 35 to about 110 ug, about 50 to about 105 ug, preferably about 100 ug, preferably about 50 ug, preferably about 25 ug, preferably about 12.5 ug Vitamin D.

In one embodiment the composition is a unit dosage form that comprises about 4 to 150 ug, 6.5 to 140 ug, 13 to 130 ug, 25 to 120 ug, 35 to 110 ug, 50 to 105 ug, preferably 100 ug, preferably 50 ug, preferably 25 ug, preferably 12.5 ug Vitamin D.

In one embodiment vitamin D is Cholecalciferol (vitamin D3).

Menthol may be included as component in a composition as described herein may be an organic compound made synthetically or obtained from mint oils, such as peppermint. Both peppermint leaves and the essential oil from peppermint have been used for health purposes.

In one embodiment the composition is an unit dosage form that comprises about 3 to about 150 mg, preferably about 6 to about 145 mg, about 12 to about 140 mg, about 24 to about 135 mg, 48 to about 130 mg, about 60 to about 125 mg, about 80 to about 120 mg, about 90 to about 110 mg, about 100 mg, preferably about 96 mg menthol. In one embodiment the composition comprises about 100, preferably about 96, preferably about 50 mg, preferably about 48 mg menthol.

In one embodiment the composition is an unit dosage form that comprises 3 to 200 mg, preferably 6 to 150 mg, 12 to 145 mg, 24 to 140 mg, 48 to 135 mg, 60 to 130 mg, 80 to 120 mg, 90 to 110 mg, 100 mg, preferably 96 mg menthol. In one embodiment the composition comprises 100, preferably 96, preferably 50 mg, preferably 48 mg menthol.

In one embodiment the menthol is L-menthol. In one embodiment the menthol is D-menthol.

The compositions disclosed herein may be presented in unit dosage form and may be prepared by any of the methods well known in the art of formulation and pharmacy. The term "unit dosage form" means a single dose to be given to a patient wherein all active agents and inactive ingredients are combined in a suitable system, such that the single dose is available in a single container or package, the entire "unit dosage form" being contained therein. The "unit dosage form" does not encompass mixing any components together from two or more containers or packages.

Typical examples of unit dosage forms are pills, tablets, minitabs, capsules, granules, particles and/or microparticles for oral administration of a composition as described herein (i.e., an oral dosage form), or transdermal patches comprising a unit dosage of a composition as described herein (i.e., a topical dosage form). These examples of unit dosage forms are not intended to be limiting in any way, but merely to represent typical examples in the pharmacy arts of unit dosage forms.

Accordingly, in another aspect the invention relates to a unit dosage form for treatment or prophylaxis of IBD or IBS comprising about 1 to about 10 g of a composition of the invention. In one embodiment the unit dosage form is an oral dosage form.

In one embodiment the oral dosage form comprises about 1 to about 8 g, about 1 to about 7 g, about 1 to about 6 g, about 1 to about 5 g, about 1 to about 4, about 1 to 3 g of the composition. In one embodiment the oral dosage form comprises 1 to 8 g, 1 to 7 g, 1 to 6 g, 1 to 5 g, 1 to 4, 1 to 3 g of the composition.

In one embodiment the oral dosage form comprises about 4 g to about 4.5 g of the composition, preferably about 4.22 g of the composition.

In one embodiment the oral dosage form comprises 4 g to 4.5 g of the composition, preferably 4.22 g of the composition.

In one embodiment the oral dosage form comprises about 2 g to about 2.5 g of the composition, preferably about 2.21g of the composition.

In one embodiment the oral dosage form comprises 2 g to 2.5 g of the composition, preferably 2.21g of the composition.

In one embodiment the oral dosage form consists essentially of about 1 to about 8 g, about 1 to about 7 g, about 1 to about 6 g, about 1 to about 5 g, about 1 to about 4, about 1 to 3 g of the composition. In one embodiment the oral dosage form consists essentially of 1 to 8 g, 1 to 7 g, 1 to 6 g, 1 to 5 g, 1 to 4, 1 to 3 g of the composition.

In one embodiment the oral dosage form consists essentially of about 1 to about 10 g of the composition, about 1.25 to about 8 g, preferably about 1.5 to about 6 g, of the composition. In one embodiment the oral dosage form consists essentially of 1 to 10 g of the composition, 1 to 8 g, preferably 1.5 to 6 g of the composition.

In one embodiment the oral dosage form consists essentially about 4 g to about 4.5 g of the composition, preferably about 4.22 g of the composition.

In one embodiment the oral dosage form consists essentially 4 g to 4.5 g of the composition, preferably 4.22 g of the composition.

In one embodiment the oral dosage form consists essentially about 2 g to about 2.5 g of the composition, preferably about 2.21g of the composition.

In one embodiment the oral dosage form consists essentially 2 g to 2.5 g of the composition, preferably 2.21g of the composition.

In one embodiment, the oral dosage form comprises 50-2500 mg bromelain, preferably 720 mg bromelain, 45-1800 mg sodium butyrate, preferably 300 mg sodium butyrate, 67.5-2700 mg L-threonine, preferably 450 mg L-threonine, 4-150 µg Vitamin D, preferably 25 µg Vitamin D, and optionally 3-150 mg menthol, preferably 48 mg menthol.

In one embodiment, the oral dosage form comprises about 460 mg to about 500 mg bromelain, preferably about 480 mg bromelain, about 550 mg to about 650 mg sodium butyrate, preferably about 600 mg sodium butyrate, about 850 mg L-threonine to about 950 mg L-threonine, preferably about 900 mg L-threonine, about 10 ug Vitamin D to about 15 µg Vitamin D, preferably about 12.5 µg Vitamin D, and optionally about 65 mg menthol to about 75 mg menthol, preferably about 70 mg menthol.

In one embodiment, the oral dosage form comprises 460 mg to 500 mg bromelain, preferably 480 mg bromelain, 550 mg to 650 mg sodium butyrate, preferably 600 mg sodium butyrate, 850 mg L-threonine to 950 mg L-threonine, preferably 900 mg L-threonine, 10 ug Vitamin D to 15 µg Vitamin D, preferably 12.5 µg Vitamin D, and optionally 65 mg menthol to 75 mg menthol, preferably about 70 mg menthol.

In one embodiment, the oral dosage form consists essentially of about 460 mg to about 500 mg bromelain, preferably about 480 mg bromelain, about 550 mg to about 650 mg sodium butyrate, preferably about 600 mg sodium butyrate, about 850 mg L-threonine to about 950 mg L-threonine, preferably about 900 mg L-threonine, about 10 ug Vitamin D to about 15 µg Vitamin D, preferably about 12.5 µg Vitamin D, and optionally about 65 mg menthol to about 75 mg menthol, preferably about 70 mg menthol.

In one embodiment, the oral dosage form consists essentially of 460 mg to 500 mg bromelain, preferably 480 mg bromelain, 550 mg to 650 mg sodium butyrate, preferably 600 mg sodium butyrate, 850 mg L-threonine to 950 mg L-threonine, preferably 900 mg L-threonine, 10 ug Vitamin D to 15 µg Vitamin D, preferably 12.5 µg Vitamin D, and optionally 65 mg menthol to 75 mg menthol, preferably about 70 mg menthol

In one embodiment, the oral dosage form comprises about 700 mg to about 740 mg bromelain, preferably about 720 mg bromelain, about 550 mg to about 650 mg sodium butyrate, preferably about 600 mg sodium butyrate, about 850 mg L-threonine to about 950 mg L-threonine, preferably about 900 mg L-threonine, about 10 ug Vitamin D to about 15 µg Vitamin D, preferably about 12.5 µg Vitamin D, and optionally about 100 mg menthol to about 110 mg menthol, preferably about 105 mg menthol.

In one embodiment, the oral dosage form comprises 700 mg to 740 mg bromelain, preferably 720 mg bromelain, 550 mg to 650 mg sodium butyrate, preferably 600 mg sodium butyrate, 850 mg L-threonine to 950 mg L-threonine, preferably 900 mg L-threonine, 10 ug Vitamin D to 15 µg Vitamin D, preferably 12.5 µg Vitamin D, and optionally 100 mg menthol to 110 mg menthol, preferably 105 mg menthol.

In one embodiment, the oral dosage form consists essentially of about 700 mg to about 740 mg bromelain, preferably about 720 mg bromelain, about 550 mg to about 650 mg sodium butyrate, preferably about 600 mg sodium butyrate, about 850 mg L-threonine to about 950 mg L-threonine, preferably about 900 mg L-threonine, about 10 ug Vitamin D to about 15 µg Vitamin D, preferably about 12.5 µg Vitamin D, and optionally about 100 mg menthol to about 110 mg menthol, preferably about 105 mg menthol.

In one embodiment, the oral dosage form consists essentially of 700 mg to 740 mg bromelain, preferably 720 mg bromelain, 550 mg to 650 mg sodium butyrate, preferably 600 mg sodium butyrate, 850 mg L-threonine to 950 mg L-threonine, preferably 900 mg L-threonine, 10 ug Vitamin D to 15 µg Vitamin D, preferably 12.5 µg Vitamin D, and optionally 100 mg menthol to 110 mg menthol, preferably 105 mg menthol.

In one embodiment the oral dosage form is formulated for administration of 4.22 grams of the dosage form twice per day per subject. In this embodiment, the oral dosage from provides a total daily dose of menthol 140 mg, bromelain 960 mg, threonine 1800 mg, sodium butyrate 1200 mg and cholecalciferol 25 micrograms.

In another embodiment the oral dosage form is formulated for administration of 2.21 grams twice per day per subject. In this embodiment the oral dosage from provides a total daily dose of menthol 70 mg, bromelain 480 mg, threonine 900 mg, sodium butyrate 600 mg and cholecalciferol 12.5 micrograms.

In one embodiment the oral dosage form comprises at least one enterically coated pill, tablet, minitab, capsule, granule, particle or microparticle or any combination thereof comprising 1440mg of bromelain and 288mg menthol targeted to the small intestine and at least one enterically coated pill, tablet, minitab, capsule, granule, particle or microparticle or any combination thereof comprising a colon-targeted dose of 1200mg butyric acid (sodium salt), 1800mg of L-threonine and 0.025mg of Vitamin D3.

In one embodiment the oral dosage form comprises at least one enterically coated pill, tablet, minitab, capsule, granule, particle or microparticle or any combination thereof comprising about 480 mg of bromelain and about 70 mg menthol targeted to the small intestine and at least one enterically coated pill, tablet, minitab, capsule, granule, particle or microparticle or any combination thereof comprising a colon-targeted dose of about 600 mg butyric acid (sodium salt), about 900 mg of L-threonine and about 0.0125mg of Vitamin D3.

In one embodiment the oral dosage form comprises at least one enterically coated pill, tablet, minitab, capsule, granule, particle or microparticle or any combination thereof comprising 480 mg of bromelain and 70 mg menthol targeted to the small intestine and at least one enterically coated pill, tablet, minitab, capsule, granule, particle or microparticle or any combination thereof comprising a colon-targeted dose of 600 mg butyric acid (sodium salt), 900 mg of L-threonine and 0.0125mg of Vitamin D3.

In one embodiment the oral dosage form consists essentially of at least one enterically coated pill, tablet, minitab, capsule, granule, particle or microparticle or any combination thereof comprising about 480 mg of bromelain and about 70 mg menthol targeted to the small intestine and at least one enterically coated pill, tablet, minitab, capsule, granule, particle or microparticle or any combination thereof comprising a colon-targeted dose of about 600 mg butyric acid (sodium salt), about 900 mg of L-threonine and about 0.0125mg of Vitamin D3.

In one embodiment the oral dosage form consists essentially of at least one enterically coated pill, tablet, minitab, capsule, granule, particle or microparticle or any combination thereof comprising 480 mg of bromelain and 70 mg menthol targeted to the small intestine and at least one enterically coated pill, tablet, minitab, capsule, granule, particle or microparticle or any combination thereof comprising a colon-targeted dose of 600 mg butyric acid (sodium salt), 900 mg of L-threonine and 0.0125mg of Vitamin D3.

In one embodiment the oral dosage form comprises at least one enterically coated pill, tablet, minitab, capsule, granule, particle or microparticle or any combination thereof comprising about 960 mg of bromelain and about 140 mg menthol targeted to the small intestine and at least one enterically coated pill, tablet, minitab, capsule, granule, particle or microparticle or any combination thereof comprising a colon-targeted dose of about 1200 mg butyric acid (sodium salt), about 1800 mg of L-threonine and about 0.025mg of Vitamin D3.

In one embodiment the oral dosage form comprises at least one enterically coated pill, tablet, minitab, capsule, granule, particle or microparticle or any combination thereof comprising 960 mg of bromelain and 140 mg menthol targeted to the small intestine and at least one enterically coated pill, tablet, minitab, capsule, granule, particle or microparticle or any combination thereof comprising a colon-targeted dose of 1200 mg butyric acid (sodium salt), 1800 mg of L-threonine and 0.025mg of Vitamin D3.

In one embodiment the oral dosage form consists essentially of at least one enterically coated pill, tablet, minitab, capsule, granule, particle or microparticle or any combination thereof comprising about 960 mg of bromelain and about 140 mg menthol targeted to the small intestine and at least one enterically coated pill, tablet, minitab, capsule, granule, particle or microparticle or any combination thereof comprising a colon-targeted dose of about 1200 mg butyric acid (sodium salt), about 1800 mg of L-threonine and about 0.025mg of Vitamin D3.

In one embodiment the oral dosage form consists essentially of at least one enterically coated pill, tablet, minitab, capsule, granule, particle or microparticle or any combination thereof comprising 960 mg of bromelain and 140 mg menthol targeted to the small intestine and at least one enterically coated pill, tablet, minitab, capsule, granule, particle or microparticle or any combination thereof comprising a colon-targeted dose of 1200 mg butyric acid (sodium salt), 1800 mg of L-threonine and 0.025mg of Vitamin D3.

In one embodiment the oral dosage form provides the concentrations of components per enterocyte and bacteria contained within the gut microbiome as shown in Table 1.

In one embodiment the oral dosage form comprises one or more pills, tablets, minitabs, capsules, granules, particles or microparticles or any combination thereof.

In one embodiment the oral dosage form comprises a composition comprising or consisting essentially of two different types of pills, tablets, minitabs, capsules, granules, particles or microparticles, wherein each different type is formulated to release its contents to a different part of the gastrointestinal tract. In one embodiment the different parts of the gastrointestinal tract are the duodenum (also termed herein the "upper intestine") and the colon (also termed herein the "lower intestine").

The applicant has surprisingly found that the efficacy of a composition of the invention is increased where the components of the composition are formulated in two different types of pills, tablets, minitabs, capsules, granules, particles or microparticles (termed herein type A and type B), wherein each type is formulated for delivery of the components contained therein, to a specific part of the intestine.

In one example, the components of the composition work together to alleviate the symptoms of IBD and/or IBS by acting simultaneously to reduce pain in the upper intestine (type A) inflammation throughout the upper and lower intestine (type A) and restore tight junction function and mucin generation in the lower intestine (type B). The unexpected benefit of this approach devised by the applicant can be clearly seen in the improved endoscopic results obtained in mice (Examples 11 and 12) and the histopathology (Example 14).

In one embodiment the two different types of pills, tablets, minitabs, capsules, granules, particles or microparticles are Type A and Type B pills, tablets, minitabs, capsules, granules particles or microparticles, wherein the Type A and the Type B pills, tablets, minitabs, capsules, granules, particles or microparticles each comprise at least one component, preferably at least two components, of the composition described herein.

In one embodiment Type A and Type B pills, tablets, minitabs, capsules, granules, particles or microparticles each comprise at least one different component of the composition than each other, preferably at least two different components of the composition from each other.

In one embodiment the Type A pills, tablets, minitabs, capsules, granules, particles or microparticles comprise or consist essentially of bromelain. In one embodiment the Type A pills, tablets, minitabs, capsules, granules, particles or microparticles comprise menthol.

In one embodiment the Type A pills, tablets, minitabs, capsules, granules, particle or microparticles comprise an amount of bromelain that provides a dose of about 10 to 40 fg/bacteria, about 15 to 35 fg/bacteria, about 20 to 30 fg/bacteria, preferably about 28 fg/bacteria, preferably about 25 fg/bacteria, preferably about 20 fg/bacteria of bromelain in the ileum/small intestine of the subject. In one embodiment the dose is an effective dose.

In one embodiment the Type A pills, tablets, minitabs, capsules, granules, particle or microparticles comprise an amount of bromelain that provides a dose of 10 to 40 fg/bacteria, 15 to 35 fg/bacteria, 20 to 30 fg/bacteria, preferably 28 fg/bacteria, preferably 25 fg/bacteria, preferably 20 fg/bacteria of bromelain in the ileum/small intestine of the subject. In one embodiment the dose is an effective dose.

In one embodiment the effective dose is about 10 to 40 fg/bacteria, about 15 to 35 fg/bacteria, about 20 to 30 fg/bacteria, preferably about 28 fg/bacteria, preferably about 25 fg/bacteria, preferably about 20 fg/bacteria of bromelain in the ileum/small intestine of the subject.

In one embodiment the effective dose is 10 to 40 fg/bacteria, 15 to 35 fg/bacteria, 20 to 30 fg/bacteria, preferably 28 fg/bacteria, preferably 25 fg/bacteria, preferably 20 fg/bacteria of bromelain in the ileum/small intestine of the subject.

In one embodiment the Type A pill, tablet, minitab, granule, capsule, particle or microparticle comprises an amount of bromelain that provides a dose of about 25 to 125 pg/ileal enterocyte (pg/ie), about 50 to 100 pg/ie, about 60 to about 90 pg/ie, about 70 to 80 pg/ie, preferably about 75 pg/ie, in the ileum/small intestine of the subject. In one embodiment the dose is an effective dose.

In one embodiment the Type A pill, tablet, minitab, granule, capsule, particle or microparticle comprises an amount of bromelain that provides a dose of 25 to 125 pg/ileal enterocyte (pg/ie), 50 to 100 pg/ie, 60 to 90 pg/ie, 70 to 80 pg/ie, preferably 75 pg/ie, in the ileum/small intestine of the subject. In one embodiment the dose is an effective dose.

In one embodiment the Type A pill, tablet, minitab, granule, capsule, particle or microparticle comprises or consists essentially of about 400 mg to 1100 mg bromelain, about 600 mg to 1000 mg, about 700 mg to 900 mg, about 750 mg to 850 mg, preferably about 800 mg bromelain.

In one embodiment the Type A pill, tablet, minitab, granule, capsule, particle or microparticle comprises or consists essentially of 400 mg to 1100 mg bromelain, 600 mg to 1000 mg, 700 mg to 900 mg, 750 mg to 850 mg, preferably 800 mg bromelain.

In one embodiment the Type A pill, tablet, minitab, granule, capsule, particle or microparticle comprises or consists essentially of about 460 mg to about 500 mg bromelain, about 470 mg to about 490 mg, about 475 mg to about 485 mg, preferably about 480 mg bromelain.

In one embodiment the Type A pill, tablet, minitab, granule, capsule, particle or microparticle comprises or consists essentially of 460 mg to 500 mg bromelain, 470 mg to 490 mg, 475 mg to 485 mg, preferably 480 mg bromelain.

In one embodiment the Type A pill, tablet, minitab, granule, capsule, particle or microparticle comprises or consists essentially of about 700 mg to about 740 mg bromelain, about 710 mg to about 730 mg, about 715 mg to about 725 mg, preferably about 720 mg bromelain.

In one embodiment the Type A pill, tablet, minitab, granule, capsule, particle or microparticle comprises or consists essentially of 700 mg to 740 mg bromelain, 710 mg to 730 mg, 715 mg to 725 mg, preferably 720 mg bromelain.

In one embodiment the Type A pill, tablet, minitab, granule, capsule, particle or microparticle comprises menthol.

In one embodiment the Type B pill, tablet, minitab, capsule, granule, particle or microparticle comprises L-threonine, L-tryptophan, L-glutamine, L-arginine or L-methionine or any combination thereof, sodium butyrate and Vitamin D.

In one embodiment the Type B pill, tablet, minitab, capsule, granule, particle or microparticle comprises L-threonine, sodium butyrate and Vitamin D.

In one embodiment the Type B pill, tablet, minitab, capsule, granule, particle or microparticle consists essentially of L-threonine, L-tryptophan, L-glutamine, L-arginine or L-methionine or any combination thereof, sodium butyrate and Vitamin D.

In one embodiment the Type B pill, tablet, minitab, capsule, granule, particle or microparticle consists essentially of L-threonine, sodium butyrate and Vitamin D. In one embodiment Vitamin D is Vitamin D₃.

In one embodiment Vitamin D is Vitamin D₃.

In one embodiment the Type B pill, tablet, minitab, granule, capsule, particle or microparticles comprises or consists essentially of about 67.5 to about 2700 mg, preferably about 112.5 to about 2250 mg, about 250 to about 2000 mg, about 500 to about 1900 mg, about 900 to about 1800 mg, preferably about 1800 mg of L-threonine, L-tryptophan, L-glutamine, L-arginine or L-methionine or any combination thereof. In one embodiment the Type B pill, tablet, minitab, granule, capsule, particle or microparticle comprises or consists essentially of about 1800 mg, preferably about 900 mg, preferably about 450 mg of L-threonine, L-tryptophan, L-glutamine, L-arginine or L-methionine or any combination thereof.

In one embodiment the Type B pill, tablet, minitab, granule, capsule, particle or microparticles comprises or consists essentially of about 67.5 to about 2700 mg, preferably about 112.5 to about 2250 mg, about 250 to about 2000 mg, about 500 to about 1900 mg, about 900 to about 1800 mg, preferably about 1800 mg L-threonine. In one embodiment the Type B pill, tablet, minitab, granule, capsule, particle or microparticle comprises or consists essentially of about 1800 mg, preferably about 900 mg, preferably about 450 mg L-threonine.

In one embodiment the Type B pill, tablet, minitab, granule, capsule, particle or microparticle comprises or consists essentially of 67.5 to 2700 mg, preferably 112.5 to 2250 mg, 250 to 2000 mg, 500 to 1900 mg, 900 to 1800 mg, preferably 1800 mg L-threonine, L-tryptophan, L-glutamine, L-arginine or L-methionine or any combination thereof. In one embodiment the Type B pill, tablet, minitab, granule, capsule, particle or microparticle comprises or consists essentially 1800 mg, preferably 900 mg, preferably 450 mg L-threonine, L-tryptophan, L-glutamine, L-arginine or L-methionine or any combination thereof.

In one embodiment the Type B pill, tablet, minitab, granule, capsule, particle or microparticle comprises or consists essentially of 67.5 to 2700 mg, preferably 112.5 to 2250 mg, 250 to 2000 mg, 500 to 1900 mg, 900 to 1800 mg, preferably 1800 mg L-threonine. In one embodiment the Type B pill, tablet, minitab, granule, capsule, particle or microparticle comprises or consists essentially 1800 mg, preferably 900 mg, preferably 450 mg L-threonine.

In one embodiment the Type B pill, tablet, minitab, granule, capsule, particle or microparticle comprises or consists essentially of about 45 to 1800 mg sodium butyrate, preferably about 75 to about 1650 mg, about 250 to about 1500, about 500 to about 1400, about 750 to about 1500, about 1000 to about 1400, about 1100 to about 1300 mg, preferably about 1200 mg sodium butyrate. In one embodiment the Type B pill, tablet, minitab, granule, capsule, particle or microparticle comprises about 1200 mg, preferably about 600 mg, preferably about 300 mg sodium butyrate.

In one embodiment the Type B pill, tablet, minitab, granule, capsule, particle or microparticle comprises or consists essentially of 45 to 1800 mg sodium butyrate, preferably 75 to 1650 mg, 250 to 1500, 500 to 1400, 750 to 1500, 1000 to 1400, 1100 to 1300 mg, preferably 1200 mg sodium butyrate. In one embodiment the Type B pill, tablet, minitab, granule, capsule, particle or microparticle comprises or consists essentially of 1200 mg, preferably 600 mg, preferably 300 mg sodium butyrate.

In one embodiment the Type B pill, tablet, minitab, granule, capsule, particle or microparticle comprises or consists essentially about 4 to about 150 ug, about 6.5 to about 140 ug, about 13 to about 130 ug, about 25 to about 120 ug, about 35 to about 110 ug, about 50 to about 105 ug, preferably about 100 ug Vitamin D.

In one embodiment the Type B pill, tablet, minitab, granule, capsule, particle or microparticle comprises or consists essentially of 4 to 150 ug, 6.5 to 140 ug, 13 to 130 ug, 25 to 120 ug, 35 to 110 ug, 50 to 105 ug, preferably 100 ug Vitamin D.

In one embodiment the Type B pill, tablet, minitab, granule, capsule, particle or microparticle comprises or consists essentially of about 1 to about 50 ug Vitamin D, preferably about 10 to about 40 ug, about 20 to about 30 ug, preferably about 25 ug Vitamin D.

In one embodiment the Type B pill, tablet, minitab, granule, capsule, particle or microparticle comprises or consists essentially of 1 to 50 ug Vitamin D, preferably 10 to 40 ug, 20 to 30 ug, preferably 25 ug Vitamin D.

In one embodiment the Type B pill, tablet, minitab, granule, capsule, particle or microparticle comprises or consists essentially of about 5 to about 30 ug Vitamin D, preferably about 10 to about 25 ug, about 12 to about 13 ug, preferably about 12.5 ug Vitamin D.

In one embodiment the Type B pill, tablet, minitab, granule, capsule, particle or microparticle comprises or consists essentially of 5 to 30 ug Vitamin D, preferably 10 to 25 ug, 12 to 13 ug, preferably 12.5 ug Vitamin D.

In one embodiment vitamin D is Cholecalciferol (vitamin D3).

In one embodiment, Type B pills, tablets, minitabs, granules, capsules, particles or microparticles can comprise subtype pills, tablets, minitabs, granules, capsules, particles or microparticles that are Type B1 and B2 subtype pills, tablets, minitabs, granules, capsules, particles or microparticles.

In one embodiment the Type B1 pills, tablets, minitabs, granules, capsules, particles or microparticles comprise the L-threonine and sodium butyrate components comprised in the Type B pills, tablets, minitabs, granules, capsules, particles or microparticles and the Type B2 pills, tablets, minitabs, granules, capsules, particles or microparticles comprise the vitamin D component comprised in the Type B pills, tablets, minitabs, granules, capsules, particles or microparticles.

Additional embodiments of Type B pills, tablets, minitabs, granules, capsules, particles or microparticles comprising subtype B1 and B2 pills, tablets, minitabs, granules, capsules, particles or microparticles are specifically contemplated herein as options of all of the specific embodiments described herein and set out for Type B pills, tablets, minitabs, granules, capsules, particles or microparticles.

In some embodiments, the Type A and/or Type B of pills, tablets, minitabs, capsules, granules, particles or microparticles can be modified for differential release of one or more components, including sustained release and delayed release. A skilled worker can design an oral dosage form according to any of the modified release dosage forms known and described in the art (See for example U.S. Pat. No. 7,108,865).

In some embodiments the pills, tablets, minitabs, capsules, granules, particles or microparticles comprise a water-soluble, water-insoluble, or enteric coating.

Enteric and other pH-sensitive polymers which are relatively insoluble and impermeable at the pH of the stomach, but which are more soluble and permeable at the pH of the small intestine and colon include polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, styrene-maleic acid dibutyl phthalate copolymer, styrene-maleic acid polyvinylacetate phthalate copolymer, styrene and malice acid copolymers, polyacrylic acid derivatives such as acrylic acid and acrylic ester copolymers, polymethacrylic acid and esters thereof, poly acrylic methacrylic acid copolymers, shellac, and vinyl acetate and crotonic acid copolymers. Preferred pH-sensitive polymers include shellac; phthalate derivatives, particularly cellulose acetate phthalate, polyvinylacetate phthalate, and hydroxypropylmethylcellulose phthalate; polyacrylic acid derivatives, particularly polymethyl methacrylate blended with acrylic acid and acrylic ester copolymers; and vinyl acetate and crotonic acid copolymers.

In one embodiment the Type A pills, tablets, minitabs, capsules, granules, particles or microparticles comprise an enteric coating. In one embodiment the enteric coating forms an external layer on the Type A pill, tablet, minitab, granule, capsule, particle or microparticle. In some embodiments the enteric coating is comprised within an internal layer within the Type A the pill, tablet, minitab, granule, capsule, particle or microparticle.

In one embodiment the Type A pills, tablets, minitabs, capsules, granules, particles or microparticles are formulated with an excipient that releases the components comprised therein in the upper part of the small intestine. In one embodiment the excipients comprise one or more diluents, binders, lubricants and disintegrating agents.

In one embodiment the diluent is microcrystalline cellulose or dicalcium phosphate dihydrate or a combination thereof. In one embodiment the disintegrant is crospovidone or croscarmellose sodium or a combination thereof. In one embodiment the polymer is hydroxy propyl methyl cellulose or hydroxy propyl methyl cellulose Phthalate HP 55 or Eudragit L100-55. In one embodiment the lubricant is magnesium stearate or talc or a combination thereof.

In one embodiment the Type B pills, tablets, minitabs, capsules, granules, particles or microparticles are formulated with an excipient that releases the components comprised therein in the lower part of the small intestine. In one embodiment the excipients comprise one or more diluents, binders, lubricants and disintegrating agents.

In one embodiment the diluent is microcrystalline cellulose or dicalcium phosphate dihydrate or a combination thereof. In one embodiment the disintegrant is crospovidone or croscarmellose sodium or a combination thereof. In one embodiment the polymer is hydroxy propyl methyl cellulose or ethyl cellulose or Eudragit S100 or Shellac or Eudraguard biotic. In one embodiment the lubricant is magnesium stearate or talc or a combination thereof.

In one embodiment the Type B pills, tablets, minitabs, capsules, granules, particles or microparticles comprise an enteric coating. In one embodiment the enteric coating forms an external layer on the Type B pill, tablet, minitab, granule, capsule, particle or microparticle. In some embodiments the enteric coating is comprised within an internal layer within the Type B the pill, tablet, minitab, granule, capsule, particle or microparticle.

What is important is that the enteric coating is designed to dissolve from the pill, tablet, minitab, capsule, granule, particle or microparticle at the region of the gastrointestinal (GI) tract of a subject at the optimal location identified by the inventor for releasing the components of the composition comprised therein into the GI tract.

In one embodiment the enteric coating dissolves from the Type A pill, tablet, minitab, granule, capsule, particle or microparticle at about pH 6.0. In one embodiment the pH is < pH6.0.

In one embodiment the enteric coating dissolves from the Type A microparticle at a predetermined pH. In one embodiment the predetermined pH is about pH 6.0. In one embodiment the predetermined pH is > pH 6.0.

In one embodiment the enteric coating is formulated to release the bromelain and menthol from the Type A pill, tablet, minitab, granule, capsule, particle or microparticle into the ileum/small intestine of a subject.

In one embodiment the enteric coating dissolves from the Type A pill, tablet, minitab, granule, capsule, particle or microparticle in the ileum/small intestine of a subject, releasing bromelain and menthol.

In one embodiment the enteric coating dissolves from the Type B the pill, tablet, minitab, granule, capsule, particle or microparticle at a neutral pH. In one embodiment the pH is >pH 6.5. In one embodiment the pH is about 7.0.

In one embodiment the enteric coating dissolves from the Type B microparticle at a predetermined pH. In one embodiment the predetermined pH is a neutral pH. In one embodiment the pH is >pH 6.5. In one embodiment the pH is about 7.0.

In one embodiment the enteric coating is formulated to release the L-threonine, L-tryptophan, L-glutamine, L-arginine or L-methionine or any combination thereof, sodium butyrate and Vitamin D from the pill, tablet, minitab, capsule, granule, particle or microparticle close to and/or in the colon of a subject.

In one embodiment the enteric coating is formulated to release the L-threonine, sodium butyrate and Vitamin D from the pill, tablet, minitab, capsule, granule, particle or microparticle close to and/or in the colon of a subject.

In one embodiment the enteric coating dissolves from the Type B pill, tablet, minitab, capsule, granule, particle or microparticle close to and/or in the colon of a subject releasing the L-threonine, L-tryptophan, L-glutamine, L-arginine or L-methionine or any combination thereof, sodium butyrate and Vitamin D.

In one embodiment the enteric coating dissolves from the Type B pill, tablet, minitab, capsule, granule, particle or microparticle close to and/or in the colon of a subject releasing the L-threonine, sodium butyrate and Vitamin D.

In some embodiments the enteric coating is designed to deliver the components of the composition to a predetermined region of the GI tract. In some embodiments the enteric coating delivers the components of the composition to the predetermined region of the GI tract.

In some embodiments the enteric coating is designed to dissolve from the pill, tablet, minitab, capsule, granule, particle or microparticle at a predetermined pH within the GI tract of a subject to release the components of the composition comprised therein. In some embodiments the enteric coating dissolves at the predetermined pH within the GI tract of the subject.

In some embodiments the Type A and/or Type B pills, tablets, minitabs, capsules, granules, particles or microparticles are formulated to deliver an effective amount of at least one, preferably at least two, preferably at least three, preferably at least four, preferably five components of the composition to the bacteria in the GI tract of the subject.

In some embodiments the different types of pills, tablets, minitabs, capsules, granules, particles or microparticles deliver an effective amount of at least one, preferably at least two, preferably at least three, preferably at least four, preferably five components of the composition to the bacteria in the GI tract of the subject upon dissolution of the enteric coating.

In some embodiments delivery of an effective amount comprises delivery to the ileum/small intestine of the subject. In some the effective amount is 10 to 40 fg/bacteria, 15 to 35 fg/bacteria, 20 to 30 fg/bacteria, preferably 28 fg/bacteria, preferably 25 fg/bacteria of bromelain in the ileum/small intestine of the subject.

In one embodiment the effective amount is 10 to 40 fg/bacteria, 15 to 35 fg/bacteria, 20 to 30 fg/bacteria, preferably 28 fg/bacteria, preferably 25 fg/bacteria, preferably 20 fg/bacteria of bromelain in the ileum/small intestine of the subject.

In some embodiments delivery of the effective amount comprises delivery to the colon of the subject. In some embodiments the effective amount is 25 to 125 pg/ileal enterocyte (pg/ie), 50 to 100 pg/ie, 60 to 90 pg/ie, 70 to 80 pg/ie, preferably 75 pg/ie, of bromelain in the ileum/small intestine of the subject.

In one embodiment the at least first one pill, tablet, minitab, capsule, granule, particle or microparticle comprises an amount of bromelain that is sufficient to provide an effective dose of 25 to 125 pg/ileal enterocyte (pg/ie), 50 to 100 pg/ie, 60 to 90 pg/ie, 70 to 80 pg/ie, preferably 75 pg/ie, in the ileum/small intestine of the subject.

As will be understood by a person skilled in the art, a number of different types of enteric coatings that will dissolve at a predetermined location in the GI tract can be prepared by the skilled person in the pharmaceutical arts by following the guidelines for enteric coatings, for example, according to conventional formulation practice, see, e.g., (Allen) and (Encyclopaedia of Pharmaceutical Technology). It is believed that the preparation of such enteric coatings is within the skill in the art. For example, a composition as described herein is formulated to facilitate targeted delivery of bromalin to the colon. Colon-targeted oral drug discovery systems are well understood in the art (Amidon, 2016).

The present application provides substantial disclosure as to where the components of the composition of the invention are to be released in the GI tract of a subject. Accordingly, it is believed that a person of skill in the art can formulate an appropriate enteric coating to release bromelain and menthol components to the ileum/small intestine of a subject following the direction provided in the disclosure of the present application combined with common general knowledge.

For example, the components of the composition can be dispersed in any pharmaceutically acceptable carrier, which may be an immediate release or a slow release carrier. The carrier may comprise microcrystalline cellulose (MCC), dextran, corn starch, flour, talc, sucrose, mannitol, lactose, calcium carbonate, polyvinylpyrrolidone (PVP), polyethylene oxide, hydroxypropyl methylcellulose (HPMC), hydroxypropylcellulose (HPC), polyvinyl alcohol (PVA) or the like as known in the art.

In one non-limiting example, the carrier may be compressed into a solid or semi-solid and then coated with a polymeric coating to modify the release profile of the components. The coating can comprise either a water-soluble polymer including polyvinylpyrrolidone (PVP), polyvinylpolypyrrolidone (crospovidone), or polyethylene glycol, or a water insoluble polymer including ethers of cellulose, esters of cellulose, cellulose acetate, ethyl cellulose, polyvinyl acetate, neutral copolymers based on ethylacrylate and methylmethacrylate, copolymers of acrylic and methacrylic acid esters with quaternary ammonium groups, pH-insensitive ammonio methacrylic acid copolymers, and mixtures thereof. The coating can also comprise methylcellulose, ethylcellulose, hydroxyethyl cellulose, hydroxypropyl methyl cellulose (HPMC), hydroxypropyl cellulose (HPC), or a combination of such natural polymers. For example, the use of methylcellulose in combination with hydroxypropyl methyl cellulose (HPMC) is well known.

Standard ingredients and methods of preparation of various oral dosage forms is described in "Remington: The science and practice of pharmacy,". Additional excipients include, without limitation, lubricants, disintegrants, and the like.

In one embodiment a composition of the invention comprises a unit dosage form as described herein , preferably an oral dosage form as described herein, comprising a mixture of Type A and Type B pills, tablets, minitabs, capsules, granules, particles or microparticles as described herein in a single container.

In one embodiment a composition of the invention comprises a unit dosage form as described herein, preferably an oral dosage form as described herein, comprising Type A or Type B pills, tablets, minitabs, capsules, granules, particles or microparticles described herein in a single container.

In one embodiment the container is a sachet. In one embodiment the mixture is suspended in a carrier. In one embodiment the carrier is a consumable aqueous liquid selected from the group consisting of water, juice, artificial juices, artificially flavored beverages, animal milks, whey, whey-based beverages, vegetable milks and nut milks. In one embodiment the carrier is a consumable gel. In one embodiment the consumable gel is yogurt.

In one embodiment the oral dosage form comprises Type A and Type B particles or microparticles, preferably microparticles.

In one embodiment the oral dosage form comprises Type A or Type B particles or microparticles, preferably microparticles.

In one embodiment the oral dosage comprises suspended microparticles. In one embodiment the suspended microparticles are suspended in a liquid or gel.

In one embodiment the oral dosage comprises dispersed microparticles. In one embodiment the dispersed microparticles are dispersed in a powder.

In one embodiment the oral dosage comprises a mixture of Type A and Type B microparticles in a single container. In one embodiment the mixture is suspended in a carrier. In one embodiment the carrier is a consumable liquid selected from the group consisting of water, juice, artificial juices, artificially flavored beverages, animal milks, vegetable milks, nut milks, whey, and whey-based beverages. In one embodiment the carrier is a consumable gel. In one embodiment the consumable gel is yogurt.

In one embodiment the subject is a mammal. In one embodiment the subject is a human. The subject may be any subject, including a male, female, adult, geriatric or pediatric subjects. In some embodiments the subject has had a clinical diagnosis of IBD, and in particular, ulcerative colitis or Crohn's disease. Veterinary use in mammals is also contemplated herein. Accordingly, in some embodiments the subject is a non-human mammal. In one embodiment the non-human mammal is a cat or a dog.

In another aspect the invention relates to an oral dosage form consisting essentially of a composition of the invention wherein at least two components of the composition are comprised in a delayed release formulation.

Specifically contemplated herein as embodiments of the oral dosage form of the invention consisting essentially of a composition of the invention wherein at least two components of the composition are comprised in a delayed release formulation, are all of the embodiments contemplated within the aspects of the invention disclosed herein that are a composition comprising (a)-(d) and an oral dosage form comprising about 1 to about 6g of the composition of the invention.

In another aspect, the invention relates to a unit dosage form, preferably an oral dosage form that comprises or consists essentially of the components listed in Table 1 in about the amounts, preferably in the amounts, shown as total dose/80kg person (mg/day), wherein the components are comprised in either Type A or Type B as shown, wherein Type A and Type B are targeted to the small and large intestine respectively or are formulated for release of the components shown in the small and large intestine respectively.

**Table 1 - Components of GaRP**

| **Formulation Component** | | **Human Dose (mg/kg/day)** | **mg/dose** | **Alternative mg/dose** | **Total dose/ 80 kg person (mg/day)** | **Alternative total dose/80 kg person (mg/day)** |
|---|---|---|---|---|---|---|
| Bromelain | Type A* | 18 | 720 1×10⁶ CDU* | 480 1×10⁶ CDU* | 1440 2×10⁶ CDU | 960 1×10⁶ CDU* |
| Peppermint | | 3.6 | 144 | 70 | 288 | 140 |
| Butyric acid (sodium salt) | Type B** | 15 | 600 | 600 | 1200 | 1200 |
| L-threonine | Type B** | 22.5 | 900 | 900 | 1800 | 1800 |
| Cholecalciferol (vitamin D3) | Type B** | 0.0003 | 0.0125 | 0.0125 | 0.025 | 0.025 |

| | | | | | | |
|---|---|---|---|---|---|---|
| *Type A pill, tablet, minitab, granule, capsule, particle or microparticle as contemplated herein. **Type B pill, tablet, minitab, granule, capsule, particle or microparticle as contemplated herein. | | | | | | |

In another aspect the invention relates to a unit dosage form comprising as discrete parts, Type A and Type B pills, tablets, minitabs, granules, capsules, particles or microparticles, wherein
the Type A pills, tablets, minitabs, granules, capsules, particles or microparticles comprise bromelain and are formulated for release of the bromelain to the small intestine of a subject, wherein the Type A pills, tablets, minitabs, granules, capsules, particles or microparticles optionally comprise menthol, and
the Type B pills, tablets, minitabs, granules, capsules, particles or microparticles consist essentially of sodium butyrate and one or more of L-threonine, L-tryptophan, L-glutamine, L-arginine or L-methionine, and Vitamin D and the Type B pills, tablets, minitabs, granules, capsules, particles or microparticles are formulated for release in the lower intestine of a subject.

In another aspect the invention relates to a unit dosage form comprising as discrete parts, Type A and Type B pills, tablets, minitabs, granules, capsules, particles or microparticles, wherein
the Type A pills, tablets, minitabs, granules, capsules, particles or microparticles consist essentially of 720 mg of bromelain and are formulated for release of the bromelain to the small intestine of a subject, wherein the Type A pills, tablets, minitabs, granules, capsules, particles or microparticles optionally comprise 144 mg menthol, and
the Type B pills, tablets, minitabs, granules, capsules, particles or microparticles consist essentially of 600 mg sodium butyrate, 900 mg L-threonine, L-tryptophan, L-glutamine, L-arginine or L-methionine or any combination thereof, and 0.0125 mg Vitamin D and are formulated for release of the sodium butyrate, L-threonine, L-tryptophan, L-glutamine, L-arginine or L-methionine or any combination thereof, and Vitamin D to the lower intestine of a subject.

In another aspect the invention relates to a unit dosage form comprising as discrete parts, Type A and Type B pills, tablets, minitabs, granules, capsules, particles or microparticles, wherein
the Type A pills, tablets, minitabs, granules, capsules, particles or microparticles consist essentially of 720 mg of bromelain and are formulated for release of the bromelain to the small intestine of a subject, wherein the Type A pills, tablets, minitabs, granules, capsules, particles or microparticles optionally comprise 105 mg menthol, and
the Type B pills, tablets, minitabs, granules, capsules, particles or microparticles consist essentially of 600 mg sodium butyrate, 900 mg L-threonine, L-tryptophan, L-glutamine, L-arginine or L-methionine or any combination thereof, and 0.0125 mg Vitamin D and are formulated for release of the sodium butyrate, L-threonine, L-tryptophan, L-glutamine, L-arginine or L-methionine or any combination thereof, and Vitamin D to the lower intestine of a subject.

In another aspect the invention relates to a unit dosage form comprising as discrete parts, Type A and Type B pills, tablets, minitabs, granules, capsules, particles or microparticles, wherein
the Type A pills, tablets, minitabs, granules, capsules, particles or microparticles consist essentially of 480 mg of bromelain and are formulated for release of the bromelain to the small intestine of a subject, wherein the Type A pills, tablets, minitabs, granules, capsules, particles or microparticles optionally comprise 70 mg menthol, and
the Type B pills, tablets, minitabs, granules, capsules, particles or microparticles consist essentially of 600 mg sodium butyrate, 900 mg L-threonine, L-tryptophan, L-glutamine, L-arginine or L-methionine or any combination thereof, and 0.0125 mg Vitamin D and are formulated for release of the sodium butyrate, L-threonine, L-tryptophan, L-glutamine, L-arginine or L-methionine or any combination thereof, and Vitamin D to the lower intestine of a subject.

In one embodiment the Type B pills, tablets, minitabs, granules, capsules, particles or microparticles consist essentially of 600 mg sodium butyrate, 900 mg L-threonine, and 0.0125 mg Vitamin D.

In another aspect the invention relates to a unit dosage form comprising as discrete parts, Type A and Type B pills, tablets, minitabs, granules, capsules, particles or microparticles, wherein
the Type A pills, tablets, minitabs, granules, capsules, particles or microparticles consist essentially of 1440 mg of bromelain and are formulated for release of the bromelain to the small intestine of a subject, wherein the Type A pills, tablets, minitabs, granules, capsules, particles or microparticles optionally comprise 288 mg menthol, and
the Type B pills, tablets, minitabs, granules, capsules, particles or microparticles consist essentially of 1200 mg sodium butyrate, 1800 mg L-threonine, L-tryptophan, L-glutamine, L-arginine or L-methionine or any combination thereof, and 0.025 mg Vitamin D and are formulated for release of the sodium butyrate, L-threonine, L-tryptophan, L-glutamine, L-arginine or L-methionine or any combination thereof, and Vitamin D to the lower intestine of a subject.

In one embodiment the Type B pills, tablets, minitabs, granules, capsules, particles or microparticles consist essentially of 1200 mg sodium butyrate, 1800 mg L-threonine and 0.025 mg Vitamin D.

In one embodiment the Type A and Type B pills, tablets, minitabs, granules, capsules, particles or microparticles are of similar size and characteristics to each other, and may be mixed in any useful ratio depending on the stage of the condition to be treated.

In one embodiment, the Type A and Type B pills, tablets, minitabs, granules, capsules, particles or microparticles are mixed in the ratio of 1.94 part A to 2.28 part B (w/w in mg), providing a ratio of components of Menthol 70 : bromelain 480 : threonine 900 : sodium butyrate 600 : cholecalciferol 0.0125.

In another embodiment, the Type A and Type B pills, tablets, minitabs, granules, capsules, particles or microparticles are mixed in the ratio of 2.91 part A to 2.28 Part B (w/w in mg), providing a ratio of components of Menthol 105: bromelain 720 : threonine 900 : sodium butyrate 600 : cholecalciferol 0.0125.

The inventors have also surprisingly found that a composition comprising sodium butyrate, L-threonine and Vitamin D is surprisingly effective at treating the symptoms of IBD/IBS by promoting the healing of lesions related to gastrointestinal dysfunction. When used together as described herein these components, sodium butyrate, L-threonine and Vitamin D, act synergistically to increase the expression of several mucin genes involved in gut integrity, maintaining gut microbiome diversity and preventing inflammation. This effect can be seen in the results presented in Example 10. These same genes were unaffected when the components were dosed individually. Further demonstration that the combination of sodium butyrate, threonine and vitamin D3 is effective in treating colitis in a mouse model is found in Example 11. Mice in the experiment described exhibited significantly reduced lesion scores by 135% as measured 7 days after induction with TNBS. The results obtained from Example 12 further demonstrate that the combination of sodium butyrate, threonine and vitamin D3 has a similar efficacy to 2 mg/kg prednisolone in treating the lesions induced with TNBS.

Accordingly, in another aspect, the invention relates to a composition comprising sodium butyrate, one or more of L-threonine, L-tryptophan, L-glutamine, L-arginine or L-methionine, and Vitamin D.

In one embodiment the composition consists essentially of sodium butyrate and one or more of L-threonine, L-tryptophan, L-glutamine, L-arginine or L-methionine, and Vitamin D.

In one embodiment the composition is formulated for release of the sodium butyrate, L-threonine, L-tryptophan, L-glutamine, L-arginine or L-methionine or any combination thereof, and Vitamin D to the lower intestine of a subject.

In one embodiment the composition, when administered, administers the sodium butyrate, L-threonine, L-tryptophan, L-glutamine, L-arginine or L-methionine or any combination thereof, and Vitamin D to the lower intestine of a subject.

In one embodiment the composition comprises sodium butyrate, L-threonine and Vitamin D.

In one embodiment the composition consists essentially of sodium butyrate, L-threonine and Vitamin D.

In another aspect the invention relates to a composition comprising 600 mg sodium butyrate, 900 mg L-threonine, L-tryptophan, L-glutamine, L-arginine or L-methionine or any combination thereof, and 0.0125 mg Vitamin D.

In one embodiment the composition comprises 600 mg sodium butyrate, 900 mg L-threonine, and 0.0125 mg Vitamin D.

In one embodiment the composition consists essentially of 600 mg sodium butyrate, 900 mg L-threonine, and 0.0125 mg Vitamin D.

In one embodiment the composition is formulated for release of the sodium butyrate, L-threonine, and Vitamin D to the lower intestine of a subject.

In one embodiment the composition, when administered, administers the sodium butyrate, L-threonine, and Vitamin D to the lower intestine of a subject.

In another aspect the invention relates to a composition comprising 1200 mg sodium butyrate, 1800 mg L-threonine, L-tryptophan, L-glutamine, L-arginine or L-methionine or any combination thereof, and 0.025 mg Vitamin D.

In one embodiment the composition comprises 1200 mg sodium butyrate, 1800 mg L-threonine and 0.025 mg Vitamin D.

In one embodiment the composition consists essentially of 1200 mg sodium butyrate, 1800 mg L-threonine and 0.025 mg Vitamin D.

In one embodiment the composition is formulated for release of the sodium butyrate, L-threonine, and Vitamin D to the lower intestine of a subject.

In one embodiment the composition, when administered, administers the sodium butyrate, L-threonine, Vitamin D to the lower intestine of a subject.

Specifically contemplated herein as embodiments of each of the aspects of the invention related to compositions comprising and/or consisting essentially of sodium butyrate, L-threonine, L-tryptophan, L-glutamine, L-arginine or L-methionine or any combination thereof, and Vitamin D, are all of the embodiments described herein related to unit dosage forms and oral dosage forms including the determination, formulation and administration thereof.

In another aspect the invention relates to a unit dosage form comprising Type B pills, tablets, minitabs, granules, capsules, particles or microparticles, wherein
the Type B pills, tablets, minitabs, granules, capsules, particles or microparticles comprise sodium butyrate and one or more of L-threonine, L-tryptophan, L-glutamine, L-arginine or L-methionine, and Vitamin D.

In one embodiment the Type B pills, tablets, minitabs, granules, capsules, particles or microparticles consist essentially of sodium butyrate and one or more of L-threonine, L-tryptophan, L-glutamine, L-arginine or L-methionine, and Vitamin D.

In one embodiment the Type B pills, tablets, minitabs, granules, capsules, particles or microparticles are formulated for release of the sodium butyrate and one or more of L-threonine, L-tryptophan, L-glutamine, L-arginine or L-methionine, and Vitamin D to the lower intestine of a subject.

In one embodiment the Type B pills, tablets, minitabs, granules, capsules, particles or microparticles are formulated for release of the sodium butyrate and one or more of L-threonine, L-tryptophan, L-glutamine, L-arginine or L-methionine, and Vitamin D to the lower intestine of a subject.

In one embodiment the unit dosage form, when administered, administers the Type B pills, tablets, minitabs, granules, capsules, particles or microparticles to the lower intestine of a subject.

In one embodiment the Type B pills, tablets, minitabs, granules, capsules, particles or microparticles comprise sodium butyrate, L-threonine, and Vitamin D.

In one embodiment the Type B pills, tablets, minitabs, granules, capsules, particles or microparticles consist essentially of sodium butyrate, L-threonine, and Vitamin D.

In one embodiment the unit dosage form is an oral dosage form as described herein.

In another aspect the invention relates to a unit dosage form comprising Type B pills, tablets, minitabs, granules, capsules, particles or microparticles, wherein
the Type B pills, tablets, minitabs, granules, capsules, particles or microparticles consist essentially of 600 mg sodium butyrate, 900 mg L-threonine, L-tryptophan, L-glutamine, L-arginine or L-methionine or any combination thereof, and 0.0125 mg Vitamin D.

In one embodiment the Type B pills, tablets, minitabs, granules, capsules, particles or microparticles comprise 600 mg sodium butyrate, 900 mg L-threonine, and 0.0125 mg Vitamin D.

In one embodiment the Type B pills, tablets, minitabs, granules, capsules, particles or microparticles consist essentially of 600 mg sodium butyrate, 900 mg L-threonine, and 0.0125 mg Vitamin D.

In one embodiment the Type B pills, tablets, minitabs, granules, capsules, particles or microparticles are formulated for release of the sodium butyrate, L-threonine and Vitamin D to the lower intestine of a subject.

In one embodiment the unit dosage form, when administered, administers the Type B pills, tablets, minitabs, granules, capsules, particles or microparticles to the lower intestine of a subject.

In one embodiment the unit dosage form is an oral dosage form as described herein.

In another aspect the invention relates to a unit dosage form comprising Type B pills, tablets, minitabs, granules, capsules, particles or microparticles, wherein
the Type B pills, tablets, minitabs, granules, capsules, particles or microparticles consist essentially of 1200 mg sodium butyrate, 1800 mg L-threonine, L-tryptophan, L-glutamine, L-arginine or L-methionine or any combination thereof, and 0.025 mg Vitamin D.

In one embodiment the Type B pills, tablets, minitabs, granules, capsules, particles or microparticles comprise 1200 mg sodium butyrate, 1800 mg L-threonine and 0.025 mg Vitamin D.

In one embodiment the Type B pills, tablets, minitabs, granules, capsules, particles or microparticles consist essentially of 1200 mg sodium butyrate, 1800 mg L-threonine and 0.025 mg Vitamin D.

In one embodiment the Type B pills, tablets, minitabs, granules, capsules, particles or microparticles are formulated for release of the sodium butyrate, L-threonine and Vitamin D to the lower intestine of a subject.

In one embodiment the unit dosage form, when administered, administers the Type B pills, tablets, minitabs, granules, capsules, particles or microparticles to the lower intestine of a subject.

Specifically contemplated herein as embodiments of the aspects of the invention above related compositions and Type B pills, tablets, minitabs, granules, capsules, particles or microparticles comprising and/or consisting essentially of sodium butyrate, L-threonine, L-tryptophan, L-glutamine, L-arginine or L-methionine or any combination thereof, and Vitamin D, are all of the embodiments set forth herein related to unit dosage forms and oral dosage forms including the dosage, formulation and administration of such forms as described herein.

### Methods

In another aspect the invention relates to a method of reducing bacterial attachment to the cells of the gastrointestinal tract of a subject comprising contacting the bacteria with an effective amount of the composition, unit dosage form or oral dosage form of the invention.

In another aspect the invention relates to a method of reducing bacterial invasion into the cells of the gastrointestinal tract of a subject comprising contacting the bacteria with an effective amount of the composition, unit dosage form or oral dosage form of the invention.

In another aspect the invention relates to a method of improving gastrointestinal barrier integrity in a subject comprising administering to a subject in need thereof, a therapeutically effective amount of a composition, unit dosage form or oral dosage form of the invention.

In one aspect the invention relates a method of preventing, treating or managing IBD or IBS comprising administering to a subject in need thereof, a therapeutically effective amount of a composition, unit dosage form or oral dosage form of the invention.

In another aspect the invention relates to a method of at least partially alleviating at least one symptom of IBD or IBS comprising administering to a subject in need thereof, a therapeutically effective amount of a composition, unit dosage form, or oral dosage form of the invention.

In one embodiment, the least one symptom is selected from the group comprising diarrhoea, abdominal pain, abdominal cramping, fever, fatigue, nausea, flatulence, bloating, weight loss, reduced appetite and bloody stools.

In another aspect the invention relates to method of improving at least one parameter associated with IBD or IBS comprising administering to a subject in need thereof, a therapeutically effective amount of a composition, unit dosage form or oral dosage form of the invention.

In one embodiment, at least one parameter associated with IBD or IBS is selected from the group comprising:
- Circulating serum levels of inflammatory markers such as IL-6, IL-8, IL-17A, IL-17F, IL-18, IL-21, IL-22, TL1A, TGF-β, ICAM-1, IFN-gamma, MADCAM-1 and E-selectin
- Fecal markers of inflammation such as calprotectin, lactoferrin and lysozyme
- Circulating levels of serum oxidative stress markers such as oxidised LDL, serum oxidisability and F2-isoprostanes
- Biopathogens in stool samples such as bacteria belonging to Proteobacteria Enterobacteriaceae *Escherichia coli,* to Proteobacteria Enterobacteriaceae *Klebsiella* spp, Proteobacteria Pseudomonadaceae *Pseudomonas* spp., Proteobacteria enterobacteriaceae *Salmonella* spp., Proteobacteria Desulfovibrionaceae *Bilophilia wadsworthia,* Proteobacteria *Desulfovibrionaceae desulfovibrio* spp., Proteobacteria *Desulfovibrionaceae Desulfuromonas* spp., Proteobacteria Campylobacteraceae *Campylobacter concisus,* Proteobacteria Campylobacteraceae *Campylobacter jejuni,* Bacteroidetes Bacteroidaceae Enterotoxigenic *Bacteroides fragilis,* Actinobacteria Coriobacteriaceae *Atopobium parvulum* and Firmicutes Clostridiaceae *Clostridium difficile.*

In the above aspects:
In one embodiment the subject is suffering from ulcerative colitis. In one embodiment the subject is suffering from Crohn's disease. In one embodiment the subject is suffering from IBS. In one embodiment administration is oral administration.

In one embodiment administration is about 20 - 90 minutes, preferably about 30 - 60, preferably about 45 minutes before a meal. In one embodiment administration is 20-90 minutes, preferably 30-60, preferably 45 minutes before a meal.

In one embodiment, administration is once to five times daily before meals, preferably three times daily before meals, preferably two times daily before meals. In one embodiment administration is before breakfast and lunch. In one embodiment administration is before breakfast and dinner.

In one embodiment administration is about 20 - 90 minutes, preferably about 30 - 60, preferably about 45 minutes after a meal. In one embodiment administration is 20-90 minutes, preferably 30-60, preferably 45 minutes after a meal.

In one embodiment, administration is once to five times daily after meals, preferably three times daily after meals, preferably two times daily after meals. In one embodiment administration is after breakfast and lunch. In one embodiment administration is after breakfast and dinner.

A subject in need of prevention, treatment or management of IBD or IBS is a subject diagnosed with such a condition, predisposed and/or at risk of such a condition, or who has recovered from or is in remission from such a condition. A subject may be predisposed and/or at risk of the condition because of genetic and/or environmental factors.

The amount of the composition, unit dosage form or oral dosage form as described herein that will be effective in the prevention, treatment or management of a said condition will vary with the nature and severity of the condition and the route by which the extract is to be administered, as well as factors specific to the subject such as their age, weight, sex and past medical history.

Generally, the composition or dosage form used in the treatment of an acute condition will comprise a larger amount of the bromelain composition, unit dosage or oral dosage of the invention than would be used in the treatment of the chronic condition. Formulation of the specific dosage form to address either an acute or chronic condition is believed to be achievable by a person skilled in the art of pharmacy combined with the present disclosure of the invention.

In some embodiments, the subject is a non-human mammal.

In one embodiment administration to the non-human mammal comprises administration of a composition or unit dosage form described herein comprising or consisting essentially of an oral dosage form as described herein.

In one embodiment the oral dosage form administered to the non-human mammal comprises about 1 to about 50 mg bromelain, preferably about 5 to about 25 mg, about 7 to about 20 mg, about 9 to about 18 mg, about 10 to about 15 mg, about 13 mg bromelain per kg subject.

In one embodiment the oral dosage form administered to the non-human mammal comprises about 9 mg/kg, about 13 mg or about 18 mg bromelain per kg subject.

In one embodiment the oral dosage form administered to the non-human mammal comprises 1 to 50 mg bromelain, preferably 5 to 25 mg, 7 to 20 mg, 9 to 18 mg, 10 to 15 mg, or 13 mg bromelain per kg subject.

In one embodiment the oral dosage form administered to the non-human mammal comprises 9 mg/kg, 13 mg or 18 mg bromelain per kg subject.

In one embodiment the oral dosage form is an initial dosage determined based on an art accepted conversion from a human dosage contemplated herein using the Guidance for Industry "Estimating the Maximum Safe Starting Dose in Initial Clinical Trials for Therapeutics in Adult Healthy Volunteers" published by the U.S. Department of Health and Human Services Food and Drug Administration Center for Drug Evaluation and Research (CDER), July 2005.

In one embodiment the human dose in mg/kg is multiplied to 1.8 to provide a dog dosage in mg/kg.

In one embodiment the non-human mammal is a domestic pet. In one embodiment the domestic pet is selected from the group consisting of dogs, cats, rats, mice, hamsters, guinea pigs, ferrets, and gerbils. In one embodiment the domestic pet is a dog. In one embodiment the domestic pet is a cat. In this specification where reference has been made to patent specifications, other external documents, or other sources of information, this is generally for the purpose of providing a context for discussing the features of the invention. Unless specifically stated otherwise, reference to such external documents is not to be construed as an admission that such documents; or such sources of information, in any jurisdiction, are prior art, or form part of the common general knowledge in the art.

The invention will now be illustrated in a non-limiting way by reference to the following examples.

### 1. EXAMPLES

### General Methodology

**Macrophage cell culture:** The human monocytic THP-1 cells were cultured in RPMI 1640 medium modified to contain 2 mM L-glutamine, 10 mM HEPES, 1 mM sodium pyruvate, 4500 mg/L glucose, and 1500 mg/L sodium bicarbonate and supplemented with 10% FBS, 100 U/ml penicillin, 100 mg/ml streptomycin 5mM β-mercaptoethanol. Cell density never exceeded 1×10⁶/ml. To induce differentiation, THP-1 cells were plated at 5-10×10⁵ cells/cm² into 24 well plates and treated with 200nm PMA for 48h. After 48h the macrophages were washed and grown in PMA and antibiotic-free media for 24h before commencing the experiment. Cell cultures were incubated in a humidified 5% CO₂ incubator at 37°C. All experiments were conducted on cells within passages number 10-30.

**Caco-2 cell culture:** The human colorectal adenocarcinoma cell-line Caco-2-BBe1 was grown and maintained in Dulbecco's modified Eagle's medium (DMEM) supplemented with 10% v/v fetal bovine serum (FBS), 4 mM L-glutamine, 100 U/ml penicillin and 100 mg/ml streptomycin. All experiments were conducted on Caco-2-BBe1 cells within 12 passages after thawing. Cell cultures were incubated in a humidified 5% CO₂ incubator at 37°C.

**Caco-2:HT-29-MTX 75:25 coculture:** This co-culture model was used to simulate the human colonic epithelium (Hilgendorh C, 2000 ). The Caco-2 cells (passage 8) and the HT29-MTX cells (passage 9) were combined at a 75:25 ratio in DMEM with 10% FBS. The combined cells (6.4 × 10⁴ cells/ml) were seeded into 25 cm² in a total of 5 ml per flask. The coculture was incubated at 37°C in a 5% CO₂ atmosphere with medium refreshed every 48h. At 15 days post seeding, the coculture monolayer was washed three times in DMEM and replaced with 5 ml of pre-warmed test solution, and then incubated for 24 h.

**Bacterial Strains and Growth Conditions:** The AIEC strains of HM 95, HM605, HM 615 and HM484 were cultured in LB broth. All bacteria were grown overnight at 37°C in air, followed by a 1:100 dilution in fresh media before incubating for a further 2h. Bacteria were used in experiments in the mid-log phase confirmed by measuring 0.8 at Ab₆₀₀ₙₘ

**Transepithelial electrical resistance (TER) measurement:** The integrity of the Caco-2 monolayer was determined by measuring the TER value. Tight junctions serve as barriers to paracellular diffusion where TER is indicative of the level of integrity between epithelial cells (Mynott TL, 1999). Before measuring the TER, cell cultures were equilibrated to room temperature for 30 min. TER was measured using a millicell-ERS instrument (Millipore, Eschborn, Germany).

**Cytokine and TNF-α quantitation:** Levels of IL-8, IL-6 and TNF-α were determined using a commercial ELISA kits. At the end of the incubation, apical and basolateral media was collected, centrifuged at 400gav for 5 min and the supernatant frozen at -80°C until analysis. No more than one repeat/thaw cycle was conducted with the frozen supernatant. All ELISAs were performed as per manufactures instructions.

**Inflammatory model of a Caco-2/THP-1 macrophage co-culture system:** Caco-2-BBe1 cells were seeded at a 5×10⁵ cells/ well onto 24-well Transwell insert plates (4.67 cm², 0.4 µm pore size). The cell culture medium was changed every 3 day until the cells were fully differentiated (TER value >1200/cm²). In a separate 24 well plate, THP-1 monocytes cells were seeded at 1×10⁶ cells/well and differentiated with 200nM PMA for 48h followed by culture in normal media for 24h prior to the experiment. After replacing all media with RPMI1640, all Transwell inserts containing the Caco-2 differentiated monolayers were loaded into the wells containing differentiated THP-1 cells. In experiments to evaluate the anti-inflammatory effect of different bromelain formulations, 0.25 ml of test samples was applied to the apical side for 24 h, at the same time 50ng/ml of LPS was added to the basolateral side in this model. After the 24h incubation culture supernatants from the apical side were collected for IL-8 measurement.

**Inflammatory model of Caco-2 polarised monolayer stimulated with inflammatory mediators LPS, TNF-α, IFN-β:** Polarised Caco-2 models were generated as previously described. When cell cultures reached a baseline TER of >1000/cm², the monolayers were stimulated by addition of 0.6 ml containing 50ng/ml IFN-β, 10µg/ml LPS and 5ng/ml TNFα to the basolateral compartment. Immediately after the addition of the stimulation cocktail 0.25ml of GaRP ± 0-80µg/ml bromelain was applied to the apical compartment. After the 24h incubation culture supernatants from the apical side were collected for quantitation of IL-8.

**Gentamicin protection assay-measure of bacterial uptake, survival and replication:** THP1 cells were infected with a multiplicity of infection (MOI) of 30 bacteria per macrophage. After a 3h incubation, the media was removed and fresh cell culture medium containing 100 µg/ml of gentamicin was added to kill extracellular bacteria. To determine the number of intracellular bacteria, at 1 and 3h, the medium was removed and 0.5 ml of 1% Triton X-100 in deionised water was added to each well for 5 min to lyse the THP-1 cells. This concentration of Triton X-100 had no effect on bacterial viability for at least 30 min. Samples were mixed, diluted and plated onto LB agar plates to determine the number of colony-forming units (cfu) recovered from the lysed monolayers. The number of intracellular bacteria at 1h were compared to the RPMI no treatment control and these were considered as the adhered bacteria. Bacterial replication was expressed as the mean percentage of bacteria recovered at 4h post-infection relative to the number of bacteria recovered after 1h of gentamicin treatment, defined as 100%.

### Quantitative polymerase chain reaction (qPCR) analysis of gene expression

Before RNA extraction, cells were collected by centrifugation at 5,000 g for 5 min, 4°C. the cell pellet was washed three times in ice-cold PBS followed by resuspension in 500 µl of ice-cold PBS. The total RNA of 2 × 10⁶ cells per sample was extracted using a PureLink RNA mini kit with 1.5µg of RNA (with A260/A280 ratio between 2.00 and 2.20) reverse-transcribed into cDNA using a High Capacity cDNA RT kit. The expression of the genes of interest was quantified using the PowerUp SYBR green Master mix. In brief, triplicate qPCR reactions was set up using 20 ng cDNA and 500 nM primers in a MicroAmp optical 384-well reaction plates using a Biomek-4000 automated liquid handler (Beckman Coulter, Brea, CA, USA). Amplification was run in a ViiA7 Real-Time PCR system (Thermo Fisher Scientific) at the following conditions: 95°C for 20 seconds followed by 40 cycles of 95°C for 1 second and 60°C for 20 seconds; and a melt curve stage: 95°C, 15 s; 60°C, 60 s. The results of qPCR were analysed with the 2-ΔΔCT method as previously described using the QuantStudio Software V1.3 (Applied Biosystems). Relative gene expression values were normalized by using GAPDH and 18S as the multiple endogenous reference genes.

**Statistical analyses.** Results were generated from 3 independent experiments (n = 3) with 3 technical replicates unless stated otherwise. The data analysis was performed with Microsoft Excel. The results were illustrated using SigmaPlotv11 and variations between results were expressed as standard deviation (SD). The data were statistically analysed by one-way ANOVA and post-hoc Dunnett's test, unless stated otherwise, using Sigmaplot v11. A value of p ≤ 0.05 was accepted as statistically significant. No graphical difference was made for p-values ≤0.05-0.001

### Example 1: Components and dosage modelling

The components of compositions of the invention as described herein are set out below in Table 2 including their sources. In some embodiments, the compositions were prepared by mixing bromelain with the remainder of ingredients in the quantities shown in Table 2a or 2b.

**Table 2a - Components**

| **Formulation Ingredient** | **Supplier** | **Catalogue Number** | **Batch/Lot Number** | **Dose (mg/kg)** |
|---|---|---|---|---|
| **Oral Formulation** | | | | |
| Bromelain | Hong Mao | 3000 GDU | 18246 | 50-220 |
| Menthol natural | Sigma Aldrich | W266523-100G-K | MKCB3457 | 15 |
| Propylene glycol USP | Sigma Aldrich | P4347 | MKCG8590 | 462 |
| Hydroxypropylmethyl cellulose | Sigma | 09963 | BCBJ5753V | 122 |
| Water for injection | Troy Laboratories | ILIUM | 151208 | |
| Sodium Hydrogen Carbonate | Amresco | 0865 | 1258B162 | 2 |
| Prednisolone | Sigma | P6004 | SLCB2470 | 2.5 |
| Tween-80 non-animal origin | Sigma Aldrich | P6224 | MKCC8526 | 142 |

| **Rectal Formulation** | | | | |
|---|---|---|---|---|
| Sodium butyrate USP-NF Pharma grade | Sigma Aldrich | ARK2161 | SBUT132-19 | 183 |
| L-threonine USP, EP, JP | Sigma Aldrich | T8441 | SLBR5358V | 275 |
| Vitamin D3 USP | Sigma-Aldrich | C1357 | SLBV6276 | 0.016 |
| Hydroxypropylmethyl cellulose | Sigma | 09963 | BCBJ5753V | 350 |
| Propylene glycol USP | Sigma Aldrich | P4347 | MKCG8590 | 4 |
| Water for injection | Troy Laboratories | ILIUM | 151208 | |

**Table 2b: Compositions**

| **Component** | **Formulation 1 µg/ml** | **Formulation 2 µg/ml** |
|---|---|---|
| Bromelain | Dose titrated 5-640 | Dose titrated 5-640 |
| Sodium butyrate | Dose titrated 200-800 | 400 |
| I-threonine | Dose titrated 200-800 | 400 |
| Cholecalciferol | Dose titrated 0.24 to 0.72 | 0.480 |
| L-tryptophan | Dose titrated 200-800 | |
| I-glutamine | Dose titrated 200-800 | |
| L-arginine | Dose titrated 200-400 | |
| L-methionine | Dose titrated 200-800 | |

### Modelling of the potential doses of the components of the invention reaching the ileum and colon after administration of GArP.

### Dose justification modelling

To determine the dose range of bromelain to be tested in the proof of concept *in vitro* studies, the following gastrointestinal distribution model was developed

### Assumptions:

- Less than 1% oral bioavailability
- Bromelain tablet has a rapid and uniform dissolution in the ileal fluid
- Bromelain maintains its full biological activity as it enters the colon
- Mixing of the bromelain in the colon is uniform and does not occur as a gradient mix

**Table 2c: Gastrointestinal distribution model**

| **Tissue** | **Length (cm)** | **Total Enterocytes** | **Typical [bacteria] (#/ml)** | **Fluid volume (ml)** | **Total bacteria** | **Bacteria/ enterocyte** |
|---|---|---|---|---|---|---|
| Ileum | 300 | 1×10¹⁰ | 10⁸ | 94 ± 24 | 9.4 ×10⁹ | 1 |
| Colon | 150 | 3.3×10⁸ | 10¹¹ | 400⁷ | 4 ×10¹⁴ | 1.2 ×10⁵ |

**Enterocytes in the ileum:** To calculate the surface of the ileum we considered it a cylinder with a length of 3m and a diameter of 2cm (Sender, 2016). Then, considering the valvulae conniventes, the total surface increases 3-fold (Mudie DM, 2014). Cattaneo and Baratta (Borley, 2005), estimated 1000 ± 3.4 cells per villus. As there are 13 ± 1 villi/mm², the total number of cells covering them is estimated at 1.35 ×10¹⁰. The total number of cells covering the cryptae is 3.24 ± 0.14×10⁹ if we consider that they represent 12/50 of the cryptae covering each villus (Sender, 2016) (Teodori, 1987) and that the estimated number of cryptae and villi is roughly the same (Sender, 2016).Therefore, the total enterocyte cell number was calculated to be 1×10¹⁰ per 3m ileum.

**Enterocytes in the colon:** To calculate the surface of the colon we considered it a cylinder with a length of 1.5m and a diameter of 7.5cm (Cattaneo L, 1989) and it is 1/30 of the small intestine (Weiss L, 1981) because it lacks villi. Therefore, the total enterocyte cell number per 1.5m colon is 3.3×10⁸.

**Concentration of bromelain in the ileum:** If given a 60kg person orally administered 1.5g of ileum/colon-targeted bromelain with a glass of water, then the bromelain would be distributed in 94 ± 24ml (Weiss L, 1981) or 16mg/ml. At this concentration, assuming a full dissolution of the bromelain tablet in the ileum, the maximum exposure per bacteria would be 170 fg/bacteria.

**Concentration of bromelain in the colon:** If given a 60kg person orally administered 1.5g of ileum/colon-targeted bromelain, then the bromelain would be distributed in 400ml of colonic fluid or 40µg/ml. At this concentration, assuming a full mixing of the bromelain into the colonic fluid, the maximum exposure per bacteria would be 38 fg/bacteria.

**Concentration of bromelain per ileum enterocyte:** If given a 60kg person orally administered 1.5g of ileum/colon-targeted bromelain with a glass of water, then the bromelain would be distributed in 94 ± 24ml (Weiss L, 1981) or 16mg/ml. At this concentration, assuming a full dissolution of the bromelain tablet in the ileum, the maximum exposure per enterocyte would be 150 pg/enterocyte.

**Concentration of bromelain per colonic enterocyte:** If given a 60kg person orally administered 1.5g of ileum/colon-targeted bromelain, then the bromelain would be distributed in 400ml of colonic fluid or 40µg/ml. At this concentration, assuming a full mixing of the bromelain into the colonic fluid, the maximum exposure per bacteria would be 4.5ng/ enterocyte.

Using the model outlined in above, if a 80kg person was to take a total daily dose of enterically coated 1440mg of bromelain and 288mg menthol targeted to the small intestine and a colon-targeted dose of 1200mg butyric acid (sodium salt), 1800mg of I-threonine and 0.025mg of vitamin D3 then then the following concentrations would be achieved per enterocyte and bacteria contained within the gut microbiome (Table 3).

**Table 3: Summary of the achievable ileal and colonic concentrations of each GaRP formulation component**

| **Formulation Component** | **Ileum** | | **Colon** | |
|---|---|---|---|---|
| | **Bacteria** | **Enterocyte** | **Bacteria** | **Enterocyte** |
| **Bromelain** | 170 fg | 150 pg | 38 fg | 4.5ng |
| **Menthol** | 33 fg | 30 pg | 7.5 fg | 0.9 ng |
| **Butyric acid (sodium salt)** | 142 fg | 125 pg | 32 fg | 3.8 ng |
| **L-threonine** | 213 fg | 188 pg | 48 fg | 5.6 ng |
| **Vitamin D3** | 0.01 fg | 0.01 pg | 0.003 fg | 0.32 pg |

The observed therapeutic effective dose was determined in the *in vitro* experiments described herein.

**Table 4** summarises the observed therapeutic effective dose for each GaRP formulation component.

**Table 4: Summary of the observed therapeutic effective dose of each GaRP formulation component**

| **Formulation Component** | **Target cell** | | |
|---|---|---|---|
| | **Bacteria (fg/cell)** | **Enterocyte (pg/cell)** | **Macrophage (pg/cell)** |
| **Bromelain** | ≥ 28 | ≥ 3 | ≥ 60 |
| **Menthol*** | Not relevant | 20 | 20 |
| **Butyric acid (sodium salt)** | Not relevant | 15 | 300 |
| **L-threonine** | Not relevant | 15 | 300 |
| **Vitamin D3** | Not relevant | 0.018 | 0.36 |

| | | | |
|---|---|---|---|
| **Note: Not relevant** means that the specific formulation component did not exert any anti-attachment or anti-invasive effect on the AIEC obtained from IBD and IBS patients. Without wishing to be bound by theory the inventor acknowledges that * was not tested *in vitro* but published literature suggests that 190mg-240/day (35% of an enterically-coated 540mg/day peppermint oil) would achieve the above ileal and colonic concentrations. | | | |

In conclusion, the inventor believes that the except for the modelled menthol dose that would need to be achieved in the ileum, the GaRP formulation described herein will successfully achieve therapeutic doses in both the ileum and colon.

### Example 2: Evaluating the effect of bromelain pre-treatment on the invasion and intracellular proliferation of Adhesive Invasive E. coli (AIEC) in THP-1 macrophages

In these studies, the bacteria were pre-treated with the bromelain.

### Macrophage Culture

The human monocytic THP-1 cells (ATCC TIB-202) were cultured in RPMI 1640 medium modified to contain 2 mM L-glutamine, 10 mM HEPES, 1 mM sodium pyruvate, 4500 mg/L glucose, and 1500 mg/L sodium bicarbonate and supplemented with 10% FBS, 100 U/ml penicillin, 100 mg/ml streptomycin 5mM β-mercaptoethanol. Cell density never exceeded 1×10⁶/ml. To induce differentiation, THP1 cells were plated at 1×10⁵ cells/cm² into 24 well plates and treated with 200nm PMA for 48h. After 48h the macrophages were washed and grown in PMA and antibiotic-free media for 24h before commencing the experiment.

### Bacterial Strains and Growth Conditions

### The HM 605 AIEC (from CD patient), HM 615 AIEC (from CD patient) and HM484

E.coli (isolated from IBS patient) were grown overnight on nutrient agar and were suspended in sterile saline and left at room temperature for 24 hours as this has been found to maximise fimbrial expression (HM Martin, 2004). On the day of the experiment the bacteria were centrifuged at 300gav for 15 min at room temperature and resuspended to 1.4×10⁷ bacteria/ml in the treatment or control media. The treatments are summarised in Table 5.

**Table 5 - Pre-treatment of bacteria with bromelain**

| **Treatment** | **Concentration Range of bromelain/bacteria** |
|---|---|
| RPMI media only | 0 |
| GaRPv1 minus bromelain | 0 |
| GaRPv1 with 20-640 µg/ml bromelain | 1- 60 fg |
| RPMI with 20-640 µg/ml bromelain | 1- 60 fg |

The bacteria were incubated at 37°C for 2h with constant shaking. At the end of the incubation the bacteria were washed 3X with RPMI and then resuspended in THP-1 media to a final concentration of 1.4×10⁷ bacteria/ml.

### Bacterial uptake, survival and replication assay were measured by the gentamicin protection assay

THP1 cells were infected with a multiplicity of infection (MOI) of 30 bacteria per macrophage. After a 3-h incubation, the media was removed and fresh cell culture medium containing 100 µg/ml of gentamicin was added to kill extracellular bacteria. To determine the number of intracellular bacteria, at 1 and 3h, the medium was removed and 0.5 ml of 1% Triton X-100 in deionized water was added to each well for 5 min to lyse the THP-1 cells. This concentration of Triton X-100 had no effect on bacterial viability for at least 30 min. Samples were mixed, diluted and plated onto LB agar plates to determine the number of colony-forming units (cfu) recovered from the lysed monolayers. The number of intracellular bacteria at 1h were compared to the RPMI no treatment control and these were considered as the adhered bacteria. Bacterial replication was expressed as the mean percentage of bacteria recovered at 4 h post-infection relative to the number of bacteria recovered after 1 h of gentamicin treatment, defined as 100%.

### Results! Discussion:

In the case of the HM605 and HM484 bacteria, bromelain concentrations of ≥20fg/bacteria were able to inhibit bacterial invasion of the THP-1 macrophages (Table 6). The pre-treatment of the bacteria with bromelain were less effective at inhibiting intracellular proliferation.

**Table 6: Pre-treatment of AIEC with bromelain in GaRP inhibits their invasion of THP-1 macrophages**

| Bacterial Line | Disease Type | Inhibition of Invasion (Mean ± SD)* | Effective Concentration Range (fq/bacteria) | Inhibition of proliferation (Mean ± SD)* |
|---|---|---|---|---|
| **Bromelain** | | | | |
| HM 605 | IBD-CD | 56 ± 6 | >14 | 15 ± 3 |
| HM 615 | IBD-CD | 0 ± 1 | >60 | 0 ± 0 |
| HM 484 | IBS | 62 ± 2 | >19 | 8 ± 2 |
| N=2-3 independent experiments | | | | |
| *Calculated by comparing the treated bacteria to the untreated RPMI control which is defined as 100% of invasion. | | | | |

Inserting the data from Table 5 into the gastrointestinal distribution model set forth herein based on enterocytes/cm² and bacteria/enterocyte, the inventors believe that a person would require 867 mg/80 kg of ileum-targeted(small intestine targeted?) bromelain to achieve the effective dose of 20 fg/bacteria. In one embodiment, a composition as described herein provides a total daily dose of 1440 mg of ileum-targeted bromelain.

### Example 3: Bromelain pre-treatment of AIEC and the effect on the invasion and intracellular proliferation of AIEC in Caco-2 enterocytes

### Cell culture

Caco-2-BBe1 enterocytes were plated at 2×10⁵ cells/cm² into 24 well plates and grown until confluent. Cells were grown in antibiotic-free media for 24h prior to commencing the experiment. On the day of the experiment the HM605 and HM484 *E. coli* were centrifuged at 300gav for 15 min at room temperature and resuspended to 1.4×10⁷ bacteria/ml in Caco-2 media ± 20-640µg/ml bromelain. The bacteria were incubated at 37°C for 2h with constant shaking. At the end of the incubation period the bacteria were washed 3 times with DMEM and then resuspended in Caco-2 media to a final concentration of 1.4×10⁷ bacteria/ml. The gentamicin protection assay was then performed as previously described. To simulate the intestinal environment, a series of experiments were conducted where the bacteria were resuspended in fasted-state simulated intestinal fluid (Rieder F, 2012) containing 20-640µg/ml bromelain.

### Results! Discussion:

In the case of the HM605 and HM484 bacteria, bromelain concentrations of ≥20fg/bacteria were able to inhibit bacterial invasion of the enterocytes and intracellular proliferation (Table 7).

The pre-treatment of the bacteria in simulated intestinal fluid increased (20 fg increased to 28 fg) the concentration of bromelain required to achieve the same level of inhibition of bacterial invasion and intracellular proliferation. Accordingly, when using the gastrointestinal exposure model described herein an appropriate patient dosage will be 800mg/60kg/day in order to achieve 20 fg of bromelain per bacteria.

**Table 7: Pre-treatment of AIEC with bromelain in GaRPv1 inhibits the invasion of Caco-2 enterocytes**

| Bacterial Line | Disease Type | Inhibition of Invasion (Mean ± SD)* | | Effective Concentration Range (fq/bacteria) | | Inhibition of proliferation (Mean ± SD)* | |
|---|---|---|---|---|---|---|---|
| **Bromelain** | | | | | | | |
| | | Simulated intestinal fluid | | Simulated intestinal fluid | | Simulated intestinal fluid | |
| | | No (n=4) | Yes (n=2) | No (n=4) | Yes (n=2) | No (n=4) | Yes (n=2) |
| HM 605 | IBD-CD | 95 ± 2 | 93 ± 4 | >20 | >14 | 99 ± 1 | 92 ± 4 |
| HM 484 | IBS | 93 ± 2 | 91 ± 6 | >19 | >28 | 100 ± 1 | 91 ± 3 |
| N=2-3 independent experiments | | | | | | | |
| *Calculated by comparing the treated bacteria to the untreated RPMI control which is defined as 100% of invasion. | | | | | | | |

Inserting the data from Table 7 into the gastrointestinal distribution model based on enterocytes/cm² and bacteria/enterocyte as described herein, the inventor believes that an appropriate patient dosage will be 1067 mg/80 kg/day of small intestine targeted bromelain to achieve the effective dose of 28 fg/bacteria.

### Example 4 - GaRP containing bromelain is effective in inhibiting the release of TNFa from LPS-stimulated THP-1 macrophages

### Macrophage Culture

The human monocytic THP-1 cells (ATCC TIB-202) were cultured in RPMI 1640 medium modified to contain 2 mM L-glutamine, 10 mM HEPES, 1 mM sodium pyruvate, 4500 mg/L glucose, and 1500 mg/L sodium bicarbonate and supplemented with 10% FBS, 100 U/ml penicillin, 100 mg/ml streptomycin 5mM β-mercaptoethanol. Cell density never exceeded 1×10⁶/ml. To induce differentiation, THP1 cells were plated at 5×10⁵ cells/cm² into 96 well plates and treated with 200nm PMA for 48h. After 48h the macrophages were washed and grown in PMA antibiotic-free media for 24h before commencing the experiment. Transwells are shown in Figure 1. Treatments are shown in Table 8.

**Table 8 - Bromelain treatments evaluating the effect of GaRpv1 containing bromelain on the release of pro-inflammatory cytokines from THP-1 macrophages**

| **Treatment** | **Concentration Range of bromelain/macrophage** |
|---|---|
| RPMI media only | 0 |
| GaRPv1 minus bromelain | 0 |
| GaRPv1 with 0-80 µg/ml bromelain | 7- 107 pg |
| RPMI with 0-80 µg/ml bromelain | 7- 107 pg |

The test compounds and controls were applied to the cells where macrophages were stimulated with 50ng/ml of LPS using a non-stimulated culture as the control. After 8h the media was removed and the TNFα was quantitated using an ELISA.

### Results/ Discussion:

As seen in Figure 2, bromelain was able to inhibit the release of TNFa from THP-1 macrophages which had been stimulated with LPS. When compared to the RPMI control, at bromelain concentrations higher that 13pg/macrophage there was a significant inhibition in the release of TNFa (p<0.001, Mann Whitney rank sum test).

### Conclusion:

Without wishing to be bound by theory, the inventor believes that their modelling using the gastrointestinal distribution model, shows that, if a person was to take 800mg of enterically coated bromelain then the exposure would be 75pg/cell per ileal enterocyte and 4500pg/cell in the colon.

### Example 5 - GaRP containing bromelain inhibits the release of IL-8 from polarised Caco-2 monolayers co-incubated with LPS-stimulated THP-1 macrophages

Using the Industry-standard model most commonly used to evaluate food products and pharmaceuticals, the effects of bromelain on the release of IL-8 was investigated. Apical compartment represents the lumen (fluids & mucosa of GIT)

### Co-Culture Cell culture

Caco-2 cells were seeded at 1 ×10⁶ cells/well onto Transwell insert plates (1.12 cm², 0.4 µm pore size), The cell culture medium was changed every 3-day until the cells were fully differentiated (TER value >1200 X cm²), and the cells were used in passages number within 12 passages after thawing. In a separate 12 well plate, THP-1 monocytes cells were seeded at 8.5 × 10⁵ cells/well and differentiated with 200nM PMA for 48h followed by culture in normal media for 24h prior to the experiment. After replacing all media with RPMI1640, the Transwell insert on which Caco-2 cells were loaded into the wells containing THP-1 cells. When cell cultures reached a baseline TER of >1000cm², the monolayers were stimulated by addition of 0.6 ml containing 50ng/ml IFN-β, 10µg/ml LPS and 5ng/ml TNFa to the basolateral compartment. Immediately after the addition of the stimulation cocktail 0.25ml of GaRP ± 0-80µg/ml bromelain (0-20pg/enterocyte) was applied to the apical compartment. After the 24h incubation culture supernatants from the apical side and basolateral sides were collected for IL-8 measurement.

### Results/ Discussion:

As seen in Figure 3, bromelain is able to inhibit the release of IL-8 from Caco-2 cells co-cultured with LPs stimulated THP-1 macrophages. The GaRPv1 without bromelain was able to inhibit the release of IL-8 by 18 ± 6.

### Example 6 - Evaluating the effect of GaRPv1 containing bromelain on the restoration of gut integrity as measured by trans-epithelial electrical resistance (TER).

Preclinical studies have shown that vitamin D3 and sodium butyrate are effective in regulating tight gap junctions (Kong J, 2008) (Ma X, 2012) a finding which played a role in their selection as part of the GaRP formulation. To determine if GaRPv1 could restore gut integrity, a M cell/caco-2 transwell model was infected with HM605 AIEC ± GaRP and the TER was monitored for 2 days post-infection.

### M cell/Caco-2 model

Caco2-cl1 cells were grown and maintained as previously described. The human Burkitt's lymphoma cell-line Raji B was maintained in RPMI-1640 medium supplemented with 10% FBS, 8 mM L-glutamine, 100 U/ml penicillin and 100 mg/ml streptomycin. Raji B cells were seeded at 3×10⁵/ml and every third day cell suspensions were allowed to settle. Two thirds of the media was replaced with fresh culture media. Every ninth day, cells were split 1:3. All cells were maintained at 37°C with 5% CO2 in a humidified atmosphere.

M cells were generated by plating 5×10⁵ caco-2 cells onto the apical face of 12 mm Transwell cell culture inserts which had been precoated with Matrigel basement membrane matrix and DMEM media was placed in the basolateral compartment. Cells were maintained for 14-18 days, until the TER was > 600 Ω per Transwell (equivalent to 300 Ω cm² tissue monolayer). On the day of co-culture, Raji B cells were harvested, re-suspended in complete DMEM and 5×10⁵ cells added to the basal Transwell compartment. Importantly, following co-culture, both the apical and basal media were changed each day, with care being taken not to disturb the Raji B cells. Co-cultures were maintained for 4-6 days, until M-cells were generated. Parallel Caco2-cl1 monocultures (without Raji B cells in the basal compartment) were also generated on Transwell inserts and maintained as per M-cells. TER was measured throughout to monitor successful monolayer generation.

### Infection of the M cell model with AIEC HM605

It has previously been reported that HM 615 AIEC cause the breakdown of tight cell junctions in Caco-2 and T84 monolayers and induced cell death (Bailey JR, 2011). To recreate this effect, when the M cell models had been generated (day 20-24 dependent on when there was a concomitant drop in the TER), the medium was then changed to be antibiotic free and bacteria were added to the apical well of the Transwell insert at an MOI of 30 for a period of 4h. After 4h the cells were washed and incubated with 100µg/ml of gentamicin to kill any residual bacteria. At that point, GaRP minus bromelain was placed on the cells and the TER of all monolayers was measured at 2-hour intervals up to 12 hours and bacteria in the basal well were enumerated every 1 hours up to 4 hours.

### Results! Discussion:

### Restoration of the gastrointestinal cell layer after infection with AIEC

When the AIEC HM605 were suspended in normal media (no treatment control) or in GaRP (minus bromelain) and applied to the M cell model, GaRP formulation provided a protective effect on the integrity of the cell layer (Figure 4).

### Example 7 - Comparison of the anti-inflammatory activity of different gastrointestinal reprogramming compositions without bromelain.

The human colorectal adenocarcinoma cell-line C2BBe1 was grown and maintained in Dulbecco's modified Eagle's medium (DMEM) supplemented with 10% v/v fetal bovine serum (FBS), 4 mM L-glutamine, 100 U/ml penicillin and 100 mg/ml streptomycin. All experiments were conducted on C2BBe1 cells within 12 passages after thawing. Caco-2 cells were seeded at 5 ×10⁵ cells/well onto Transwell insert plates (0.33 cm², 0.4 µm pore size), The cell culture medium was changed every 3-day until the cells were fully differentiated (starting mean TER 1121 ± 196 cm^{2;} mean ± SD), and the cells were used in passages number within 12 passages after thawing.

In an experiment to evaluate the anti-inflammatory effect of bromelain or various GaRP formulations, 0.25 ml of test samples was applied to the apical side for 24 h, at the same time 0.6ml of a cocktail containing 50ng/ml IFN+ 10ug/ml LPS+5ng/ml TNF was added to the basolateral side in this model. After the 24h incubation culture supernatants from the apical side were collected and IL-8 quantitated by ELISA.

**Table 9** outlines the different permutations and combinations of formulation components that were tested for their ability to inhibit the release of IL-8 from polarized Caco-2 monolayers stimulated with LPS, TNF-o, IFN-β as previously described herein.

**Table 9**

| Formulation | Sodium butyrate | L-Thr | L-Trp | L-Glu | L-Arg | L-Met | Cholecalciferol |
|---|---|---|---|---|---|---|---|
| | | | | | | | |
| GaRPv1-1 | | 400 | 200 | 400 | 400 | 400 | 0.48 |
| GaRPv1-6 | | | | | | | 0.48 |
| GaRPv1-5 | | | | | | 400 | 0.48 |
| GaRPv1-2 | | | 200 | 400 | 400 | 400 | 0.48 |
| GaRPv1-3 | | | | 400 | 400 | 400 | 0.48 |
| GaRPv5 | 200 | 400 | | | | | 0.48 |
| GaRPv6 | 200 | 400 | 200 | | | | 0.48 |
| GaRPv1-4 | | | | | 400 | 400 | 0.48 |
| GaRPv8 | 400 | 400 | | | 400 | | 0.48 |
| GaRPv4 | 400 | 400 | 400 | 400 | 400 | 400 | 0.48 |
| GaRPv13 | 400 | 400 | | 400 | 400 | 400 | 0.48 |
| GaRPv12 | 400 | 400 | 400 | | 400 | 400 | 0.48 |
| GaRPv1 | 200 | 400 | 200 | 400 | 400 | 400 | 0.48 |
| GaRPv3 | 400 | 400 | 400 | | | | 0.48 |
| GaRPv11 | 400 | 400 | 400 | 400 | | 400 | 0.48 |
| GaRPv7 | 400 | 400 | | 400 | | | 0.48 |
| GaRPv9 | 400 | 400 | | | | 400 | 0.48 |
| GaRPv2 | 400 | 400 | | | | | 0.48 |
| GaRPv10 | 400 | 400 | 400 | 400 | 400 | | 0.48 |

### Results/ Discussion:

The data presented in Table 10 confirms that GARP without bromelain can significantly reduce the release of IL-8 compared to standard growth media (DMEM), p<0.001. When comparing all GaRP formulations to the original GaRPv1 there were no statistically significant differences, but when ranking the highest to lowest anti-inflammatory activity, on general specific observations could be made as shown in Table 11.

**Table 10 - The IL-8 inhibitory ability of various GaRP formulations and the individual formulation components**

| Formulation | % inhibition of the release of IL-8 Median ± SEM | P value compared to DMEM | P value compared to GaRPv4 |
|---|---|---|---|
| | | | |
| DMEM* | 0 | | <0.001 |
| GaRPv1* | 34 ± 6 | <0.001 | 0.589 |
| GaRPv1-1** | 24 ± 3 | | 0.526 |
| GaRPv1-2** | 23 ± 2 | | 0.483 |
| GaRPv1-3** | 29 ± 8 | | 0.518 |
| GaRPv1-4 | 10 ± 12 | | 0.603 |
| GaRPv1-5 | 19 ± 8 | | 0.440 |
| GaRPv1-6 | 11 ± 3 | | 0.703 |
| GaRPv2 | 23 ± 6 | | 0.725 |
| GaRPv3 | 29 ± 6 | | 0.843 |
| GaRPv4 | 25 ± 6 | | NT |
| GaRPv5 | 24 ± 8 | | 0.319 |
| GaRPv6 | 23 ± 10 | | 0.394 |
| GaRPv7 | 20 ± 8 | | 0.561 |
| GaRPv8 | 20 ± 8 | | 0.682 |
| GaRPv9 | 25 ± 7 | | 0.839 |
| GaRPv10 | 22 ± 13 | | 0.589 |
| GaRPv11 | 25 ± 6 | | 0.155 |
| GaRPv12 | 24 ± 10 | | 0.779 |
| GaRPv13 | 31 ± 7 | | 0.493 |
| sodium butyrate | 11 ± 15 | 0.033 | 0.220 |
| I-threonine | 18 ± 1 | <0.001 | 0.268 |
| L-tryptophan | 14 ± 7 | <0.001 | 0.188 |
| L-glutamine | 11 ± 7 | <0.001 | 0.145 |
| L-arginine | 6 ± 8 | 0.033 | 0.420 |
| L-methionine | 20 ± 5 | <0.001 | 0.387 |

| | | | |
|---|---|---|---|
| * The data is the mean and standard deviation of 11 independent experiments. Mann Whitney Rank Sum T test ** The data is the mean and standard deviation of 3 independent experiments. Mann Whitney Rank Sum T test | | | |

**Table 11 - Ranking of the various GaRP formulations without bromelain**

| **Formulation** | **Sodium butyrate** | **threonine** | **tryptophan** | **glutamine** | **arginine** | **methionine** | **Vit. D3** | **% inhibition of the release of IL-8** |
|---|---|---|---|---|---|---|---|---|
| **GaRPv1** | 200 | 400 | 200 | 400 | 400 | 400 | 0.48 | **34** |
| **GaRPv13** | 400 | 400 | | 400 | 400 | 400 | 0.48 | **31** |
| **GaRPv1-3** | | | | 400 | 400 | 400 | 0.48 | **29** |
| **GaRPv3** | 400 | | 400 | | | | 0.48 | **29** |
| **GaRPv4** | 400 | 400 | 400 | 400 | 400 | 400 | 0.48 | **25** |
| **GaRPv9** | 400 | 400 | | | | 400 | 0.48 | **25** |
| **GaRPv11** | 400 | 400 | 400 | 400 | | 400 | 0.48 | **25** |
| **GaRPv5** | 200 | | 400 | | | | 0.48 | **24** |
| **GaRPv1-1** | | 400 | 200 | 400 | 400 | 400 | 0.48 | **24** |
| **GaRPv12** | 400 | 400 | 400 | | 400 | 400 | 0.48 | **24** |
| **GaRPv2** | 400 | 400 | | | | | 0.48 | **23** |
| **GaRPv1-2** | | | 200 | 400 | 400 | 400 | 0.48 | **23** |
| **GaRPv6** | 200 | 400 | 200 | | | | 0.48 | **23** |
| **GaRPv10** | 400 | 400 | 400 | 400 | 400 | | 0.48 | **22** |
| **GaRPv7** | 400 | 400 | | 400 | | | 0.48 | **20** |
| **GaRPv8** | 400 | 400 | | | 400 | | 0.48 | **20** |
| **GaRPv1-5** | | | | | | 400 | 0.48 | **19** |
| **GaRPv1-6** | | | | | | | 0.48 | **11** |
| **GaRPv1-4** | | | | | 400 | 400 | 0.48 | **10** |
| **DMEM** | | | | | | | | 0 |

When ranking all GaRP formulations from highest to lowest anti-inflammatory activity, the inventor believes that several specific observations could be made (Table 11). Namely, sodium butyrate, L-threonine and cholecalciferol are all correlated with better anti-inflammatory effects, and in general, higher anti-inflammatory effects are observed using formulations which contained L-glutamine and L-tryptophan.

The examples provided herein confirm that bromelain is the key anti-inflammatory component of a GaRP dietary supplement as described herein where, as a sole agent, bromelain inhibited IL-8 secretion by 70 ± 7 (mean ± SEM). Without wishing to be bound by theory the inventor believes that the additional components of a GaRP formulation as described herein (sodium butyrate, amino acids and vitamin D3) act as a source of cellular energy and mucin synthetic precursors which enhance the anti-inflammatory activity of the bromelain. These experiments confirm previously published studies that each formulation component itself demonstrates a low level of anti-inflammatory activity. In one embodiment a GaRP formulation as described herein comprises or consists essentially of 80µg/ml bromelain, 400µg/ml sodium butyrate, 400pg/ml l-threonine and 0.48µg/ml vitamin D3.

### Example 8- Comparison of the anti-inflammatory activity of different gastrointestinal reprogramming compositions with 80ug/ml (equivalent to 40 pg/cell) bromelain.

To determine if any of the GaRP formulations exerted an additive or synergistic effect on the anti-inflammatory activity of bromelain, each formulation was re-tested after the addition of 80µg/ml bromelain (equivalent to 20pg/cell).

### Methodology

The human colorectal adenocarcinoma cell-line C2BBe1 was grown and maintained in Dulbecco's modified Eagle's medium (DMEM) supplemented with 10% v/v fetal bovine serum (FBS), 4 mM L-glutamine, 100 U/ml penicillin and 100 mg/ml streptomycin. All experiments were conducted on C2BBe1 cells within 12 passages after thawing. Caco-2 cells were seeded at 5 ×10⁵ cells/well onto Transwell insert plates (0.33 cm², 0.4 µm pore size), The cell culture medium was changed every 3-day until the cells were fully differentiated (starting mean TER 1121 ± 196 cm^{2;} mean ± SD), and the cells were used in passages number within 12 passages after thawing.

In an experiment to evaluate the anti-inflammatory effect of bromelain or various GaRP formulations, 0.25 ml of test samples was applied to the apical side for 24 h, at the same time 0.6ml of a cocktail containing **50ng/ml IFN+ 10ug/ml LPS+5ng/ml TNF** was added to the basolateral side in this model. After the 24h incubation culture supernatants from the apical side were collected and IL-8 quantitated by ELISA.

### Results! Discussion:

**Table 12** shows the results of the addition of 80ug/ml bromelain (equivalent to 40pg/cell) to the formulations tested in Example 7. The bromelain added 30-70% increase in the ability to suppress IL-8 secretion.

**Table 12 - The IL-8 inhibitory ability of various GaRP formulations plus 80ug/ml bromelain and the individual formulation components**

| **Formulation** | **% inhibition of the release of IL-8 (Median ± SEM)** | **P value compared to GaRPv1** + **80ug/ml bromelain** | **% increase in anti-inflammatory activity compared to equivalent formulation without bromelain (difference of means)** |
|---|---|---|---|
| DMEM* | 70 ± 7 | 0.340 | 70 |
| GaRPv1 * | 62 ± 7 | NT | 28 |
| GaRPv1-1** | 76 ± 10 | 0.948 | 52 |
| GaRPv1-2** | 71 ± 9 | 0.815 | 48 |
| GaRPv1-3** | 76± 4 | 0.619 | 47 |
| GaRPv1-4 | 77 ± 11 | 0.906 | 67 |
| GaRPv1-5 | 72 ± 11 | 0.741 | 53 |
| GaRPv1-6 | 75 ± 9 | 0.914 | 64 |
| GaRPv2 | 75 ± 8 | 0.625 | 52 |
| GaRPv3 | 67 ± 10 | 0.558 | 38 |
| GaRPv4 | 86 ± 12 | 0.294 | 61 |
| GaRPv5 | 85 ± 10 | 0.901 | 61 |
| GaRPv6 | 67 ± 13 | 0.718 | 44 |
| GaRPv7 | 75 ± 13 | 0.776 | 55 |
| GaRPv8 | 63 ± 10 | 0.590 | 43 |
| GaRPv9 | 65 ± 13 | 0.639 | 40 |
| GaRPv10 | 73 ± 14 | 0.701 | 51 |
| GaRPv11 | 63 ± 10 | 0.523 | 38 |
| GaRPv12 | 66± 9 | 0.498 | 42 |
| GaRPv13 | 78 ± 20 | 0.863 | 47 |
| sodium butyrate | 71 ± 7 | 0.609 | 60 |
| L-threonine | 73 ± 11 | 0.960 | 55 |
| L-tryptophan | 76 ± 2 | 0.934 | 62 |
| L-glutamine | 83 ± 1 | 0.710 | 72 |
| L-arginine | 73 ± 8 | 0.825 | 67 |
| L-methionine | 73 ± 10 | 0.825 | 53 |

| | | | |
|---|---|---|---|
| * The data is the mean and standard deviation of 11 independent experiments. Mann Whitney Rank Sum T test ** The data is the mean and standard deviation of 3 independent experiments. Mann Whitney Rank Sum T test | | | |

When ranking the formulations containing bromelain the best anti-inflammatory effect was observed as shown in **Table 13.**

**Table 13**

| Formulation: | Sodium butyrate | L-Thr | L-Trp | L-Glu | L-Arg | L-Met | Cholecalciferol | % inhibition of the release of IL-8 in the presence of 80ug/ml bromelain (Median ± SEM) |
|---|---|---|---|---|---|---|---|---|
| GaRPv4 | 400 | 400 | 400 | 400 | 400 | 400 | 0.48 | 86 ± 12 |
| GaRPv5 | 200 | | 400 | | | | 0.48 | 85 ± 10 |
| GaRPv13 | 400 | 400 | | 400 | 400 | 400 | 0.48 | 78 ± 20 |
| GaRPv1-4 | | | | | 400 | 400 | 0.48 | 77 ± 11 |
| GaRPv1-1 | | 400 | 200 | 400 | 400 | 400 | 0.48 | 76 ± 10 |
| GaRPv1-3 | | | | 400 | 400 | 400 | 0.48 | 76 ± 4 |
| GaRPv1-6 | | | | | | | 0.48 | 75 ± 9 |
| GaRPv2 | 400 | 400 | | | | | 0.48 | 75 ± 8 |
| GaRPv7 | 400 | 400 | | 400 | | | 0.48 | 75 ± 13 |
| GaRPv10 | 400 | 400 | 400 | 400 | 400 | | 0.48 | 73 ± 14 |
| GaRPv1-5 | | | | | | 400 | 0.48 | 72 ± 11 |
| GaRPv1-2 | | | 200 | 400 | 400 | 400 | 0.48 | 71 ± 9 |
| DMEM | | | | | | | | 70 ± 7 |
| GaRPv3 | 400 | | 400 | | | | 0.48 | 67 ± 10 |
| GaRPv6 | 200 | 400 | 200 | | | | 0.48 | 67 ± 13 |
| GaRPv12 | 400 | 400 | 400 | | 400 | 400 | 0.48 | 66 ± 9 |
| GaRPv9 | 400 | 400 | | | | 400 | 0.48 | 65 ± 13 |
| GaRPv8 | 400 | 400 | | | 400 | | 0.48 | 63 ± 10 |
| GaRPv11 | 400 | 400 | 400 | 400 | | 400 | 0.48 | 63 ± 10 |
| **GaRPv1** | **200** | **400** | **200** | **400** | **400** | **400** | **0.48** | **62 ± 72** |

| | | | | | | | | |
|---|---|---|---|---|---|---|---|---|
| Units for numbers are ug; e.g., 200 ug; 400 ug; 0/48 ug | | | | | | | | |

### Example 9 - Quantitation of the effect of GaRP and its individual formulation components on the expression of mucin toll-like receptors and pro-inflammatory genes.

A 75:25 caco-2/HT-29 co-culture was established as previously described herein and the effect of a 24h exposure to bromelain, threonine, sodium butyrate, vitamin D3 and the two different GaRP formulations on the expression of the following MUC genes was investigated using qPCR. At the end of the 24 h treatment, the supernatant in the 25 cm² flasks was collected and the coculture monolayer was trypsinised as previously described herein.

| Primer Set | Amplicon (bp) | Forward Primer (5'-3') | | Reverse Primer (5'-3') | |
|---|---|---|---|---|---|
| MUC1 | 139 | AGACGTCAGCGTGAGTGATG | SEQ ID NO: 1 | GACAGCCAAGGCAATGAGAT | SEQ ID NO: 2 |
| MUC2 | 264 | CGAAACCACGGCCACAACGT | SEQ ID NO: 3 | GACCACGGCCCCGTTAAGCA | SEQ ID NO: 4 |
| MUC3 | 146 | CCGGACCTCAATGACAACACT | SEQ ID NO: 5 | ACCACGATGCTGCCATTCCT | SEQ ID NO: 6 |
| MUC4 | 101 | CAGGCCACCAACTTCATCG | SEQ ID NO: 7 | ACACGGATTGCGTCGTGAG | SEQ ID NO: 8 |
| MUC5AC | 111 | CCTTCGACGGACAGAGCTAC | SEQ ID NO: 9 | TCTCGGTGACAACACGAAAG | SEQ ID NO: 10 |
| MUC5B | 141 | GCCCACATCTCCACCTATGAT | SEQ ID NO: 11 | GCAGTTCTCGTTGTCCGTCA | SEQ ID NO: 12 |
| MUC6 | 147 | CTGCCCTATACCAGCAATGGA | SEQ ID NO: 13 | CTGACCCATGTACTTCCGCTC | SEQ ID NO: 14 |
| MUC7 | 139 | GCAACTACCCTAGACCCATCA | SEQ ID NO: 15 | TAGGTGTGGTAATTGGGGCAG | SEQ ID NO: 16 |
| MUC8 | 101 | AGACAGGGTTTCTCCTCATTGG | SEQ ID NO: 17 | GGCTCGTGCTGTAATCCCA | SEQ ID NO: 18 |
| MUC12 | 141 | CCAGTTCAAGCGACCCTTTTA | SEQ ID NO: 19 | CGCTGTGGGATACTGTTGATT | SEQ ID NO: 20 |
| MUC13 | 153 | ATGCGTGCTGATGACAAGTTT | SEQ ID NO: 21 | ACACCGAAGGGTCAAATCATAGT | SEQ ID NO: 22 |
| MUC14 | 104 | AGCAACCAGCCGGTCTTATTC | SEQ ID NO: 23 | AGCACATTCGGTACAAACCCA | SEQ ID NO: 24 |
| MUC15 | 154 | TCCCAATGCTACACCTGCTCT | SEQ ID NO: 25 | ACTTGGTTCCACTATCAAGGGG | SEQ ID NO: 26 |
| MUC16 | 105 | GGAGCACACGCTAGTTCAGAA | SEQ ID NO: 27 | GGTCTCTATTGAGGGGAAGGT | SEQ ID NO: 28 |
| MUC17 | 115 | TCTCAGCACGTTAGGACAGGT | SEQ ID NO: 29 | TCGAGGTCATCTCAGGGTTGG | SEQ ID NO: 30 |
| MUC19 | 105 | TAAGACAGGCACAGCTAAAGTG | SEQ ID NO: 31 | GGGACTGTTACTGCTTCAGTGAT | SEQ ID NO: 32 |
| MUC20 | 126 | GAGACCTCTTCTAGGGCCTCA | SEQ ID NO: 33 | CACCATGAAGTTGGGAGATGTT | SEQ ID NO: 34 |
| TLR2 | 118 | ATCCTCCAATCAGGCTTCTCT | SEQ ID NO: 35 | GGACAGGTCAAGGCTTTTTACA | SEQ ID NO: 36 |
| TLR4 | 153 | AAGCCGAAAGGTGATTGTTG | SEQ ID NO: 37 | CTGAGCAGGGTCTTCTCCAC | SEQ ID NO: 38 |
| TLR5 | 109 | TCCCTGAACTCACGAGTCTTT | SEQ ID NO: 39 | GGTTGTCAAGTCCGTAAAATGC | SEQ ID NO: 40 |
| TLR9 | 223 | TTCCTCTATTCTCTGAGCCG | SEQ ID NO: 41 | GTAGGAAGGCAGGCAAGGTA | SEQ ID NO: 42 |
| NFkβ | 137 | GAAGCACGAATGACAGAGGC | SEQ ID NO: 43 | GCTTGGCGGATTAGCTCTTTT | SEQ ID NO: 44 |
| GAPDH | 87 | TGCACCACCAACTGCTTAGC | SEQ ID NO: 45 | GGCATGGACTGTGGTCATGAG | SEQ ID NO: 46 |
| 18S | 151 | GTAACCCGTTGAACCCCATT | SEQ ID NO: 47 | CCATCCAATCGGTAGTAGCG | SEQ ID NO: 48 |

When interpreting the gene expression data, the MUC genes and their associate mucins were divided into:
- Secreted mucins
- Membrane-associated mucins
- Non-gel forming mucins

### Secreted mucins

MUC2, MUC5AC, MUC5B, MUC6 and MUC19 act as secreted extracellular viscous secretions. Refer to Figure 5 for a summary of the effect of different concentrations of bromelain dissolved in the colon-targeted GaRP formulation components of sodium butyrate, L-threonine and Vitamin D3.

Table 14 summarises the association between each mucin and IBS or IBD.

**Table 14 - Summary of the association between each mucin listed and IBS or IBD**

| **Gene** | **RNA expression in normal GIT** | **Protein expression in normal GIT** | **Ulcerative colitis** | **Crohn's disease** | **IBS** |
|---|---|---|---|---|---|
| **MUC2** | Intestine | High in intestine | Decreased mRNA and protein | Decreased protein | Decreased mRNA and protein |
| **MUC5AC** | Stomach | High in stomach, not detected in intestine | Not detected | Decreased mRNA and protein | Not detected |
| **MUC5B** | Oral cavity, intestine | Minor expression in intestine | Not detected | Decreased mRNA | Not detected |
| **MUC6** | Stomach | High in stomach, not detected in small intestine, present in colon | Decreased mRNA and protein | Decreased mRNA and protein | Decreased mRNA |
| **MUC19** | Oral cavity | High in stomach, not detected in intestine | Unknown | Unknown | Unknown |

MUC 2 gene and protein expression has been found to be significantly downregulated in IBD and IBS and is considered the pivotal mucin for maintenance of the mucosal barrier. Its content in glycans, linked to mucin MUC2, serves as nutrient for bacteria, but also as a binding site and, induces the selection of specific microbial species, which are essential for maintaining the integrity, homeostasis and intestinal function. As seen in Figure 6, both 20 and 40µg/ml of bromelain in GaRP was able to increase gene expression 5 to 25-fold.

MUC6 is also downregulated in IBD and IBS. At bromelain concentrations ≥10µg/ml, the GaRP formulation components appeared to work additively if not synergistically (GaRPv1) with the bromelain to significantly increase the expression of MUC6 up to 30-fold.

MUC19 expression has not been characterised in IBS or IBD. At ≥10µg/ml the bromelain, the GaRPv1 formulation components appeared to exert and additive effect on the activity of the bromelain.

### Membrane-associated mucins

MUC1-4, MUC12, MUC15-17 and MUC20 are considered as membrane-associated mucins within the glycocalyx. Refer to Figure 6 for a summary of the effect of different concentrations of bromelain dissolved in the colon-targeted GaRP formulation components of sodium butyrate, L-threonine and vitamin D3.

**Table 15** summarises the association between each mucin and IBS or IBD.

**Table 15 - summary of the association between each mucin listed and IBS or IBD**

| **Gene** | **RNA expression in normal GIT** | **Protein expression in normal GIT** | **Ulcerative colitis** | **Crohn's disease** | **IBS** |
|---|---|---|---|---|---|
| **MUC1** | Oral cavity, stomach, intestine | High in stomach, low in intestine | Decreased mRNA and protein | Decreased mRNA | Decreased mRNA |
| **MUC3** | Intestine | High in large intestine | Unaltered | Decreased mRNA | Decreased mRNA |
| **MUC4** | Oral cavity, intestine | Low in small intestine | Unaltered | Decreased mRNA | Decreased mRNA |
| **MUC12** | Intestine | High in large intestine | Decreased protein | Decreased protein | Unknown |
| **MUC13** | Intestine | High in intestine | Increased mRNA | Increased mRNA in inflamed CD | Unknown |
| **MUC15** | Oral cavity | Low in intestine | Decreased mRNA | Unknown | Unknown |
| **MUC16** | Oral cavity, intestine | Low in intestine | Increased protein in active UC; Decreased mRNA in remission UC | Unknown | Unknown |
| **MUC17** | Intestine | High in intestine | Increased protein in active UC; | Unknown | Unknown |
| | | | Decreased mRNA in remission UC | | |
| **MUC20** | Oral cavity, stomach, intestine | High in intestine | Decreased mRNA and protein in active UC; Increased mRNA and protein in remission UC | Unknown | Unknown |

With the exception of MUC1 gene expression, at bromelain concentrations ≥10µg/ml the GARP/bromelain formulation appeared to significantly increase the expression of MUC3, MUC 4 and MUC15. Considering that these mucins are reduced in IBD and in some instanced in IBS these data suggest that the GaRP product may be able to stimulate the production of the mucins which may result in re-establishing the homeostasis of the intestine. In general, the colon targeted GaRPv1 formulation performed better than the GaRPv2, protein studies and mucin quantitation work is currently on-going to confirm this observation.

MUC13, MUC16 and MUC17 expression is increased in IBD. At bromelain concentrations ≥20µg/ml the GARPv2/bromelain formulation was able to significantly reduce the gene expression.

### Non-gel forming mucins

MUC7 and MUC8 are non-gel forming mucins which have not been well characterised in IBD or IBS. Referring to Figure 7, bromelain concentrations ≥10µg/ml was able to significantly increase the gene expression.

Without wishing to be bound by theory the inventor believes that the MUC gene expression data provided herein show that the concentrations of bromelain and colon-targeted formulation components used in the GaRP dietary supplement will be sufficient to stimulate mucin gene expression which is expected, in turn, to promote mucosal healing.

### Example 10 - Mucosal healing abilities of the GaRP product: Demonstrating the synergistic relationship between L-threonine and Vitamin D3.

### Cell lines and establishing the Caco-2:HT-29-MTX 75:25 coculture (simulating the colon)

The human colorectal adenocarcinoma cell-line Caco-2-BBe1 (obtained from professor Barry Campbell, University of Liverpool) was grown and maintained in Dulbecco's modified Eagle's medium (DMEM) supplemented with 10% v/v fetal bovine serum (FBS), 4 mM L-glutamine, 100 U/ml penicillin and 100 mg/ml streptomycin. All experiments were conducted on Caco-2-BBe1 cells within 12 passages after thawing. Cell cultures were incubated in a humidified 5% CO₂ incubator at 37°C. Cells were maintained in an incubator at 37 °C in a 5% CO₂ atmosphere with medium replaced every 48 h. At 80% confluence, cells were detached with 10 ml of 1% trypsin-EDTA (Sigma-Aldrich) for 5 min, followed by the addition of 40 ml of DMEM with 10% FBS to terminate the trypsinisation. The cell suspension was pelleted by centrifugation (200 g, 5 min) and resuspended in fresh DMEM with 10% FBS, and then sub-cultured into new flasks at 1:4 dilution.

This co-culture model was used to simulate the human colonic epithelium. The Caco-2 cells (passage 8) and the HT29-MTX cells (passage 9) were combined at a 75:25 ratio in DMEM with 10% FBS. The combined cells (6.4 × 10⁴ cells/ml) were seeded into 25 cm² in a total of 5 ml per flask. The coculture was incubated at 37°C in a 5% CO₂ atmosphere with medium refreshed every 48h. At 15 days post seeding, the coculture monolayer was washed three times in DMEM and replaced with 5 ml of prewarmed test solution, and then incubated for 24 h.

### Treatment of the Caco-2:HT-29-MTX 75:25 coculture

The test solutions of cholecalciferol (VD3) and L-Threonine (L-Thr) (Sigma-Aldrich) in culture medium were prepared one day before the treatment. At 15 days post seeding, the coculture monolayer was washed three times at hourly interval with culture medium, which was then replaced with 5 ml of prewarmed test solution, and then incubated for 24 h.

### Quantitative polymerase chain reaction (qPCR) analysis of gene expression

At the end of the 24 h treatment, the supernatant in the 25 cm² flasks was collected and the coculture monolayer was trypsinised as previously described. Cells were collected by centrifugation at 5,000 g for 5 min, 4°C. After washed three times with ice-cold PBS, the cells were resuspended in 500 µl of ice-cold PBS. The total RNA of 2 × 10⁶ cells per sample was extracted using the PureLink RNA mini kit (Invitrogen). For real-time PCR analysis, 1.5 µg of RNA (with A260/A280 ratio between 2.00 and 2.20) was reverse transcribed into cDNA using the High Capacity cDNA RT kit (Applied Biosystems, Foster City, CA, USA).

The expression of the human MUC2 and MUC7 genes in the Caco-2:HT-29-MTX 75:25 coculture was quantified using the PowerUp SYBR green Master mix (Applied Biosystems). The primers for detecting the expression of MUC2 (Forward, 5'-CGAAACCACGGCCACAACGT-3' (SEQ ID NO: 3); Reverse, 5'-GACCACGGCCCCGTTAAGCA-3' (SEQ ID NO: 4)) and MUC7 (Forward, 5'-GCAACTACCCTAGACCCATCA-3' (SEQ ID NO: 15); Reverse, 5'-TAGGTGTGGTAATTGGGGCAG-3' (SEQ ID NO: 16) were purchased from Sigma-Aldrich. The 10 µl qPCR reaction was set up with 20 ng cDNA and 500 nM primers in triplicates on MicroAmp optical 384-well reaction plates (Applied Biosystems) using a Biomek-4000 automated liquid handler (Beckman Coulter, Brea, CA, USA). Amplification was run in a ViiA7 Real-Time PCR system (Thermo Fisher Scientific) at the following conditions: 95°C for 20 seconds followed by 40 cycles of 95°C for 1 second and 60°C for 20 seconds; and a melt curve stage: 95°C, 15 s; 60°C, 60 s. The results of qPCR were analysed with the 2-ΔΔCT method as previously described using the QuantStudio Software V1.3 (Applied Biosystems). Relative gene expression values were normalized by using GAPDH and 18S as the multiple endogenous reference genes.

### Results

When interpreting the gene expression data, the MUC genes and their associated mucins were divided into three classes:
- Secreted mucins
- Membrane-associated mucins
- Non-gel forming mucins

### Secreted mucins

MUC2, MUC5AC, MUC5B, MUC6 and MUC19 act as secreted extracellular viscous secretions. Table 16 summarises the association between each mucin and IBS or IBD.

**Table 16 - Summary of the association between each mucin and IBS or IBD**

| **Gene** | **RNA expression in normal GIT** | **Protein expression in normal GIT** | **Ulcerative colitis** | **Crohn's disease** | **IBS** |
|---|---|---|---|---|---|
| **MUC2** | Intestine | High in intestine | Decreased mRNA and protein | Decreased protein | Decreased mRNA and protein |
| **MUC5AC** | Stomach | High in stomach, not detected in intestine | Not detected | Decreased mRNA and protein | Not detected |
| **MUC5B** | Oral cavity, intestine | Minor expression in intestine | Not detected | Decreased mRNA | Not detected |
| **MUC6** | Stomach | High in stomach, not detected in small intestine, present in colon | Decreased mRNA and protein | Decreased mRNA and protein | Decreased mRNA |
| **MUC19** | Oral cavity | High in stomach, not detected in intestine | Unknown | Unknown | Unknown |

### Membrane-associated mucins

MUC1-4, MUC12, MUC15-17 and MUC20 are considered as membrane-associated mucins within the glycocalyx. Table 17 summarises the association between each mucin and IBS or IBD.

**Table 17 - Summary of the association between each mucin and IBS or IBD**

| **Gene** | **RNA expression in normal GIT** | **Protein expression in normal GIT** | **Ulcerative colitis** | **Crohn's disease** | **IBS** |
|---|---|---|---|---|---|
| **MUC1** | Oral cavity, stomach, intestine | High in stomach, low in intestine | Decreased mRNA and protein | Decreased mRNA | Decreased mRNA |
| **MUC3** | Intestine | High in large intestine | Unaltered | Decreased mRNA | Decreased mRNA |
| **MUC4** | Oral cavity, intestine | Low in small intestine | Unaltered | Decreased mRNA | Decreased mRNA |
| **MUC12** | Intestine | High in large intestine | Unaltered | Decreased protein | Unknown |
| **MUC13** | Intestine | High in intestine | Increased mRNA | Increased mRNA in inflamed CD | Unknown |
| **MUC15** | Oral cavity | Low in intestine | Decreased mRNA | Unknown | Unknown |
| **MUC16** | Oral cavity, intestine | Low in intestine | Increased mRNA in remission UC | Unknown | Unknown |
| **MUC17** | Intestine | High in intestine | Increased protein in active UC; Decreased mRNA in remission UC | Unknown | Unknown |
| **MUC20** | Oral cavity, stomach, intestine | High in intestine | Decreased mRNA and protein in active UC; Increased mRNA and protein in remission UC | Unknown | Unknown |

### Non-gel forming mucins

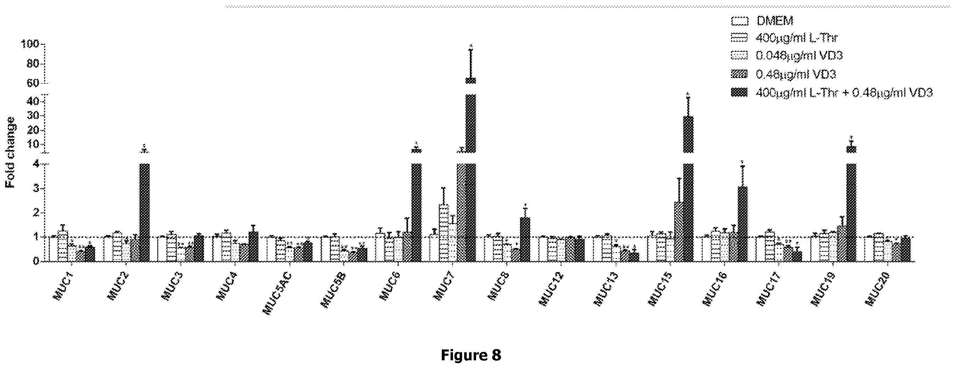

MUC7 and MUC8 are non-gel forming mucins which have not been well characterised in IBD or IBS. Refer to the below Figure 8 which demonstrated the synergistic effect resultant from the combination of L-threonine and vitamin D3 on the above-described MUC genes.

L-threonine (400µg/ml), as a sole agent did not exert a statistically significant effect on the expression of any of the MUC genes tested. The effect of i) vitamin D3 and the combination of ii) 0.48 µg/ml vitamin D3 and 400µg/ml L-threonine, on the expression of MUC genes is summarized in Table 18.

**Table 18 - Summary of the effects of the combination of vitamin D3 and L-threonine**

| | | | | | | **Effect on MUC gene expression** | | |
|---|---|---|---|---|---|---|---|---|
| **Gene** | **RNA expression in normal GIT** | **Protein expression in normal GIT** | **Ulcerative colitis** | **Crohn's disease** | **IBS** | **0.048**µg/ml vitamin D3 | **0.48**µg/ml vitamin D3 | **0.48**µg/ml vitamin D3+ 400µg/ml L-threonine |
| **MUC1** | Oral cavity, stomach, intestine | High in stomach, low in intestine | Decreased mRNA and protein | Decreased mRNA | Decreased mRNA | Decreased by 37% | Decreased by 58% | Decreased by 39% |
| **MUC3** | Intestine | High in large intestine | Unaltered | Decreased mRNA | Decreased mRNA | Decreased by 43% | No effect | No effect |
| **MUC4** | Oral cavity, intestine | Low in small intestine | Unaltered | Decreased mRNA | Decreased mRNA | No effect | No effect | No effect |
| **MUC12** | Intestine | High in large intestine | Unaltered mRNA Decreased protein | Decreased protein | Unknown | No effect | No effect | No effect |
| **MUC13** | Intestine | High in intestine | Increased mRNA | Increased mRNA in inflamed CD | Unknown | Decreased by 37% | Decreased by 56% | Decreased by 65% |
| **MUC15** | Oral cavity | Low in intestine | Decreased mRNA | Unknown | Unknown | No effect | No effect | **Increased by 2931%** |
| **MUC16** | Oral cavity, intestine | Low in intestine | Increased mRNA in remission UC | Unknown | Unknown | No effect | No effect | **Increased by 306%** |
| **MUC17** | Intestine | High in intestine | Increased protein in active UC; Decreased mRNA in remission UC | Unknown | Unknown | Decreased by 31% | Decreased by 40% | Decreased by 59% |
| **MUC20** | Oral cavity, stomach, intestine | High in intestine | Decreased mRNA and protein in active UC; | Unknown | | Decreased by 18% | Decreased by 27% | No effect |
| | | | Increased mRNA and protein in remission UC | | Unknown | | | |
| **MUC2** | Intestine | High in intestine | Decreased mRNA and protein | Decreased protein | Decreased mRNA and protein | No effect | No effect | **Increased by 446%** |
| **MUC5AC** | Stomach | High in stomach, not detected in intestine | Not detected | Increased mRNA and protein | Not detected | Decreased by 42% | Decreased by 42% | Decreased by 23% |
| **MUC5B** | Oral cavity, intestine | Minor expression in intestine | Not detected | Decreased mRNA | Not detected | Decreased by 56% | Decreased by 63% | Decreased by 44% |
| **MUC6** | Stomach | High in stomach, not detected in small intestine, present in colon | Decreased mRNA and protein | Decreased mRNA and protein | Decreased mRNA | No effect | No effect | **Increased by 655%** |
| **MUC19** | Oral cavity | High in stomach, not detected in intestine | Unknown | Unknown | Unknown | No effect | No effect | **Increased by 865%** |
| **MUC7** | Oral cavity | Not detected in small intestine | Unknown | Unknown | Unknown | No effect | No effect | **Increased by 6536%** |
| **MUC8** | Low in GIT | Unknown | Unknown | Unknown | Unknown | Decreased by 30% | Decreased by 50% | **Increased by 180%** |

Synergistic effects are highlighted in bold text.

The evidence that L-threonine and vitamin D3 unexpectedly acted in synergy by increasing the expression of MUC15, MUC2, MUC6, MUC 19, MUC7 and MUC8 was further explored by titering the L-threonine and vitamin D3. The MUC2 and MUC7 expression were used as exemplar mucin genes.

As seen in Figures 9A & 9B, individually, both L-threonine and vitamin D3 did not exert a major effect on the expression of MUC2 (primary secreted, gel forming mucin in the gastrointestinal track) or MUC7 (a non-gel forming mucin).

In both IBS and IBD the expression of the MUC2 gene, protein and mucin have been shown to be decreased which results in an altered gut integrity and dysbiotic microbiome, factors which lead to systemic inflammation.

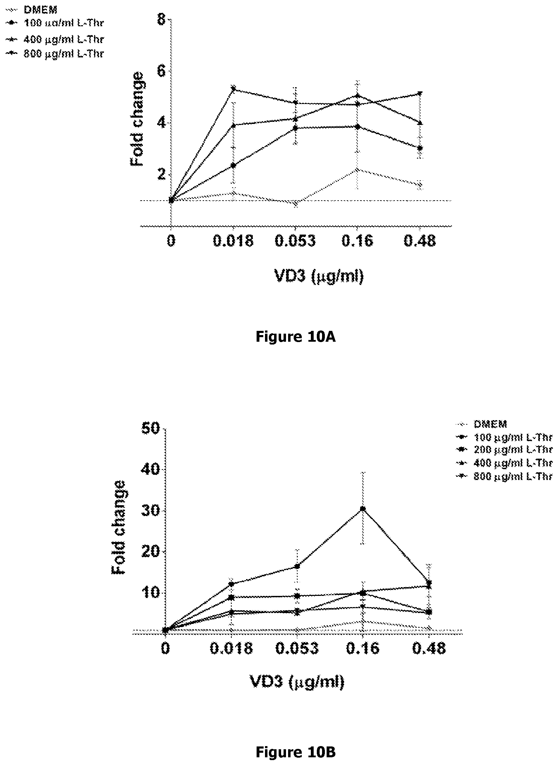

When conducting a titration of the vitamin D3 from 0-0.48µg/ml in combination with 0-800µg/ml of L-threonine, it became evident that at ≥0.053µg/ml of vitamin D3 the concentration of L-threonine was the primary factor that impacted the increase in MUC 2 and MUC 7 gene expression (Figure 10A & 10B)

### Example 11 - Bromelain and GaRP can reduce endoscopic disease in a TNBS colitis model Evaluating GaRP in a TNBS mouse model

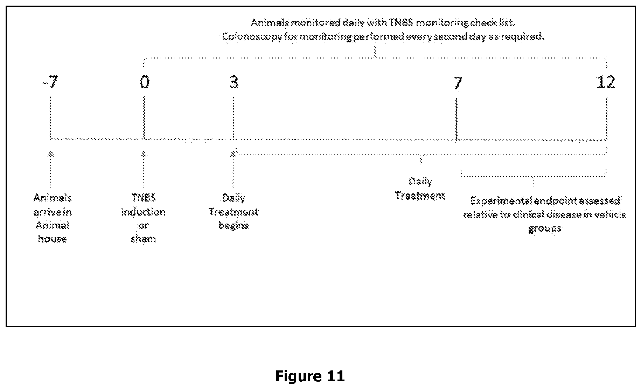

The 2,4,6-Trinitrobenzenesulfonic acid solution (TNBS) colitis was be induced in 5-week-old C56/BL/6 mice by rectal installation of TNBS (5% w/v) in 50% ethanol. Animals were randomised into treatment groups and treated as outlined in Figure 11.

Eight treatment groups (n=4 for healthy controls and 8-9 animals/TNBS group) have been completed. A detailed description of the treatment groups is provided in Table 19.

**Table 19 - Detailed description of treatment groups**

| **Group No.** | **Treatment Group** | | | | | |
|---|---|---|---|---|---|---|
| **1** | **Animals with rectal installation of 50% ethanol, no treatment** | | | | | |
| **2** | **TNBS treated:** | | | | | |
| | Orally administered: **Oral vehicle containing** 200ul water containing 1.52% w/v Tween 80/1.35% and hydroxypropylmethyl cellulose | | | | | |
| **3** | **TNBS treated:** | | | | | |
| | Rectally administered: **Rectal vehicle containing** 160ul water containing 1.5% w/v Tween 80, 3.3% hydroxypropylmethyl cellulose & 0.11% w/w propylene glycol | | | | | |

| | | **Treatment contains (mg/kg/day)** | | | | |
|---|---|---|---|---|---|---|
| | | **Brom elain** | **Sodium butyrat e** | **L-threoni ne** | **Vitami n D3** | **Menth ol** |
| **4** | **TNBS treated:** | 0 | 184 | 277 | 0.02 | 0 |
| | Rectally administered: **GaRP in rectal vehicle** | | | | | |
| **5** | **TNBS treated:** | 75 | 0 | 0 | 0 | 0 |
| | Orally administered **75mg/kg bromelain in water** | | | | | |
| **6** | **TNBS treated:** | 75 | 0 | 0 | 0 | 0 |
| | Orally administered:**75mg/kg bromelain in oral vehicle** | | | | | |
| 7 | **TNBS treated:** | 75 | 0 | 0 | 0 | 15 |
| | Orally administered **75mg/kg bromelain and menthol in oral vehicle** | | | | | |
| **8** | **TNBS treated:** | 75 | 184 | 277 | 0.02 | 15 |
| | Orally administered: **75mg/kg bromelain and menthol in oral vehicle** | | | | | |
| | Rectally administered: **GaRP in rectal vehicle** | | | | | |

The following endoscopic scoring system was used: i) Score 5-7 = mild disease, ii) Score 8-11 = moderate disease and iii) Score 12-15 = severe disease. The population baseline disease severity was:
- Mild: 34% (21/62 animals)
- Moderate: 62% (38/62 animals)
- Severe: 4% (3/62 animals)

### 8.1: Safety

Of the original 72 animals that were randomised in the study, ten animals were excluded from analysis as they died from complications from anaesthesia or losing >20% of body before commencement of treatment. Deaths on study are summarised in Table 20.

**Table 20 - Summary of deaths in study**

| **Treatment Group** | **Number of deaths** | **Reason for death** | **Days after commencement of treatment** |
|---|---|---|---|
| TNBS: Rectal GaRP | 1 | >20% weight loss | 1 |
| TNBS: Bromelain in water | 2 | >20% weight loss | 1 and 5 |
| TNBS: Bromelain in oral vehicle | 1 | >20% weight loss | 7 |
| TNBS: Bromelain/menthol in oral vehicle and rectal GaRP | 1 | >20% weight loss | 8 |

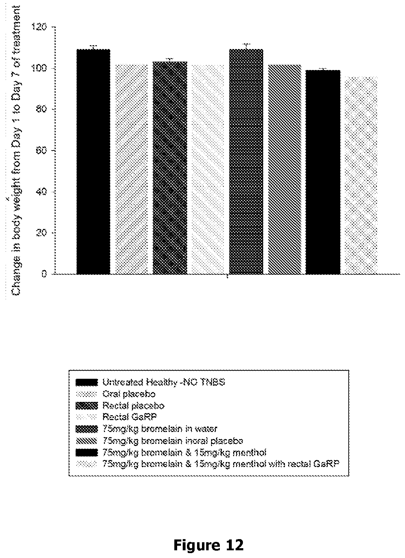

The animals that were euthanised due to weight loss looked well and were functioning normally but due to ethics guidelines the animals were euthanised. Refer to Figure 12 which compares the change in body weight from Day 0 of treatment to experimental endpoint (Day 7)

The only groups which showed any significant difference in experimental endpoint body weight were the:
- TNBS- 75mg/kg bromelain/15mg/kg menthol in oral vehicle: When compared to 75mg/kg bromelain in water or oral vehicle, the 75mg/kg bromelain/15mg/kg menthol in oral vehicle reduced the endpoint weight by 9% (p=0.024).
- TNBS-75mg/kg bromelain/15mg/kg menthol in oral vehicle and GaRP in rectal vehicle: When compared to 75mg/kg bromelain in water, oral or rectal vehicle; the 75mg/kg bromelain/15mg/kg menthol/rectal GaRP reduced the endpoint weight by 5-12% (p=0.001).

When comparing these groups over the treatment period, the animals maintained their original weight as can be observed in Figure 13.

Published studies found that administering 10mg/kg L-menthol to mice increases core temperature, uncoupling protein 1 expression in brown adipose tissue, non-shivering thermogenesis activity and insulin sensitivity leading to attenuated body weight gain. In the GaRP product, the mice receive 15mg/kg daily which may account for the observed lack of weight gain. No toxicities were noted.

### Efficacy

Refer to Figure 14 which compares the change in endoscopic score from Day 0 (before treatment) to Day 7 (experimental endpoint). Statistically significant differences were noted in the following treatment groups:
- **Colon-targeted components of the GaRP product (rectally installed sodium butyrate/L-threonine/vitaminD3):** Significantly decreased colitis as quantitated by endoscopic score. When compared to a rectal vehicle control, GaRP improved the endoscopic score by 135% (p=0.018, n=8)
- **Orally administered bromelain/peppermint oil combined with colon-targeted components of the GaRP product (rectally installed sodium butyrate/L-threonine/vitaminD3):** When compared to the oral and rectal vehicle control, this formulation improved the endoscopic score by 148% (p=0.012, n=9).
- **Orally administered bromelain in water:** When compared to an oral vehicle control, bromelain improved the endoscopic score by 65% (p=0.038, n=8).

When comparing the individual parameters such as stool consistency, extent of involved area, granularity of mucosal surface, changes in vascular pattern and thickening of colon wall there were no significant differences between treatment groups.

Refer to Figure 15 which compares the effect of the different treatments and control vehicles on colon length, colon weight and the ratio of the colon weight/length. No significant differences were noted between the treatment groups.

### Conclusions:

Colon-targeted components of the GaRP product (rectally installed sodium butyrate/L-threonine/vitaminD3) without bromelain significantly improved experimental colitis. The addition of orally administered bromelain/menthol exerted a slight additive effect in the colitis model which was not significantly better than the GaRP alone. It was noted that the bromelain in oral vehicle did not perform as well as the bromelain in water which the inventor believes suggests that some components of the oral vehicle may be interfering with the release of the bromelain in both the small and large intestine. It is believed that specific formulation to provide release of bromelain and/or any of the components of the compositions and formulations described herein is within the skill of those in the art of pharmaceutical formulations for delivery to the gastrointestinal system, also as described elsewhere herein.

### Example 12 - Bromelain and GaRP can reduce endoscopic disease in a TNBS colitis model without negative drug interactions with prednisolone.

Prednisolone is known to have 55 major, 390 moderate and 39 minor drug interactions. Amongst the known negative effects are interactions with several common ingredients such as buffering agents which may impair the absorption of prednisolone. Herbal medicinals are popular remedies and are being used by an increasing number of patients who typically do not advise their clinicians of concomitant use. Therefore, doctors and clinicians recognize that known or potential drug-herb interactions exist and should be screened for. This concept can reasonably be extended to non-herbal based nutritional agents. Of particular relevance to irritable bowel syndrome and inflammatory bowel disease are the general use of corticosteroids to control inflammation. According to Miller [Miller LG "Herbal medicinals: selected clinical considerations focusing on known or potential drug-herb interactions." Arch Intern Med 158 (1998): 2200-11], the theoretical concern underlying this drug-herb interaction is that immunostimulant herbs will offset or minimise the immunosuppressive effects of the corticosteroids. In light of these concerns the inventors examined the effect of prednisolone in the presence and absence of a formulation as described herein.

Mice were induced with colitis by administering TNBS in the same manner as example 11. After three days the lesions were scored by endoscopy and the animals treated with i). 150 mg/kg bromelain in water, ii) 150 mg bromelain and rectally administered threonine/sodium butyrate/vitamin D3, iii). 2 mg/kg oral prednisolone or iv). 150 mg bromelain and rectally administered threonine/sodium butyrate/vitamin D3 plus 2 mg/kg oral prednisolone. Endoscopic scores were measured at day 11 and the change in endoscopic score between day 3 and day 11 calculated (Fig. 16). Table 21 shows the change in endoscopic score for the above groups and the statistical significance relative to placebo.

**Table 20 - Endoscopic scores**

| Group | n | Mean reduction in endoscopic score | Standard deviation | significance |
|---|---|---|---|---|
| Placebo | 34 | 3.00 | 2.00 | |
| 150 mg bromelain in water | 8 | 5.00 | 1.41 | 0.0036 |
| 150 mg Bromelain in GaRP^{#} | 11 | 4.73 | 2.42 | 0.0294 |
| 150 mg Bromelain in GaRP^{#} with 2 mg/kg prednisolone | 7 | 4.86 | 1.36 | 0.0080 |
| 2 mg/kg prednisolone | 12 | 4.25 | 2.38 | 0.0445 |
| GaRP# no bromelain | 19 | 4.42 | 2.32 | 0.0167 |

| | | | | |
|---|---|---|---|---|
| # GaRP = Threonine/sodium butyrate/vitamin D3 in this example | | | | |

Groups are i) placebo, ii) 150 mg bromelain in water orally, iii) 150 mg bromelain & 15 mg/kg menthol orally plus GaRP rectally, iv) 150 mg bromelain & 15 mg/kg menthol orally plus GaRP and 2 mg/kg prednisolone rectally, v) 2 mg/kg prednisolone rectally, and vi) GaRP rectally. GaRP is sodium butyrate 185 mg/kg, threonine 280 mg/kg and Vitamin D3 3.84 micrograms per kg. Human equivalent dose are bromelain 960 mg/80 kg person); menthol 100 mg/80 kg person); Threonine 1800 mg/80 kg person); sodium butyrate 1200 mg/80 kg person) and vitamin D3 25 micrograms per 80 kg person). The threonine, sodium butyrate and vitamin D3 were formulated and administered as a rectal gavage at the top of the colon to simulate a formulation which releases in the colon; i.e., to achieve administration to the lower intestine. The human formulation of a composition as described herein could not be used because the pH of the mouse colon is lower than that of a human and the transit time shorter, which means that a targeted human formulation would not release the contents in an animal study because the formulation would travel to quickly through the mouses GI tract and the pH required to erode the protective coating required for release would not be achieved.

From the results presented here the inventors believe that it is apparent that the formulation comprising sodium butyrate, L-threonine and Vitamin D does not interfere with the efficacy of prednisolone, and that 1) bromelain alone, 2) threonine/butyrate/vitamin D3 alone, and 3) the combination bromelain/menthol orally plus threonine/butyrate/vitamin D3 rectally are as efficacious as 2mg/kg prednisolone in this experiment.

### Example 13 - Formulation of a dosage form

### Part A tablet/minitablet/capsule/components :

The objective was to develop a stable enteric coated mini-tablet formulation containing bromelain and menthol in the ratio 6.8:1 (±30% i.e. range 4.75:1 to 8.85:1). The developed formulation is prepared by direct compression process

### Finalized manufacturing formula

| **S. No.** | **Ingredients** | |
|---|---|---|
| **Menthol pre-mix** | | **mass** |
| 1. | L-Menthol | 100.00 |
| 2. | FujiSil | 100.00 |
| 3. | Bromelain | 528.00 |
| 4. | Avicel SMCC 90 | 350.00 |
| 5. | Fujicalin | 200.00 |
| 6. | Hydroxy propyl Cellulose(Klucel EXF) | 87.50 |
| 7. | Crospovidone (kollidon Cl) | 86.00 |
| 8. | Magnesium Stearate (Ligamed MF-2V) | 14.00 |
| 9. | Aerosil 200 | 25.00 |

| **Seal Coating** | | |
|---|---|---|
| 10. | Eudraguard Control | 22.37 |
| 11. | HPMC 5 cps (Methocel E5 premium LV) | 22.37 |
| 12. | Talc | 4.47 |
| 13. | Purified Water | q.s. |

| **Enteric coating** | | |
|---|---|---|
| 14. | Eudraguard Control | 131.8 |
| 15. | Sodium Alginate | 70.97 |
| 16. | Glycerin | 10.14 |
| 17. | Talc | 40.55 |
| 18. | Lake Blend Orange | 0.413 |
| 19. | Purified Water | q.s. |

### Manufacturing procedure:

1. **Menthol Pre-mix:** The menthol was melted under heating at 70°C. FujiSil was added to molten menthol and stirred under heating. Stirring continued until it forms a free flowing powder.
2. Aerosil 200, Fujicalin and Menthol Pre-mix shall be blended in a 10L octagonal blender for 15 min at 15 rpm.
3. Bromelain, Avicel SMCC 90, Klucel EXF and crospovidone were sifted through # 40 ASTM attached to mechanical Sifter and blended for 15 min along with step 2 materials in a 30L octagonal Blender at 15 rpm.
4. Magnesium stearate was sifted through #60 ASTM.
5. **Lubrication:** Step 4 materials were added to step 3 materials and blended for 5min in 30L octagonal blender at 15 rpm.
6. **Compression:** Lubricated blend was compressed into mini tablets using 2.50 x 2.50 mm round biconvex shaped multi tip punches.
7. **Seal Coating:** To Eudraguard Control, required quantity of purified water was added. Methocel E5 Premium LV and talc added under stirring. The stirring continued throughout the coating operation. The suspension was sprayed onto the core tablets bed in the automatic tablet coater.
8. **Enteric coating:.** Glycerin was added to Eudraguard control under stirring. Required amount of purified water was added to the Eudraguard control and glycerin mixture under stirring. Sodium Alginate and Talc were blended in a polybag and added to the above mixture slowly without formation of lumps. The required amount of lake colorant(s) is homogenized with small quantity of water and added to the suspension under stirring. Stirring continued for 3 hours prior to the coating operation.

### Compression parameters:

| • **S.No.** | • **Parameters** | • **Range** |
|---|---|---|
| 1. | • Tablet Weight (mg) | • 145±5% |
| 2. | • Hardness (kp) | • 3±2 |
| 3. | • Thickness (mm) | • 2.5±0.4 |
| 4. | • Disintegration time (min) | • NMT 15 |
| 5. | • Friability (%) | • NMT 1.0 |

Direct compression strategy was selected based on the stability profile of bromelain. The adsorption technique was employed for menthol due to its sublimation tendency at low temperature, although alternate methods including dissolution of menthol in migloyl, embedding in waxes or the incorporation of micelles of menthol in vitamin E were explored.

The minitablets were subjected to dissolution testing according to the standard USP method with type II apparatus USP29NF24<711>. Minitablets were stirred in 0.1 N hydrochloric acid for 1 hour before adjusting to pH 6.8 for 5 hours. Samples were taken at time 0, 30 minutes, 60 minutes prior to pH adjustment then post adjustment +30 minutes, +60 minutes and hourly thereafter. Bromelain was analysed and the titre plotted against time as shown in Figure 17.

### Part B tablet/minitablet/capsule/component:

The drug product was designed as minitablets for delivery of drugs in the lower part of the small intestine and colon regions. The product was designed to be physically and chemically stable and to possess potency for the duration of its shelf life. A formulation and process were developed to en sure consistent product that meets all criteria of quality. Ratio of threonine to sodium butyrate is 1.5:1 ± 30% i.e. (1.05:1 to 1.95:1).

### Manufacturing formula

| **Ingredient** | **mass** |
|---|---|
| **Dry mix** | |
| Sodium butyrate | 600 |
| L-Threonine | 900 |
| Microcrystalline cellulose (Avicel PH 102) | 100 |
| Dicalcium phosphate anhydrous (A Tab) | 200 |
| Crospovidone (Kollidon CL) | 40 |

| **Binder** | |
|---|---|
| Povidone K90 F (Kollidon 90 F) | 70 |
| Cholecalciferol | 0.0125* |
| Butylated hydroxy tolune | 0.4** |
| Isopropyl alcohol | q.s |

| **Extragranular** | |
|---|---|
| Magnesium Stearate (Ligamed MF-2V) | 13.00 |

| **Seal coating** | |
|---|---|
| Hydroxypropyl methylcellulose (HPMC 5 cps) | 48.10 |
| Talc | 48.10 |
| Isopropyl alcohol | q.s |
| Dichloromethane | q.s |

| **Eudraguard biotic layer** | |
|---|---|
| Eudraguard biotic solid | 100.00 |
| PlasACRYL T20 | 10.00 |
| Water | q.s |

| **Eudraguard Control layer^{$}** | |
|---|---|
| Eudraguard Control solid | 77.52 |
| Sodium alginate | 41.74 |
| Glycerine | 5.96 |
| Talc | 23.85 |
| Water | q.s |

| | |
|---|---|
| *Add 10% overage, ** add 20% overage | |

### Manufacturing process:

1. Sifting: Sodium butyrate, L-Threonine, microcrystalline cellulose, dicalcium phosphate anhydrous (A Tab), crospovidone (Kollidon CL) were passed through ASTM #24 mesh.
2. Binder solution: Add Butylated hydroxytolune, vitamin D3, povidone K90 F to isopropyl alcohol and continue stirring until a clear solution is formed.
3. The above materials are combined by wet granulation and the product dried on a fluid bed drier, then milled and sifted with magnesium stearate.
4. Compression: Blend was compressed into mini tablets using 2.50 × 2.50 mm round biconvex shaped multi tip punches.
5. Seal coating: Hydroxy propyl methyl cellulose in isopropyl alcohol/methylene chloride seal coat was applied.
6. Eudraguard biotic dispersion: Eudraguard biotic, water and PlasACRYL T20 dispersion were sprayed on the sealed minitablets.
7. Eudraguard control dispersion: glycerin, Eudraguard control, sodium alginate and talc were mixed to form a dispersion which was coated to the minitablets.

### Compression parameters:

| • **Parameters** | • **Range** |
|---|---|
| • Tablet Weight (mg) | • 148±5% |
| • Hardness (kp) | • 3±2 |
| • Thickness (mm) | • 2.5±0.4 |
| • Disintegration time (min) | • NMT 15 |
| • Friability (%) | • NMT 1.0 |

The minitablets were subjected to dissolution testing according to the standard USP method with type II apparatus USP29NF24<711>. Minitablets were stirred in 0.1 N hydrochloric acid for 1 hour before adjusting to pH 6.2 for 1 hour and finally pH7.4 for 4 hours. Samples were taken at time 0, and 60 minutes prior to pH adjustment then +60 minutes before the second pH adjustment and finally +30 minutes, +60 minutes and hourly thereafter. Butyrate was analysed and the titre plotted against time as shown in figure 18

### Finished formulation:

Part A and Part B minitabs are of similar size and characteristics and may be mixed in any useful ratio depending on the stage of the condition to be treated. In the current clinical trial they are mixed in the ratio of 1.94:2.28 w/w which provides a ratio of components of Menthol 70 : bromelain 480 : threonine 900 : sodium butyrate 600 : cholecalciferol 0.0125. An alternate ratio of 2.91 part A w/w to 2.28 Part B w/w provides a ratio of components of Menthol 105: bromelain 720 : threonine 900 : sodium butyrate 600 : cholecalciferol 0.0125, and a ratio.

The dose provided to the patient in the current clinical trial is either 4.22 grams twice per day providing a total daily dose of menthol 140 mg, bromelain 960 mg, threonine 1800 mg, sodium butyrate 1200 mg and cholecalciferol 25 micrograms; or 2.21 grams twice per day providing a total daily dose of menthol 70 mg, bromelain 480 mg, threonine 900 mg, sodium butyrate 600 mg and cholecalciferol 12.5 micrograms. Fill tolerances for the dosage are ± 15%.

### Example 14 - Histological scoring of sections post treatment in examples 11 and 12

### Evaluating GaRP in a TNBS mouse model

Examples 11 and 12 evaluated aspects of GaRP performance in the TNBS induced colitis in a mouse model. The effect of various treatment groups on endoscopic scores at day 11 relative to day 3 and on the weight, length and weight to length ration of the resected colon of euthanized animals was reported.

In this example, the colon was fixed, stained and sectioned before being assessed by microscopy in a blinded manner. Colons were examined not only for active disease but evidence of recovery from disease and scored according to the following criteria. Scoring was performed in triplicate blinded to the scorer with a maximum score of 3 in each of 6 categories.

### Histological Scoring of Colitis

Colitis activity (0-3 based on severity)
Hypervascularization (opening of blood vessels)
Presence of mononuclear cells (architecture also disappear)
Epithelial hyperplasia (elongated crypt and villi)
Epithelial injury (loss of epithelial continuity)
Presence of neutrophils
Lymphoid aggregates (scored 0-2) 0,1-2, >2

Data were collated and analysed by student t test for significance against placebo control. Summary statistics are reported in the table below, and mean data for each group minus the mean for healthy controls (x̅=5.1, σ=1.7) are charted in figure 19.

| | Mean | SD | P |
|---|---|---|---|
| Placebo | 4.4 | 2.5 | |
| GaRP | 2.5 | 2.3 | 0.034* |
| 2 mg/kg prednisolone | 3.5 | 2.5 | 0.196 |
| 150 mg/kg Bromelain + GaRP | 1.8 | 2.6 | 0.012* |
| 150 mg/kg Bromelain + GaRP + prednisolone | 2 | 1.5 | 0.005** |
| 75 mg/kg Bromelain + GaRP | 2.1 | 2.8 | 0.045* |

The data shows that the prednisolone treatment shows a trend to improved healing while the GaRP (threonine/butyrate/vitamin D) and complete formulations at both concentrations of bromelain show a statistically significant improvement in the histology scores irrespective of the presence or absence of prednisolone.

Without wishing to be bound by theory, the inventor believes that the data provided in the present specification shows that the use of colon-targeted GaRP components and small intestine-targeted bromelain/menthol may provide an unexpected and surprisingly robust therapeutic benefit for the treatment of colitis.

### References

Allen, e. a. (n.d.). Remington's Pharmaceutical Sciences, 22nd Ed. , ISBN 978-0-85711-062-6.
Amidon, S. B. (2016). Colon-Targeted Oral Drug Delivery Systems: Design Trends and Approaches. AAPS PharmSciTech, 16, No. 4, 731-741.
Bernstein CN, F. M. (2010). World Gastroenterology Organization Practice Guidelines for the diagnosis and management of IBD in 2010. Inflamm Bowel Dis., 16(1), 112-24.
Borley, N. (2005). Jejunum and ileum. Gray's Anatomy: the anatomical basis of clinical practice, 39, 1167-1172.
Cattaneo L, B. L. (1989). Stereogrammi di anatomia dell'uomo: aspetti istologici, 3. Encyclopaedia of Pharmaceutical Technology*.* (n.d.).
Hilgendorh C, L. H. (2000 ). JOURNAL OF PHARMACEUTICAL SCIENCES, 89, NO. 1, 63-7. HM Martin, B. C. (2004). Gastroenterology, 127, 80-93.
JR Bailey, C. P. (2011). PLos ONE, 6 (10*).*
Kong J, Z. Z. (2008). Novel role of the vitamin D receptor in maintaining the integrity of the intestinal mucosal barrier. Am J Physiol Gastrointest Liver Physiol., 294, G208-16.
Ma X, F. P. (2012). J. Anim. Sci., 90, 266-268.
Mudie DM, e. a. (2014). Mol. Pharmaceutics 2014, 11, 3039-3047.
Mynott TL, L. A. (1999). J Immunol, 163(5), 2568-75.
Rieder F, K. T.-H. (2012). Results of the 2nd scientific workshop of the ECCO (III): basic mechanisms of intestinal healing. J Crohns Colitis., 6, 373-85.
Sender, e. a. (2016). PLOS Biology. doi: 10.1371/journal.pbio.1002533 August 19, 2016
Teodori, U. (1987). Trattato di Medicina Interna. Roma: Societa' Editrice Universo.
Vamanu, E. (2018). Current Pharmaceutical Design (Vol. 24).
Weiss L, G. R. (1981). Istologia. Bologna: Zanichelli.

## Claims

1. A composition comprising the following components:
(a) bromelain,
(b) one or more short chain fatty acids (SCFAs) or esters thereof,
(c) one or more amino acids selected from the group consisting of L-threonine, L-tryptophan, L-arginine, L-glutamine and L-methionine and combinations thereof, and
(d) Vitamin D or alfacalcidiol.

2. The composition of claim 1 comprising (a), (b), L-threonine and (d).

3. The composition of any one of the preceding claims wherein the one or more SCFA or ester thereof is sodium butyrate, calcium butyrate, sodium propionate or sodium acetate, preferably sodium butyrate.

4. The composition of any one of the preceding claims consisting essentially of bromelain, sodium butyrate, L- threonine and Vitamin D or alfacalcidiol.

5. The composition of any one of the preceding claims wherein the composition is a unit dosage form that comprises about 50 mg to about 2500 mg, or about 250 to about 2250 mg, about 500 to about 2000 mg, about 750 to about 1750 mg, about 1000 to about 1600 mg, about 1300 to about 1550 mg, about 1400 to about 1500 mg, about 1420 to about 1460 mg, or about 1440 mg bromelain.

6. The composition of any one of the preceding claims wherein the composition is a unit dosage form that comprises about 45 to 1800 mg SCFA, or about 75 to about 1650 mg, about 250 to about 1500, about 500 to about 1400, about 750 to about 1500, about 1000 to about 1400, about 1100 to about 1300 mg, or about 1200 mg SCFA.

7. The composition of any one of the preceding claims wherein the composition is a unit dosage form that comprises about 67.5 to about 2700 mg, or about 112.5 to about 2250 mg, about 250 to about 2000 mg, about 500 to about 1900 mg, about 900 to about 1800 mg, or about 1800 mg L-threonine, L-tryptophan, L-glutamine, L-arginine or L-methionine or any combination thereof.

8. The composition of any one of the preceding claims wherein the composition is a unit dosage form that comprises about 4 to about 150 ug, about 6.5 to about 140 ug, about 13 to about 130 ug, about 25 to about 120 ug, about 35 to about 110 ug, about 50 to about 105 ug, about 100 ug, about 50 ug, about 25 ug, or about 12.5 ug vitamin D.

9. The composition of any one of claims 1 to 8 wherein Vitamin D is Vitamin D2 or Vitamin D3 or a combination thereof.

10. The composition of any one of claims 1 to 9 that is an oral dosage form.

11. A unit dosage form for use in a method for the treatment or prophylaxis of inflammatory bowel disease (IBD) or irritable bowel syndrome (IBS) comprising about 1 to about 10 g of a composition as defined in any one of claims 1 to 9.

12. The unit dosage form for the use of claim 11, wherein the unit dosage form is an oral dosage form.

13. The oral dosage form for the use of claim 12 comprising 50-2500 mg bromelain, 45-1800 mg sodium butyrate, 67.5-2700 mg L-threonine, 4-150 µg Vitamin D, and optionally 3-150 mg menthol, wherein Vitamin D is Vitamin D2 or Vitamin D3 or a combination thereof.

14. The oral dosage form for the use of claim 12 or claim 13 comprising two different types of pills, tablets, minitabs, capsules, granules, particles or microparticles, wherein each different type is formulated to release its contents to a different part of the gastrointestinal tract.

15. The oral dosage form for the use of claim 14 wherein the two different types of pills, tablets, minitabs, capsules, granules, particles or microparticles are Type A and Type B pills, tablets, minitabs, capsules, granules particles or microparticles, wherein the Type A and the Type B pills, tablets, minitabs, capsules, granules, particles or microparticles each comprise at least one component, preferably at least two components, of the composition defined in any one of claims 1 to 9.

16. The oral dosage form for the use of claim 15 wherein the Type A pills, tablets, minitabs, capsules, granules, particles or microparticles comprise of bromelain.

17. The oral dosage form for the use of claim 15 wherein the Type B pill, tablet, minitab, capsule, granule, particle or microparticle comprises L-threonine, sodium butyrate and Vitamin D.

18. A therapeutically effective amount of the composition of any one of claims 1 to 10, for use in a method of preventing, treating or managing IBD or IBS comprising administering to a subject in need thereof.

## Patentansprüche

1. Zusammensetzung, umfassend die folgenden Komponenten:
(a) Bromelain,
(b) eine oder mehrere kurzkettige Fettsäuren (SCFAs) oder Ester davon,
(c) eine oder mehrere Aminosäuren ausgewählt aus der Gruppe bestehend aus L-Threonin, L-Tryptophan, L-Arginin, L-Glutamin und L-Methionin und deren Kombinationen, und
(d) Vitamin D oder Alfacalcidol.

2. Zusammensetzung nach Anspruch 1, umfassend (a), (b), L-Threonin und (d).

3. Zusammensetzung nach einem der vorhergehenden Ansprüche, wobei die eine oder mehrere SCFA oder deren Ester Natriumbutyrat, Calciumbutyrat, Natriumpropionat oder Natriumacetat ist, bevorzugt Natriumbutyrat.

4. Zusammensetzung nach einem der vorhergehenden Ansprüche, die im Wesentlichen aus Bromelain, Natriumbutyrat, L-Threonin und Vitamin D oder Alfacalcidol besteht.

5. Zusammensetzung nach einem der vorhergehenden Ansprüche, wobei die Zusammensetzung eine Einheitsdosierungsform ist, die etwa 50 mg bis etwa 2500 mg, oder etwa 250 bis etwa 2250 mg, etwa 500 bis etwa 2000 mg, etwa 750 bis etwa 1750 mg, etwa 1000 bis etwa 1600 mg, etwa 1300 bis etwa 1550 mg, etwa 1400 bis etwa 1500 mg, etwa 1420 bis etwa 1460 mg oder etwa 1440 mg Bromelain umfasst.

6. Zusammensetzung nach einem der vorhergehenden Ansprüche, wobei die Zusammensetzung eine Einheitsdosierungsform ist, die etwa 45 bis 1800 mg SCFA, oder etwa 75 bis etwa 1650 mg, etwa 250 bis etwa 1500, etwa 500 bis etwa 1400, etwa 750 bis etwa 1500, etwa 1000 bis etwa 1400, etwa 1100 bis etwa 1300 mg oder etwa 1200 mg SCFA umfasst.

7. Zusammensetzung nach einem der vorhergehenden Ansprüche, wobei die Zusammensetzung eine Einheitsdosierungsform ist, die etwa 67,5 bis etwa 2700 mg, oder etwa 112,5 bis etwa 2250 mg, etwa 250 bis etwa 2000 mg, etwa 500 bis etwa 1900 mg, etwa 900 bis etwa 1800 mg oder etwa 1800 mg L-Threonin, L-Tryptophan, L-Glutamin, L-Arginin oder L-Methionin oder eine Kombination davon umfasst.

8. Zusammensetzung nach einem der vorhergehenden Ansprüche, wobei die Zusammensetzung eine Einheitsdosierungsform ist, die etwa 4 bis etwa 150 pg, etwa 6,5 bis etwa 140 pg, etwa 13 bis etwa 130 pg, etwa 25 bis etwa 120 pg, etwa 35 bis etwa 110 pg, etwa 50 bis etwa 105 pg, etwa 100 pg, etwa 50 pg, etwa 25 pg oder etwa 12,5 pg Vitamin D umfasst.

9. Zusammensetzung nach einem der Ansprüche 1 bis 8, wobei Vitamin D Vitamin D2 oder Vitamin D3 oder eine Kombination davon ist.

10. Zusammensetzung nach einem der Ansprüche 1 bis 9, die eine orale Dosierungsform ist.

11. Einheitsdosierungsform zur Verwendung in einem Verfahren zur Behandlung oder Prophylaxe von entzündlichen Darmerkrankungen (IBD) oder Reizdarmsyndrom (IBS), umfassend etwa 1 bis etwa 10 g einer Zusammensetzung gemäß einem der Ansprüche 1 bis 9.

12. Einheitsdosierungsform zur Verwendung nach Anspruch 11, wobei die Einheitsdosierungsform eine orale Dosierungsform ist.

13. Orale Dosierungsform zur Verwendung nach Anspruch 12, umfassend 50-2500 mg Bromelain, 45-1800 mg Natriumbutyrat, 67,5-2700 mg L-Threonin, 4-150 pg Vitamin D und optional 3-150 mg Menthol, wobei Vitamin D Vitamin D2 oder Vitamin D3 oder eine Kombination davon ist.

14. Orale Dosierungsform zur Verwendung nach Anspruch 12 oder Anspruch 13, umfassend zwei verschiedene Arten von Pillen, Tabletten, Mini-Tabletten, Kapseln, Granulaten, Partikeln oder Mikropartikeln, wobei jede verschiedene Art so formuliert ist, dass sie ihren Inhalt in einem anderen Teil des Magen-Darm-Trakts freisetzt.

15. Orale Dosierungsform zur Verwendung nach Anspruch 14, wobei die zwei verschiedenen Arten von Pillen, Tabletten, Mini-Tabletten, Kapseln, Granulaten, Partikeln oder Mikropartikeln Typ-A- und Typ-B-Pillen, Tabletten, Mini-Tabletten, Kapseln, Granulate, Partikel oder Mikropartikel sind, wobei die Typ-A- und die Typ-B-Pillen, Tabletten, Mini-Tabletten, Kapseln, Granulate, Partikel oder Mikropartikel jeweils mindestens eine Komponente, vorzugsweise mindestens zwei Komponenten, der in einem der Ansprüche 1 bis 9 definierten Zusammensetzung umfassen.

16. Orale Dosierungsform zur Verwendung nach Anspruch 15, wobei die Typ-A-Pillen, Tabletten, Mini-Tabletten, Kapseln, Granulate, Partikel oder Mikropartikel Bromelain umfassen.

17. Orale Dosierungsform zur Verwendung nach Anspruch 15, wobei die Typ-B-Pille, Tablette, Mini-Tablette, Kapsel, Granulat, Partikel oder Mikropartikel L-Threonin, Natriumbutyrat und Vitamin D umfasst.

18. Therapeutisch wirksame Menge der Zusammensetzung nach einem der Ansprüche 1 bis 10 zur Verwendung in einem Verfahren zur Vorbeugung, Behandlung oder Kontrolle von IBD oder IBS, umfassend die Verabreichung an ein Subjekt, das diese benötigt.

## Revendications

1. Composition comprenant les composants suivants :
(a) de la bromélaïne,
(b) un ou plusieurs acides gras à chaîne courte (SCFA) ou esters de ceux-ci,
(c) un ou plusieurs acides aminés choisis dans le groupe constitué de la L-thréonine, du L-tryptophane, de la L-arginine, de la L-glutamine et de la L-méthionine et de combinaisons de ceux-ci, et
(d) de la vitamine D ou de l'alphacalcidiol.

2. Composition selon la revendication 1 comprenant (a), (b), de la L-thréonine et (d).

3. Composition selon l'une quelconque des revendications précédentes, dans laquelle l'un ou les plusieurs SCFA ou esters de ceux-ci sont le butyrate de sodium, le butyrate de calcium, le propionate de sodium ou l'acétate de sodium, de préférence le butyrate de sodium.

4. Composition selon l'une quelconque des revendications précédentes, constituée essentiellement de bromélaïne, de butyrate de sodium, de L-thréonine et de vitamine D ou d'alfacalcidiol.

5. Composition selon l'une quelconque des revendications précédentes, dans laquelle la composition est une forme posologique unitaire qui comprend d'environ 50 mg à environ 2500 mg, ou d'environ 250 à environ 2250 mg, d'environ 500 à environ 2000 mg, d'environ 750 à environ 1750 mg, d'environ 1000 à environ 1600 mg, d'environ 1300 à environ 1550 mg, d'environ 1400 à environ 1500 mg, d'environ 1420 à environ 1460 mg ou environ 1440 mg de bromélaïne.

6. Composition selon l'une quelconque des revendications précédentes, dans laquelle la composition est une forme posologique unitaire qui comprend d'environ 45 à 1800 mg de SCFA, ou d'environ 75 à environ 1650 mg, d'environ 250 à environ 1500, d'environ 500 à environ 1400, d'environ 750 à environ 1500, d'environ 1000 à environ 1400, d'environ 1100 à environ 1300 mg ou environ 1200 mg de SCFA.

7. Composition selon l'une quelconque des revendications précédentes, dans laquelle la composition est une forme posologique unitaire qui comprend d'environ 67,5 à environ 2700 mg, ou d'environ 112,5 à environ 2250 mg, d'environ 250 à environ 2000 mg, d'environ 500 à environ 1900 mg, d'environ 900 à environ 1800 mg, ou environ 1800 mg de L-thréonine, de L-tryptophane, de L-glutamine, de L-arginine ou de L-méthionine ou de toute combinaison de ceux-ci.

8. Composition selon l'une quelconque des revendications précédentes, dans laquelle la composition est une forme posologique unitaire qui comprend environ d'environ 4 à environ 150 ug, d'environ 6,5 à environ 140 ug, d'environ 13 à environ 130 ug, d'environ 25 à environ 120 ug, d'environ 35 à environ 110 ug, d'environ 50 à environ 105 ug, environ 100 ug, environ 50 ug, environ 25 ug ou environ 12,5 ug de vitamine D.

9. Composition selon l'une quelconque des revendications 1 à 8, dans laquelle la vitamine D est la vitamine D2 ou la vitamine D3 ou une combinaison de celles-ci.

10. Composition selon l'une quelconque des revendications 1 à 9, qui est une forme posologique orale.

11. Forme posologique unitaire pour une utilisation dans un procédé de traitement ou de prophylaxie de la maladie inflammatoire de l'intestin (MII) ou du syndrome du côlon irritable (IBS), comprenant d'environ 1 à environ 10 g d'une composition selon l'une quelconque des revendications 1 à 9.

12. Forme posologique unitaire pour l'utilisation selon la revendication 11, dans laquelle la forme posologique unitaire est une forme posologique orale.

13. Forme posologique orale pour l'utilisation selon la revendication 12, comprenant de 50 à 2500 mg de bromélaïne, de 45 à 1800 mg de butyrate de sodium, de 67,5 à 2700 mg de L-thréonine, de 4 à 150 pg de vitamine D et éventuellement de 3 à 150 mg de menthol, dans laquelle la vitamine D est la vitamine D2 ou la vitamine D3 ou une combinaison de celles-ci.

14. Forme posologique orale pour l'utilisation selon la revendication 12 ou la revendication 13, comprenant deux types différents de pilules, comprimés, minitabs, capsules, granulés, particules ou microparticules, dans laquelle chaque type différent est formulé pour libérer son contenu dans une partie différente du tractus gastro-intestinal.

15. Forme posologique orale pour l'utilisation selon la revendication 14, dans laquelle les deux types différents de pilules, comprimés, minitabs, capsules, granulés, particules ou microparticules sont des pilules, comprimés, minitabs, capsules, granulés, particules ou microparticules de type A et de type B, dans laquelle les pilules, comprimés, minitabs, capsules, granulés, particules ou microparticules de type A et de type B comprennent chacun au moins un composant, de préférence au moins deux composants, de la composition selon l'une quelconque des revendications 1 à 9.

16. Forme posologique orale pour l'utilisation selon la revendication 15, dans laquelle les pilules, comprimés, minitabs, capsules, granulés, particules ou microparticules de type A comprennent de la bromélaïne.

17. Forme posologique orale pour l'utilisation selon la revendication 15, dans laquelle la pilule, le comprimé, le minitab, la capsule, le granule, la particule ou la microparticule de type B comprend de la L-thréonine, du butyrate de sodium et de la vitamine D.

18. Quantité thérapeutiquement efficace de la composition selon l'une quelconque des revendications 1 à 10, pour une utilisation dans un procédé de prévention, de traitement ou de gestion de la MII ou du SCI comprenant l'administration à un sujet en ayant besoin.
